# EUROPEAN PATENT APPLICATION

(11) **EP 4 393 484 A1**
(43) Date of publication of application: **03.07.2024**
(21) Application number: 23838762.5
(22) Date of filing: 30.06.2023
(51) Int. Cl.: A61K 9/51, A61K 47/28, A61K 9/127, A61P 35/00

(54) **COMPOSITION FOR ORGAN-SPECIFIC DELIVERY OF NUCLEIC ACID**

(30) Priority: 11.07.2022 CN 202210809436
(71) Applicant: Themedium Therapeutics Co., Ltd., Suzhou, Jiangsu 215011 (CN)
(72) Inventor: ZHANG, Lei, Suzhou, Jiangsu 215011 (CN); CHEN, Jianxin, Suzhou, Jiangsu 215011 (CN); YAN, Lu, Suzhou, Jiangsu 215011 (CN); PENG, Wei, Suzhou, Jiangsu 215011 (CN); MA, Qiaoqiao, Suzhou, Jiangsu 215011 (CN)
(74) Representative: LLR
(86) International application number: PCT/CN2023/104901
(87) International publication number: WO 2024/012270

(57) **Abstract**

The present application provides a composition for organ-specific delivery of nucleic acids, which comprises a guided on-target lipid delivery lipid; further, the composition may comprise a helper lipid; still further, the composition may comprise a cationic lipid. The guided on-target lipid delivery lipid may be selected from one or more of an ionizable anionic steroid and/or an ionizable anionic polymer conjugated lipid; the helper lipid is optionally one or more of a phospholipid, a steroid, a polymer conjugated lipid, and a modifiable lipid; and the cationic lipid may be selected from one or more of a permanently cationic lipid and/or an ionizable cationic lipid. The delivery composition is capable of specifically delivering a prophylactic/therapeutic agent, particularly a nucleic acid component, to a target organ.

## Description

### CROSS-REFERENCE

This application claims the priority of the Chinese patent application 202210809436.X filed on July 11, 2022 with the invention name "Composition for Organ-specific Delivery of Nucleic Acids", and the full contents of which are incorporated herein by reference.

### TECHNICAL FIELD

The present invention relates generally to the field of molecular biology. More particularly, it relates to use of a lipid nanoparticle composition in organ-specific delivery of substances such as nucleic acids.

### BACKGROUND

Therapeutic or prophylactic nucleic acids have the potential to revolutionize vaccination, gene therapy, protein alternative therapy and other therapies for genetic diseases. Since the first clinical study on therapeutic nucleic acids in the 2000s, significant advances have been made in the research on the design of nucleic acid molecules and the method of delivering them. However, nucleic acid drugs (including therapeutic and prophylactic drugs) still face several challenges, e.g., the accumulation of most liposome formulations through biological processes in the liver, thereby reducing the efficacy of compositions delivered into target organs. Similarly, other therapeutic agents such as proteins and small molecule drugs can also benefit from organ-specific delivery. Many different types of compounds such as chemical drugs exhibit significant cytotoxicity. If these compounds could be better directionally delivered to the desired organ, fewer off-target effects and side effects would be seen.

Currently, most lipid nanoparticle delivery systems passively target the liver. A common strategy to alter the organ a lipid nanoparticle target is to adjust the composition of the lipid nanoparticle. According to some reports, lipid nanoparticles composed of 1-2 types of lipids can achieve the purpose of targeting the spleen by adjusting the ratio in which the nucleic acid and the nanoparticles are mixed (Stephan Grabbe et al., Translating Nanoparticulate-personalized Cancer Vaccines into Clinical Applications: Case Study with RNA-Lipoplexes for the Treatment of Melanoma, 2016); however, this strategy needs improvement to formulation stability, encapsulation efficiency, etc. In addition, according to some reports (Cheng Qiang et al., Selective organ targeting (SORT) nanoparticles for tissue-specific mRNA delivery and CRISPR-Cas gene editing, 2020), the addition of permanently anionic lipids to LNPs consisting of four components-a cationizable lipid, a steroid, a phospholipid, and a PEG lipid-also enables the specific targeting of the spleen, but at a price of an increase in the composition and an increase in the complexity of the formulation process. Therefore, a lipid nanoparticle delivery system with high delivery capacity, high stability and low complexity remains to be developed.

### SUMMARY

The present invention provides a lipid nanoparticle which can target different tissues and organs for drug delivery. The lipid nanoparticle provided by the present invention comprises the guided on-target lipid delivery (GOLD) lipid of the present invention. The guided on-target lipid delivery (GOLD) lipid is selected from one of or a combination of more than one of an ionizable anionic steroid and/or an ionizable anionic polymer conjugated lipid; further, the lipid nanoparticle provided by the present invention comprises a helper lipid; still further, the lipid nanoparticle provided by the present invention comprises a cationic lipid.

The helper lipid is optionally one or more of a phospholipid, a steroid, a polymer conjugated lipid, and a modifiable lipid.

Preferably, the phospholipid is selected from any one of DOPE, DSPC, DPPC, DMPC, DOPC, POPC and SM or a combination thereof.

Preferably, the steroid is selected from one or more of cholesterol, sitosterol, stigmasterol, and ergosterol.

Preferably, the polymer in the polymer conjugated lipid is a high-molecular-weight compound formed by covalent bonding of one or more small-molecule repeating units; the polymer may be selected from polyethylene glycol, polylactic acid, polyamide, cationic polymer, poly sarcosine (pSar), poly lactic-co-glycolic acid (PLGA), polyamino acid, polypeptide, polypeptoid, etc.; preferably, the polymer conjugated lipid is selected from a polyethylene glycol conjugated lipid; further, the polyethylene glycol conjugated lipid is selected from one or more of PEG1000-DMG, PEG5000-DMG, PEG2000-DMG, and PEG2000-DSPE.

Preferably, the modifiable lipid includes lipids modified by small-molecule compounds, vitamins, carbohydrates, peptides, proteins, nucleic acids, lipopolysaccharides, inorganic molecules or particles, metal ions or particles, and combinations of the substances described above.

The cationic lipid is selected from one of or a combination of more than one of a permanently cationic lipid and/or an ionizable cationic lipid. The permanently cationic lipid is selected from one of or a combination of more than one of DOTAP, DODMA, DOTAP, DSTAP, DMTAP, DDA, and DOBAQ; the ionizable cationic lipid is selected from one of or a combination of more than one of SM-102, Lipid 5, A6, DC-chol, C12-200, CKK-E12, 5A2-SC8, G0-C14, OF-2, 306Oi10, OF-Deg-Lin, 92-0175, OF-C4-Deg-Lin, A18-iso5-2DC18, TT3, FTT5, BAMEA-O16B, Vc-Lipid, C14-4, Lipid 14, 4A3-Cit, and ssPalmO-Phe.

The specificity for tissues and organs is achieved by the guided on-target lipid delivery (GOLD) lipid in the lipid nanoparticle; further by a combination of the guided on-target lipid delivery (GOLD) lipid and the helper lipid; still further by a combination of the guided on-target lipid delivery (GOLD) lipid, the helper lipid, and the cationic lipid.

The lipid nanoparticle preferentially delivers the therapeutic/prophylactic agent to the following target organs: lung, heart, brain, spleen, lymph node, bone, skeletal muscle, stomach, small intestine, large intestine/colon and rectum, kidney, bladder, breast, testis, ovary, uterus, thymus, brainstem, cerebellum, cerebrum, spinal cord, eye, ear, tongue, or skin. Preferably, the target organ is the spleen.

The GOLD lipid in the lipid nanoparticle is optionally one or more of an ionizable anionic steroid and/or an ionizable anionic polymer conjugated lipid.

Preferably, the ionizable anionic steroid is selected from a compound of Formula I or a pharmaceutically acceptable salt, a prodrug, a stereoisomer, or a deuteride thereof: 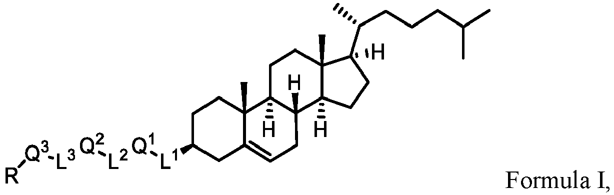
wherein L¹ is absent, -C-C-, -C=C-, -C≡C-, -O(C=O)-, -(C=O)O-, -C(=O)-, -O(C=O)O-, -O-, - S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-; Q¹ is absent or C1-C8 linear or branched hydrocarbyl, and specifically can be C1, C2, C3, C4, C5, C6, C7, or C8 linear or branched hydrocarbyl;
L² is absent, -C-C-, -C=C-, -C≡C-, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-; Q² is absent or C1-C8 linear or branched hydrocarbyl, and specifically can be C1, C2, C3, C4, C5, C6, C7, or C8 linear or branched hydrocarbyl;
L³ is absent, -C-C-, -C=C-, -C≡C-, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-; Q³ is absent or C1-C8 linear or branched hydrocarbyl, and specifically can be C1, C2, C3, C4, C5, C6, C7, or C8 linear or branched hydrocarbyl;
R^{a} is H, deuterium, C1-C8 linear or branched hydrocarbyl, or C1-C8 linear or branched hydrocarbyl substituted with carboxyl, and specifically can be C1, C2, C3, C4, C5, C6, C7, or C8 linear or branched hydrocarbyl; or -Q⁴-M, wherein Q⁴ is absent or C1-C8 linear or branched hydrocarbyl, and specifically can be C1, C2, C3, C4, C5, C6, C7, or C8 linear or branched hydrocarbyl, and M is 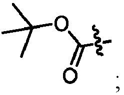 x is 0, 1, or 2;
R is 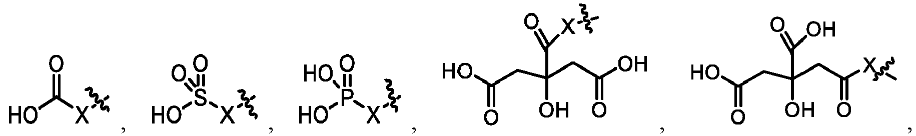 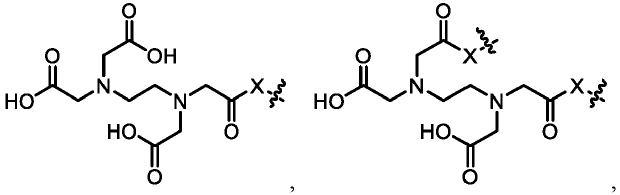 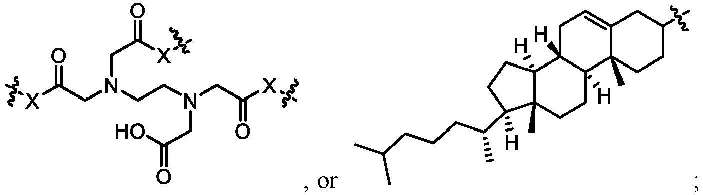 X is C, O, NR^{b}, or S; R^{b} is H, deuterium, or C1-C8 linear or branched hydrocarbyl, and specifically can be C1, C2, C3, C4, C5, C6, C7, or C8 linear or branched hydrocarbyl.

According to the above general formula, Table 1 lists, without limitation, some ionizable anionic steroid compounds.

**Table 1. Representative compounds of ionizable anionic steroids**

| No. | Structural formula |
|---|---|
| 1-1 | 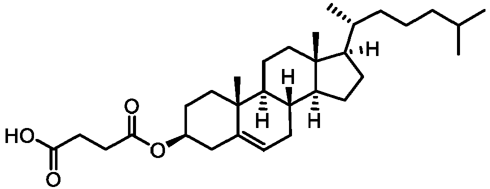 |
| 1-2 | 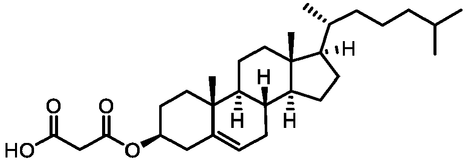 |
| 1-3 | 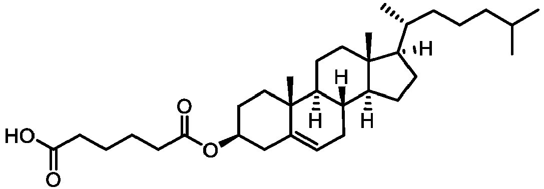 |
| 1-4 | 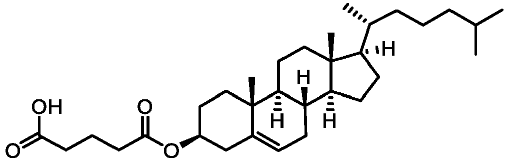 |
| 1-5 | 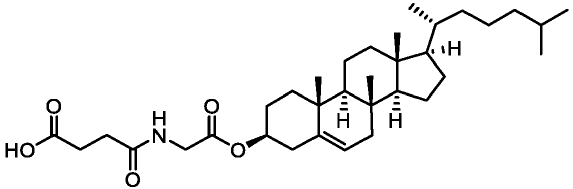 |
| 1-6 | 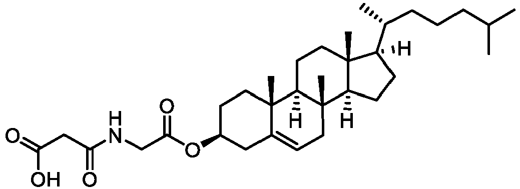 |
| 1-7 | 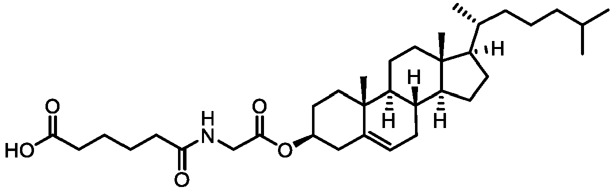 |
| 1-8 | 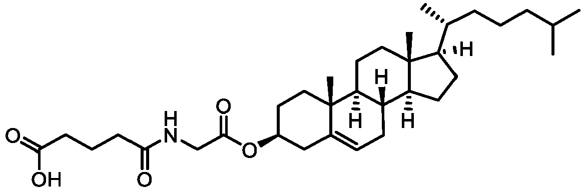 |
| 1-9 | 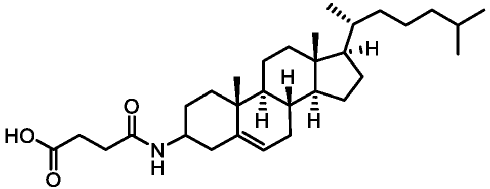 |
| 1-10 | 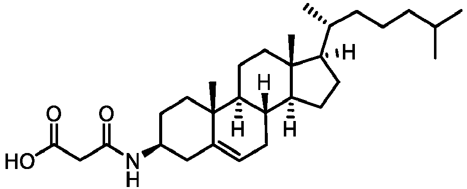 |
| 1-11 | 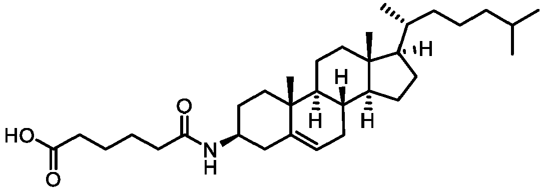 |
| 1-12 | 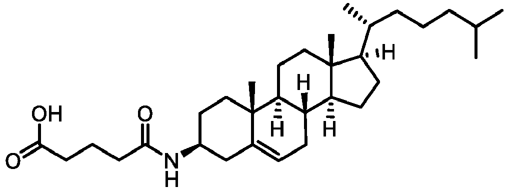 |
| 1-13 | 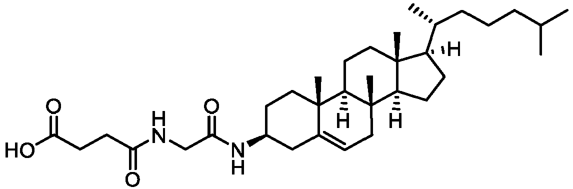 |
| 1-14 | 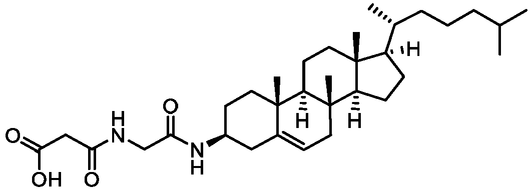 |
| 1-15 | 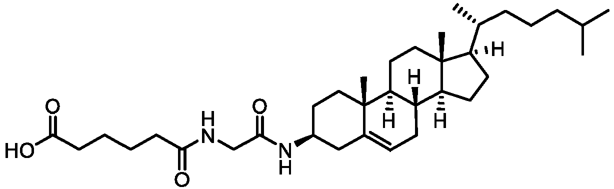 |
| 1-16 | 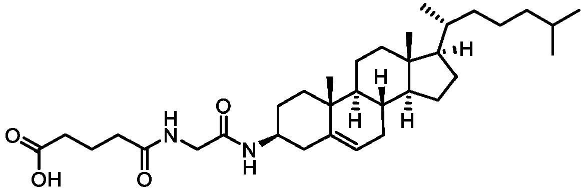 |
| 1-17 | 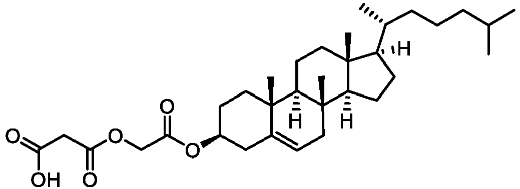 |
| 1-18 | 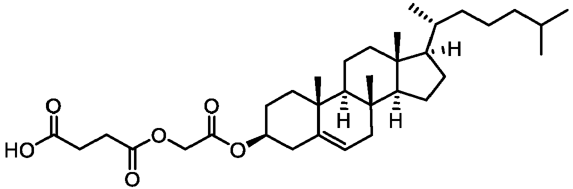 |
| 1-19 | 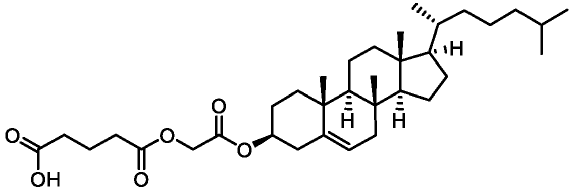 |
| 1-20 | 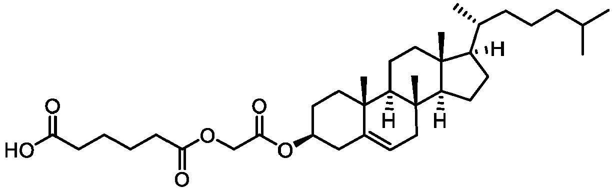 |
| 1-21 | 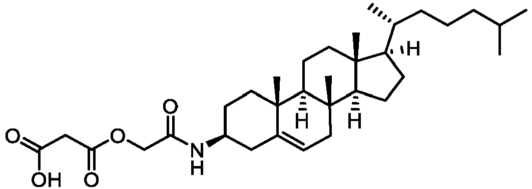 |
| 1-22 | 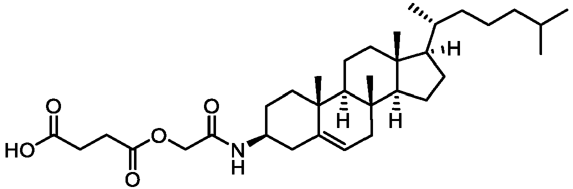 |
| 1-23 | 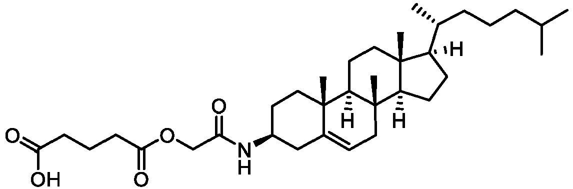 |
| 1-24 | 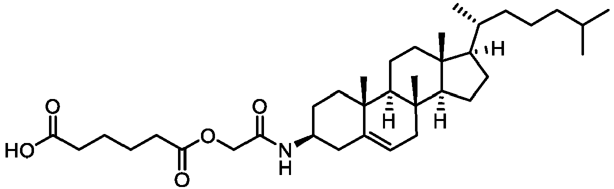 |
| 1-25 | 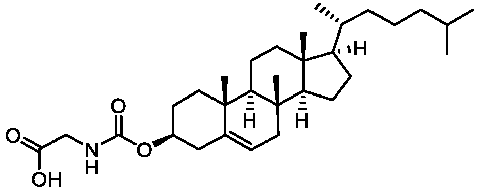 |
| 1-26 | 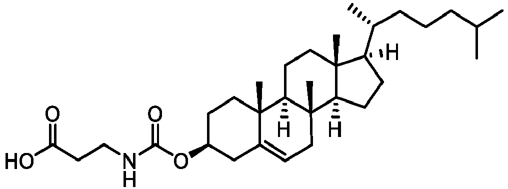 |
| 1-27 | 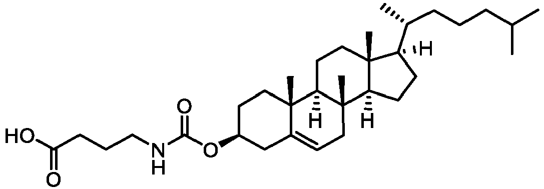 |
| 1-28 | 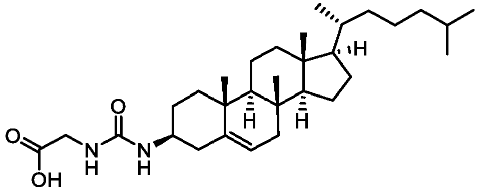 |
| 1-29 | 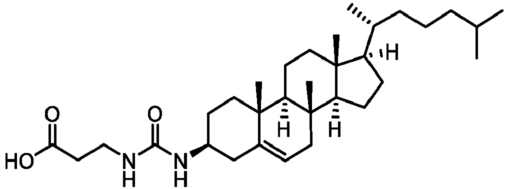 |
| 1-30 | 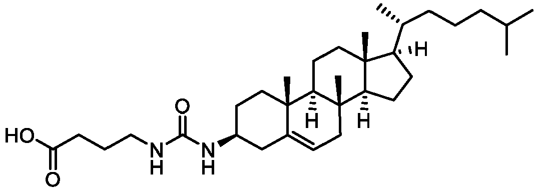 |
| 1-31 | 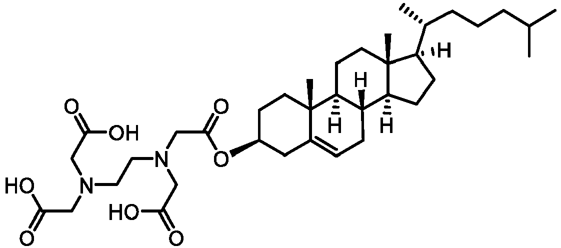 |
| 1-32 | 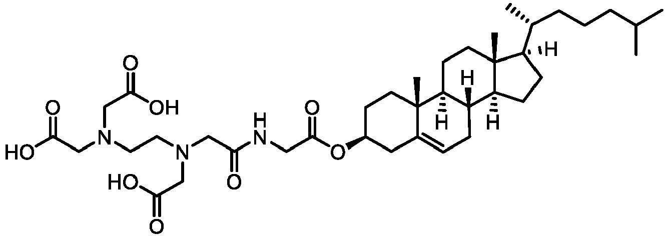 |
| 1-33 | 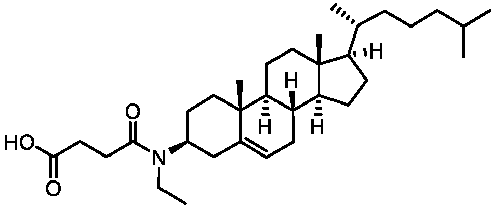 |
| 1-34 | 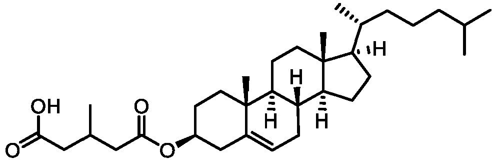 |
| 1-35 | 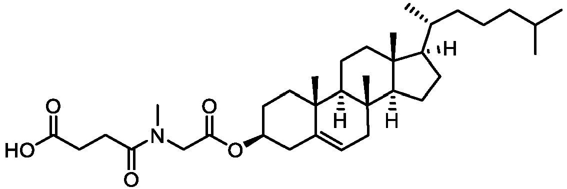 |
| 1-36 | 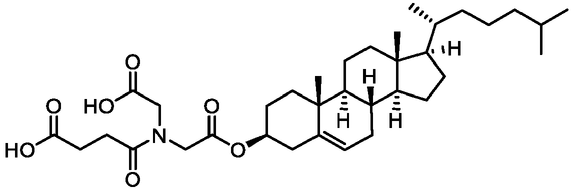 |
| 1-37 | 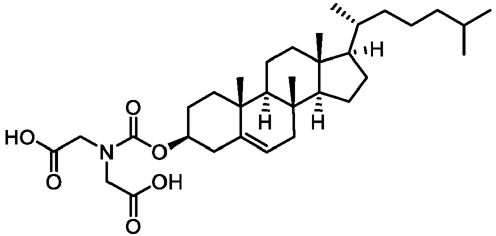 |
| 1-38 | 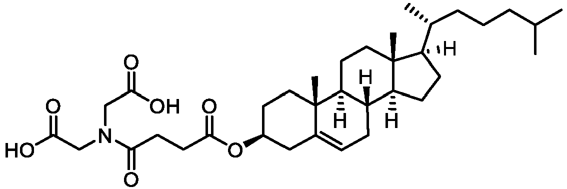 |
| 1-39 | 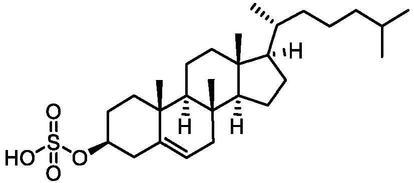 |
| 1-40 | 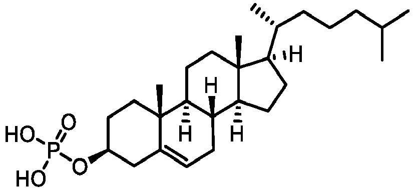 |
| 1-41 | 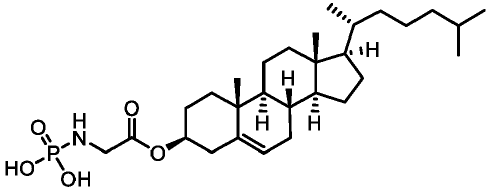 |
| 1-42 | 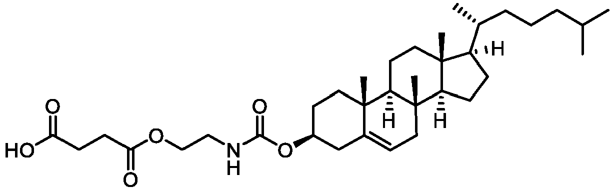 |
| 1-43 | 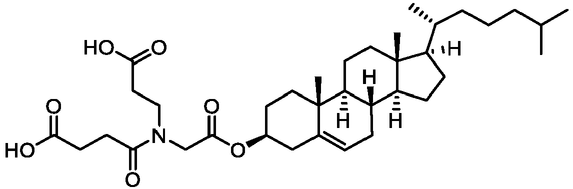 |
| 1-44 | 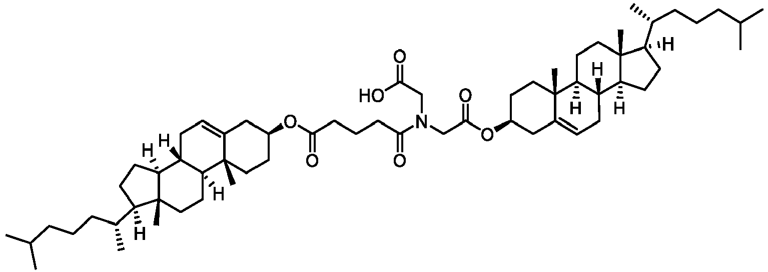 |
| 1-45 | 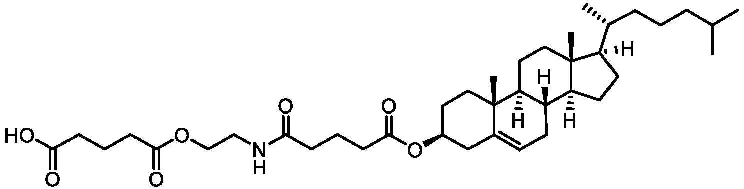 |
| 1-46 | 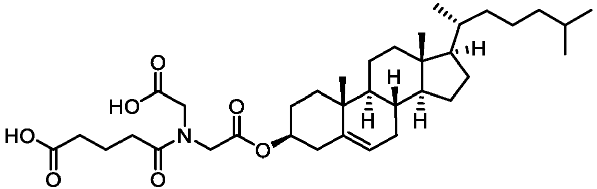 |
| 1-47 | 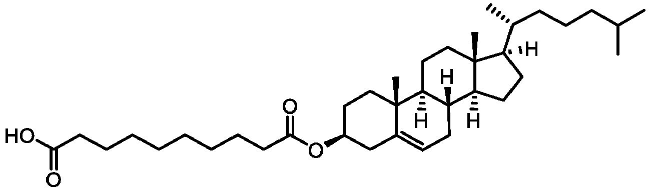 |
| 1-48 | 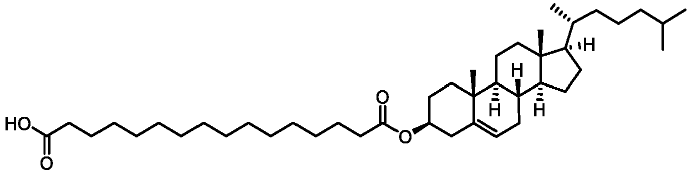 |
| 1-49 | 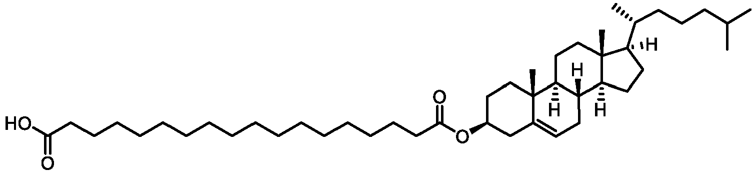 |
| 1-50 | 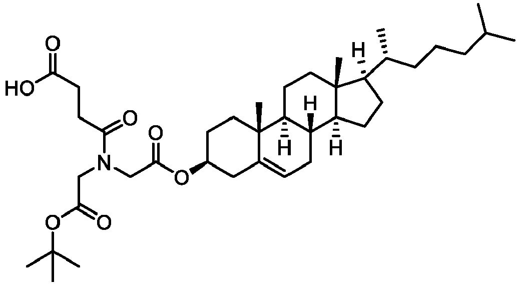 |
| 1-51 | 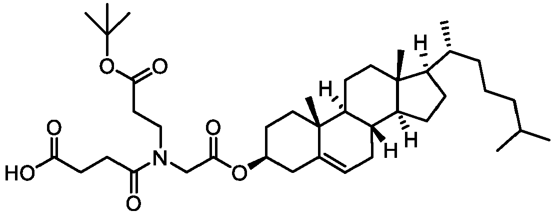 |
| 1-52 | 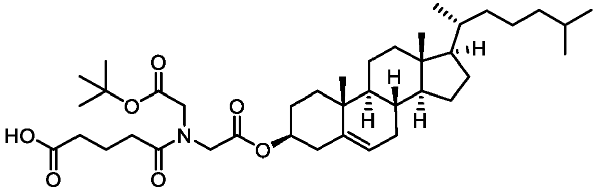 |
| 1-53 | 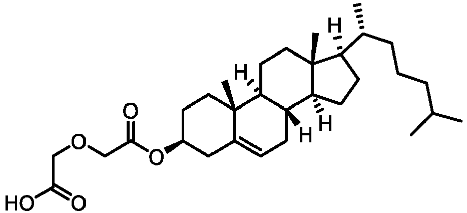 |
| 1-54 | 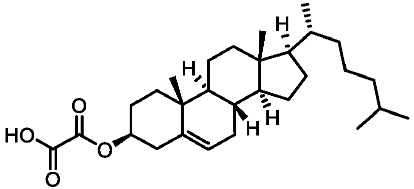 |
| 1-55 | 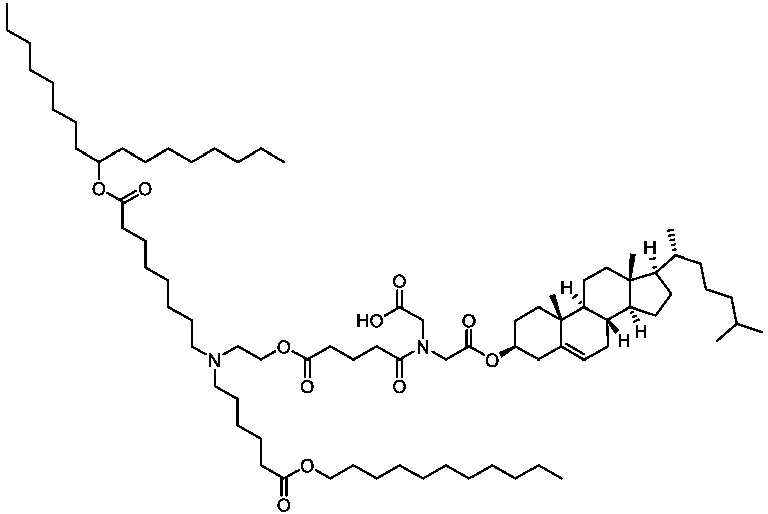 |
| 1-56 | 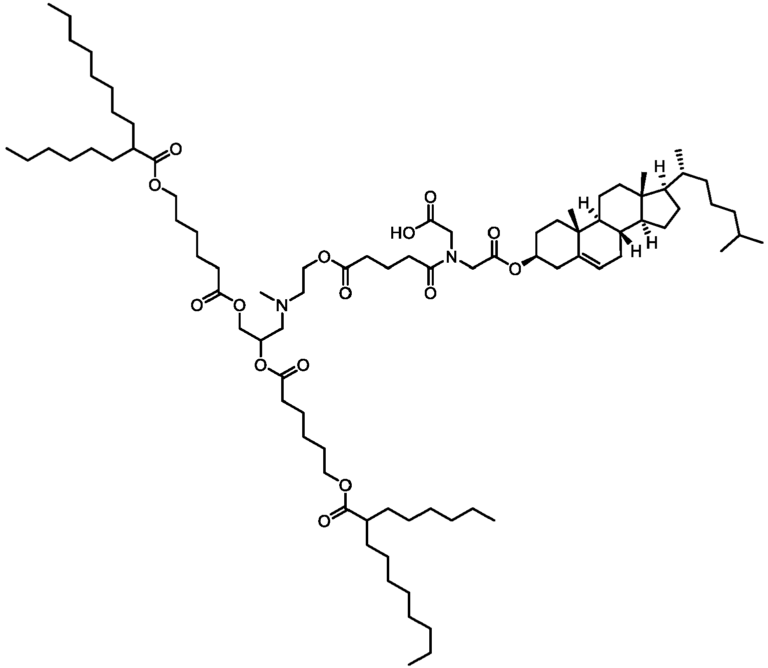 |

Preferably, the ionizable anionic polymer conjugated lipid is selected from a compound of Formula II or a pharmaceutically acceptable salt, a prodrug, a stereoisomer, or a deuteride thereof: 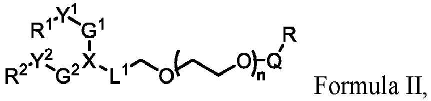
wherein L¹ is absent, -C-C-, -C=C-, -C≡C-, -O(C=O)-, -(C=O)O-, -C(=O)-, -O(C=O)O-, -O-, - S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-; Q is absent or C1-C8 linear or branched hydrocarbyl, and specifically can be C1, C2, C3, C4, C5, C6, C7, or C8 linear or branched hydrocarbyl;
R^{a} is H, deuterium, C1-C8 linear or branched hydrocarbyl, or C1-C8 linear or branched hydrocarbyl substituted with carboxyl, and specifically can be C1, C2, C3, C4, C5, C6, C7, or C8 linear or branched hydrocarbyl, or C1, C2, C3, C4, C5, C6, C7, or C8 linear or branched hydrocarbyl substituted with carboxyl;
X is CorN;
R is 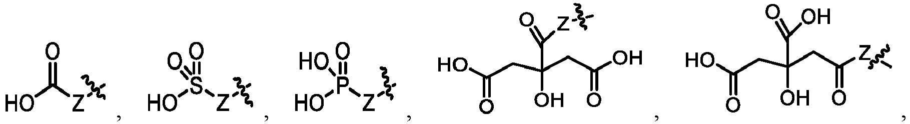 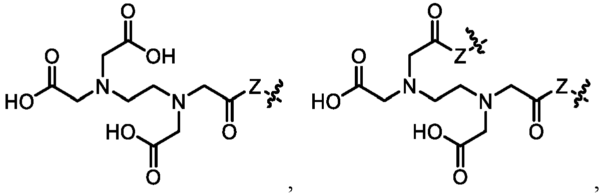 or 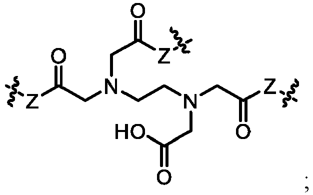 Z is C, O, NR^{b}, or S;
one of Y¹ and Y² is absent, -C-C-, -C=C-, -C=C-, -O(C=O)-, -C(OH)-, -(C=O)O-, -C(=O)-, - O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-, and the other one of Y¹ and Y² is absent, -C-C-, -C=C-, -C≡C-, -O(C=O)-, -C(OH)-, -(C=O)O-, -C(=O)-, -O(C=O)O-, -O-, -S(O)ₓ-, -S-S-, -C(=O)S-, -SC(=O)-, - NR^{a}C(=O)-, -C(=O)NR^{a}-, -NR^{a}C(=O)NR^{a}-, -OC(=O)NR^{a}-, or -NR^{a}C(=O)O-; R^{a} is H, deuterium, C1-C8 linear or branched hydrocarbyl, or C1-C8 linear or branched hydrocarbyl substituted with carboxyl, and specifically can be C1, C2, C3, C4, C5, C6, C7, or C8 linear or branched hydrocarbyl, or C1, C2, C3, C4, C5, C6, C7, or C8 linear or branched hydrocarbyl substituted with carboxyl;
G¹ and G² are each independently absent or substituted C1-C12 linear or branched hydrocarbyl, and specifically can be C1, C2, C3, C4, C5, C6, C7, C8, C9, C10, C11, or C12 linear or branched hydrocarbyl;
R¹ and R² are each independently substituted C6-C24 linear or branched hydrocarbyl, and specifically can be C6, C7, C8, C9, C10, C11, C12, C13, C14, C15, C16, C17, C18, C19, C20, C21, C22, C23, or C24 linear or branched hydrocarbyl;
x is 0, 1, or 2.

According to the above general formula, Table 2 lists, without limitation, some ionizable anionic polymer conjugated lipid compounds.

**Table 2. Representative compounds of ionizable anionic polymer conjugated lipids**

| No. | Structure |
|---|---|
| 2-130 | 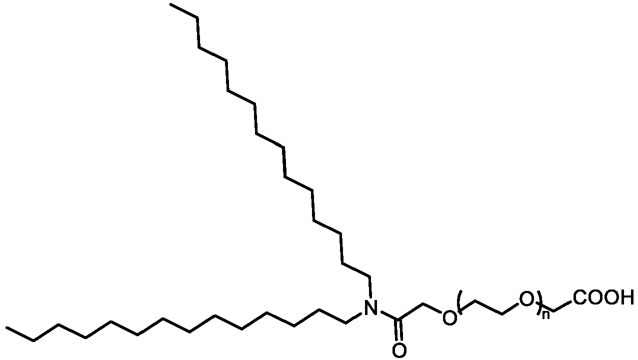 |
| 2-131 | 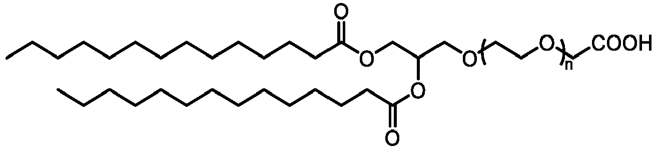 |

Further, the lipid nanoparticle may comprise a helper lipid. The helper lipid is optionally one or more of a phospholipid, a steroid, a polymer conjugated lipid, and a modifiable lipid.

Preferably, the phospholipid is selected from any one of DOPE, DSPC, DPPC, DMPC, DOPC, POPC and SM or a combination thereof.

Preferably, the steroid is selected from one or more of cholesterol, sitosterol, stigmasterol, and ergosterol.

Preferably, the polymer in the polymer conjugated lipid is a high-molecular-weight compound formed by covalent bonding of one or more small-molecule repeating units; the polymer may be selected from polyethylene glycol, polylactic acid, polyamide, cationic polymer, poly sarcosine (pSar), poly lactic-co-glycolic acid (PLGA), polyamino acid, polypeptide, polypeptoid, etc.; preferably, the polymer conjugated lipid is selected from a polyethylene glycol conjugated lipid; further, the polyethylene glycol conjugated lipid is selected from one or more of PEG1000-DMG, PEG5000-DMG, PEG2000-DMG, and PEG2000-DSPE.

Preferably, the modifiable lipid includes lipids modified by small-molecule compounds, vitamins, carbohydrates, peptides, proteins, nucleic acids, lipopolysaccharides, inorganic molecules or particles, metal ions or particles, and combinations of the substances described above.

Still further, the lipid nanoparticle comprises a cationic lipid.

The cationic lipid in the lipid nanoparticle comprises an ammonium group that is positively charged at a given pH and comprises at least two hydrophobic groups. The cationic lipid is a dendrimer or dendron. The cationic lipid comprises at least two C6-C24 hydrocarbyl groups. The cationic lipid may be one of or a combination of more than one of a permanently cationic lipid and/or an ionizable cationic lipid. The permanently cationic lipid may be selected from one or more of DOTAP, DODMA, DSTAP, DMTAP, DDA, and DOBAQ; the ionizable cationic lipid may be selected from one of or a combination of more than one of SM-102, Lipid 5, A6, DC-chol, C12-200, CKK-E12, 5A2-SC8, G0-C14, OF-2, 306Oi10, OF-Deg-Lin, 92-0175, OF-C4-Deg-Lin, A18-iso5-2DC18, TT3, FTT5, BAMEA-O16B, Vc-Lipid, C14-4, Lipid 14, 4A3-Cit, and ssPalmO-Phe.

In the lipid nanoparticle, the cationic lipid and the guided on-target delivery lipid are in a molar ratio of 1: 1 to 11: 1.

In the lipid nanoparticle, the cationic lipid, the guided on-target delivery lipid, and the helper lipid are in a molar ratio of 1:(0. 1-1):(0.5-2).

In other aspects, the present invention provides a composition comprising a therapeutic or prophylactic agent and the lipid nanoparticle described above.

In a preferred instance, the therapeutic/prophylactic agent is a nucleic acid.

Preferably, the nucleic acid includes any and all forms of nucleic acid molecules, including but not limited to, single-stranded DNA, double-stranded DNA, single-stranded RNA, double-stranded RNA, short isomers, plasmid DNA, complementary DNA/cDNA, antisense oligonucleotide/ASO, small interfering nucleic acid/siRNA, small activating nucleic acid/saRNA, asymmetric interfering nucleic acid/aiRNA, micro nucleic acid/miRNA, miRNA inhibitors (agomir, antagomir), Dicer enzyme substrate nucleic acid, small hairpin nucleic acid/shRNA, transfer RNA (tRNA), messenger RNA/mRNA, circular RNA/circRNA, self-amplifying mRNA/samRNA, aptamers, and other forms of nucleic acid molecules known in the art.

The above nucleic acid molecules may include natural nucleotides, or may include nucleotide mimics or functional analogs, or may include chemically modified forms of nucleotides. Functional nucleotide analogs include, but are not limited to, one of or a combination of more than one of a locked nucleic acid (LNA), a peptide nucleic acid (PNA), and a morpholine ring oligonucleotide nucleic acid mimic or functional analog.

The chemical modification of nucleotides may be located on a backbone bond of the nucleic acid molecule. The backbone bond may be modified by the replacement of one or more oxygen atoms. The modification to the backbone bond may include replacing at least one phosphodiester bond with a phosphorothioate bond.

The chemical modification of nucleotides may be located on a nucleoside. The modification on the nucleoside may be located on the sugar and base of the nucleoside. The sugar on the nucleoside may be selected from one or more of: 2'-fluororibose, ribose, 2'-deoxyribose, arabinose, and hexose.

The chemically modified forms of nucleotides may be selected from one or more of: 5-methylcytosine, pseudouridine, 1-methylpseudouridine, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. In a preferred instance, the nucleic acid is an mRNA capable of encoding at least one antigen or a fragment thereof or an epitope thereof, or encoding a certain therapeutic protein, the antigen being selected from a pathogenic antigen, such as a tumor-associated antigen or a pathogenic microbial antigen. The mRNA may be a monocistronic mRNA or a polycistronic mRNA.

The present invention further provides use of the above composition in the preparation of medicaments.

The present invention further provides a drug comprising the above composition and pharmaceutically acceptable auxiliary materials (also referred to as pharmaceutical auxiliary materials). The pharmaceutical auxiliary materials refer to excipients and additives used in drug production and prescription dispensing and are substances other than the active ingredient, which have been rationally evaluated in safety and are contained in a pharmaceutical formulation. In addition to shaping, serving as vehicles and enhancing stability, pharmaceutical auxiliary materials also have such important functions as solubilization, aiding dissolution, sustaining or controlling release, etc. They are important ingredients which possibly affect the quality, safety and effectiveness of drugs. According to the effect and the purpose, pharmaceutical auxiliary materials may be classified into solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesives, integrating agents, permeation promotors, pH regulators, buffers, plasticizers, surfactants, foaming agents, defoaming agents, thickeners, inclusion agents, humectants, absorbents, diluents, flocculants, deflocculants, filter aids, release retardants, etc.

The beneficial effects of the present disclosure are as follows:
The present invention provides a novel lipid nanoparticle, which can deliver a therapeutic/prophylactic agent, particularly a nucleic acid component, to a specific organ, particularly to organs other than the liver preferentially, so as to provide more options for the delivery of nucleic acid drugs, genetic drugs, vaccines and the like, and particularly have important significance for the development and application of nucleic acid prophylactic and therapeutic agents.

Conventional lipid nanoparticles generally deliver to the liver in a targeted way. The organ targeting property is greatly altered by the addition of the guided on-target lipid delivery (GOLD) lipid to the lipid nanoparticle. In the lipid nanoparticles such as TMF1, TMF2, TMF7 and TMF11-TMF38 to which the GOLD lipid is added, the ratio of the distribution (mean fluorescence intensity) of nucleic acid drugs in the spleen to that in the liver is 1.6-80 (Tables 7 and 8). However, in conventional lipid nanoparticles without the addition of the GOLD lipid, there is a higher delivery efficiency in the liver (Ansell, S. M.; Du, X. Novel Lipids and Lipid Nanoparticle Formulations for Delivery of Nucleic Acids. WO2017075531 A1).

Therefore, the guided on-target lipid delivery (GOLD) lipid plays an important role in the organ targeting property of the lipid nanoparticle.

### BRIEF DESCRIPTION OF THE DRAWINGS

FIG. 1 shows a nuclear magnetic resonance spectrum of compound 1-1.
FIG. 2 shows a nuclear magnetic resonance spectrum of compound 1-2.
FIG. 3 shows a nuclear magnetic resonance spectrum of compound 1-3.
FIG. 4 shows a nuclear magnetic resonance spectrum of compound 1-4.
FIG. 5 shows a nuclear magnetic resonance spectrum of compound 1-5.
FIG. 6 shows a nuclear magnetic resonance spectrum of compound 1-6.
FIG. 7 shows a nuclear magnetic resonance spectrum of compound 1-7.
FIG. 8 shows a nuclear magnetic resonance spectrum of compound 1-8.
FIG. 9 shows a nuclear magnetic resonance spectrum of compounds 1-9.
FIG. 10 shows a nuclear magnetic resonance spectrum of compounds 1-25.
FIG. 11 shows a nuclear magnetic resonance spectrum of compounds 1-39.
FIG. 12 shows a nuclear magnetic resonance spectrum of compounds 1-42.
FIG. 13 shows a nuclear magnetic resonance spectrum of compounds 1-44.
FIG. 14 shows a nuclear magnetic resonance spectrum of compounds 1-45.
FIG. 15 shows a nuclear magnetic resonance spectrum of compounds 1-47.
FIG. 16 shows a nuclear magnetic resonance spectrum of compounds 1-48.
FIG. 17 shows a nuclear magnetic resonance spectrum of compounds 1-49.
FIG. 18 shows a nuclear magnetic resonance spectrum of compounds 1-50.
FIG. 19 shows a nuclear magnetic resonance spectrum of compounds 1-51.
FIG. 20 shows a nuclear magnetic resonance spectrum of compounds 1-52.
FIG. 21 shows a nuclear magnetic resonance spectrum of compounds 1-55.
FIG. 22 shows a nuclear magnetic resonance spectrum of compounds 1-56.
FIG. 23 is a graph showing fluorescence signals of different organs in mice 4 h after administration by intravenous injection of a sample of lipid nanoparticle TMF1 loaded with Luciferase mRNA.

As shown in the graph, the fluorescence signal is strongest in the spleen of the mice, and very weak in the liver. It is indicated that the expression of Luciferase mRNA in the spleen is higher than that in the liver.

FIG. 24 shows summary data of fluorescence intensity ratios for different lipid nanoparticles delivering Luciferase mRNA in the spleen and liver.

### DETAILED DESCRIPTION

### A. Chemical and Formulation Definitions

When used in the context of a chemical group, "hydrogen" refers to -H; "deuterium" refers to ²H or D; "hydroxyl" refers to -OH; "oxo" refers to =O; "carbonyl" refers to -C(=O)-; "carboxyl" refers to -C(=O)OH (also written as -COOH or -CO₂H); "halo" independently refers to -F, -Cl, - Br or -I; "amino" refers to -NH₂; "hydroxyamino" refers to -NHOH; "nitro" refers to -NO₂; imino refers to =NH; "cyano" refers to -CN; "isocyanate" refers to -N=C=O; "azido" refers to -N₃; in the context of a monovalent group, "phosphate" refers to -OP(O)(OH)₂ or a deprotonated form thereof; in the context of a divalent group, "phosphate" refers to -OP(O)(OH)O- or a deprotonated form thereof; "sulfydryl" refers to -SH; and "thio" refers to =S; "sulfonyl" refers to -S(O)₂-; "hydroxysulfonyl" refers to -S(O)₂OH; "sulfonamide" refers to -S(O)₂NH₂; and "sulfinyl" refers to -S(O)-. In the context of a chemical formula, the symbol "-" refers to a single bond, "=" refers to a double bond, and "≡" refers to a triple bond. The symbol "----" represents an optional bond, which is a single or double bond, if present. When drawn perpendicularly across a bond, the symbol " 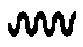 " indicates the point of attachment of the group. It should be noted that the point of attachment is generally identified only for larger groups in this manner to aid the reader in unequivocally identifying the point of attachment. The symbol " 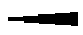 " refers to a single bond, wherein the group attached to the butt end of the wedge "emerges from the paper". The symbol " 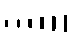 " refers to a single bond, wherein the group attached to the butt end of the wedge "goes into the paper". The symbol " 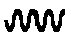 " refers to a single bond, wherein the geometry (e.g., E or Z) around the double bond is undefined. Thus, both options and combinations thereof are contemplated. Any undefined valence on an atom of a structure shown in the present application implicitly represents a hydrogen atom bonded to the atom. Bold dots on a carbon atom indicate that the hydrogen attached to the carbon is oriented out of the plane of the paper.

The term "alkyl" as used without the modifier "substituted" represents a monovalent saturated aliphatic group having a carbon atom as the point of attachment, having a linear or branched acyclic structure, and no atoms other than carbon and hydrogen. The groups -CH₃(Me), - CH₂CH₃(Et), -CH₂CH₂CH₃(n-Pr or propyl), -CH(CH₃)₂(i-Pr, iPr or isopropyl), - CH₂CH₂CH₂CH₃(n-Bu), -CH(CH₃)CH₂CH₃(sec-butyl), -CH₂CH(CH₃)₂(isobutyl), -C(CH₃)₃(*tert-*butyl, t-Bu or tBu) and -CH₂C(CH₃)₃(neopentyl) are non-limiting examples of alkyl. The term "dialkyl" as used without the modifier "substituted" represents a divalent saturated aliphatic group having 1 or 2 saturated carbon atoms as the point of attachment, having a linear or branched acyclic structure, no carbon-carbon double or triple bonds, and no atoms other than carbon and hydrogen. The groups -CH₂-(methylene), -CH₂CH₂-, -CH₂C(CH₃)₂CH₂- and -CH₂CH₂CH₂- are non-limiting examples of dialkyl. "Alkane" represents the class of compounds having formula H-R, wherein R is alkyl, and the term is as defined above. When any of these terms is used together with the modifier "substituted", one or more hydrogen atoms have been independently replaced by -OH, - F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, - S(O)₂OH or -S(O)₂NH₂. The following groups are non-limiting examples of substituted alkyl: - CH₂OH, -CH₂Cl, -CF₃, -CH₂CN, -CH₂C(O)OH, -CH₂C(O)OCH₃, -CH₂C(O)NH₂, -CH₂C(O)CH₃, -CH2OCH₃, -CH₂OC(O)CH₃, -CH₂NH₂, -CH₂N(CH₃)₂, and -CH₂CH₂Cl. The term "haloalkyl" is a subset of substituted alkyl, wherein hydrogen atom substitution is limited to halo (i.e., -F, -Cl, - Br, or -I), such that no atoms other than carbon, hydrogen and halogen are present. The group - CH₂Cl is one non-limiting example of haloalkyl. The term "fluoroalkyl" is a subset of substituted alkyl, wherein hydrogen atom substitution is limited to fluoro, such that no atoms other than carbon, hydrogen and fluorine are present. The groups -CH₂F, -CF₃ and -CH₂CF₃ are non-limiting examples of fluoroalkyl.

The term "alkenyl" as used without the modifier "substituted" represents a monovalent unsaturated aliphatic group having a carbon atom as the point of attachment, having a linear or branched acyclic structure, at least one non-aromatic carbon-carbon double bond, no carbon-carbon triple bonds, and no atoms other than carbon and hydrogen. Non-limiting examples include: - CH=CH₂(vinyl), -CH=CHCH₃, -CH=CHCH₂CH₃, -CH₂CH=CH₂(allyl), -CH₂CH=CHCH₃, and - CH=CHCH=CH₂. The term "alkenediyl" as used without the modifier "substituted" represents a divalent unsaturated aliphatic group having 2 carbon atoms as the point of attachment, having a linear or branched, linear or branched acyclic structure, at least one non-aromatic carbon-carbon double bond, no carbon-carbon triple bonds, and no atoms other than carbon and hydrogen. The groups -CH=CH-, -CH=C(CH₃)CH₂-, -CH=CHCH₂- and -CH₂CH=CHCH₂- are non-limiting examples of alkenediyl groups. It should be noted that although the alkenediyl group is aliphatic, once attached at both ends, it is not excluded that the group forms part of an aromatic structure. The terms "olefin" and "chain olefin" are synonymous and represent the class of compounds having formula H-R, wherein R is alkenyl, and the terms are as defined above. Similarly, the terms "terminal olefin" and "α-olefin" are synonymous and represent olefin having exactly one carbon-carbon double bond, wherein the bond is part of vinyl at the terminus of the molecule. When any of these terms is used together with the modifier "substituted", one or more hydrogen atoms have been independently replaced by -OH, -F, -Cl, -Br, -I, -NH₂, -NO₂, -CO₂H, -CO₂CH₃, -CN, -SH, - OCH₃, -OCH₂CH₃, -C(O)CH₃, -NHCH₃, -NHCH₂CH₃, -N(CH₃)₂, -C(O)NH₂, -C(O)NHCH₃, - C(O)N(CH₃)₂, -OC(O)CH₃, -NHC(O)CH₃, -S(O)₂OH or -S(O)₂NH₂. The groups -CH=CHF, - CH=CHCl and -CH=CHBr are non-limiting examples of substituted alkenyl.

The term "alkynyl" as used without the modifier "substituted" represents a monovalent unsaturated aliphatic group having a carbon atom as the point of attachment, having a linear or branched acyclic structure, at least one carbon-carbon triple bond, and no atoms other than carbon and hydrogen. The term alkynyl as used herein does not preclude the presence of one or more non-aromatic carbon-carbon double bonds. The groups -C=CH, -C≡CCH₃ and -CH₂C≡CCH₃ are non-limiting examples of alkynyl. "Alkyne" represents the class of compounds having formula H-R, wherein R is alkynyl. When any of these terms is used together with the modifier "substituted", one or more hydrogen atoms have been independently replaced by -OH, -F, -Cl, -Br, -I, -NH₂, - NO₂, -CO₂H, -CO₂CH₃, -CN, -SH, -OCH₃, -OCH₂CH₃, -C(O)CH₃, -NHCH₃, -NHCH₂CH₃, - N(CH₃)₂, -C(O)NH₂, -C(O)NHCH₃, -C(O)N(CH₃)₂, -OC(O)CH₃, -NHC(O)CH₃, -S(O)₂OH or - S(O)₂NH₂.

"Prodrug" refers to a compound, such as a therapeutic agent that can be converted into a biologically active compound under physiological conditions or by dissolution. The prodrug is generally rapidly converted *in vivo* to yield a parent compound, for example, by hydrolysis in blood. The prodrug compound generally has the advantage of solubility, histocompatibility, or delayed release in mammalian organisms. The term "prodrug" is also meant to include any covalently bonded carriers that release active compounds *in vivo* when the prodrug is administered to a mammalian subject. The prodrug includes a compound where hydroxyl, amino or sulfhydryl is bonded to any group that, when the prodrug is administered to the mammalian subject, cleaves to form free hydroxyl, free amino, or free sulfhydryl, respectively. Examples of the prodrug include, but are not limited to, acetate, formate and benzoate derivatives or amide derivatives of amine functional groups and the like.

"Pharmaceutically acceptable salt" refers to a salt of a compound of the present disclosure that is pharmaceutically acceptable as defined above and that has a desired pharmacological activity. Such salts include acid addition salts formed with the following acids: inorganic acids such as hydrochloric acid, hydrobromic acid, sulfuric acid, nitric acid, phosphoric acid, and the like; or organic acids such as 1,2-ethanedisulfonic acid, 2-hydroxyethanesulfonic acid, 2-naphthalenesulfonic acid, 3-phenylpropionic acid, 4,4'-methylene-bis(3-hydroxy-2-ene-1-methanoic acid), 4-methylbicyclo[2.2.2]oct-2-ene-1-methanoic acid, acetic acid, aliphatic monocarboxylic and dicarboxylic acids, aliphatic sulfuric acid, aromatic sulfuric acid, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, lauryl sulfuric acid, maleic acid, malic acid, malonic acid, mandelic acid, methanesulfonic acid, muconic acid, *o*-(4-hydroxybenzoyl)benzoic acid, oxalic acid, *p*-chlorobenzenesulfonic acid, phenyl-substituted alkanoic acid, propionic acid, *p*-toluenesulfonic acid, pyruvic acid, salicylic acid, stearic acid, succinic acid, tartaric acid, *tert*-butylacetic acid, trimethylacetic acid, and the like. The pharmaceutically acceptable salts further include base addition salts which may be formed when an acidic proton present is capable of reacting with an inorganic or organic base. The acceptable inorganic bases include sodium hydroxide, sodium carbonate, potassium hydroxide, aluminum hydroxide, and calcium hydroxide. The acceptable organic bases include ethanolamine, diethanolamine, triethanolamine, tromethamine, *N*-methylglucamine and the like. It should be recognized that the particular anion or cation forming part of any salt of the present disclosure is not critical so long as the salts as a whole are pharmacologically acceptable.

"Stereoisomers" or "optical isomers" are isomers of such a given compound, wherein the same atom is bonded to the same other atom; however, the three-dimensional configuration of those atoms is different. "Enantiomers" are stereoisomers of a given compound that are mirror images of each other as left and right hands. "Diastereoisomers" are stereoisomers of a given compound that are not enantiomers. Chiral molecules contain a chiral center (also referred to as a stereocenter or a stereogenic center), which is any point (although not necessarily an atom) in the molecule that carries multiple groups, such that the interchange of any 2 groups produces a stereoisomer. In organic compounds, the chiral center is typically a carbon, phosphorus, or sulfur atom, although other atoms may also be stereocenters in organic and inorganic compounds. A molecule may have multiple stereocenters, giving rise to many of its stereoisomers. In compounds whose stereoisomerism is due to tetrahedral stereogenic centers (e.g., tetrahedral carbon), it is assumed that the total number of possible stereoisomers does not exceed 2n, wherein n is the number of tetrahedral stereogenic centers. Molecules with symmetry often have a number that is smaller than the maximum possible number of stereoisomers. A 50:50 mixture of enantiomers is referred to as a racemic mixture. Alternatively, a mixture of enantiomers may be enantiomerically enriched such that one enantiomer is present in an amount greater than 50%. Generally, enantiomers and/or diastereomers may be resolved or separated using techniques known in the art. It is contemplated that for any stereogenic center or chiral axis for which stereochemistry has not been defined, the stereogenic center or chiral axis may exist in its R form, S form, or as a mixture of the R form and the S form (including racemic and non-racemic mixtures). As used herein, the phrase "substantially free of other stereoisomers" means that the composition contains 15% or less, more preferably 10% or less, even more preferably 5% or less, or most preferably 1% or less of another or more stereoisomers.

Deuterium (²H or D) is a stable and nonradioactive isotope of hydrogen, which has approximately twice the mass of hydrogen (H), the most common isotope of hydrogen.

"Prevention" or "preventing" includes: (1) inhibiting the onset of a disease in a subject or patient who may be at risk of and/or susceptible to the disease, but who has not yet experienced or exhibited any or all of the conditions or symptoms of the disease; and/or (2) slowing the onset of the conditions or symptoms of a disease in a subject or patient who may be at risk of and/or susceptible to the disease, but who has not experienced or exhibited any or all of the conditions or symptoms of the disease.

"Treatment" or "treating" includes: (1) inhibiting a disease (e.g., arresting the further development of the conditions and/or symptoms) in a subject or patient experiencing or exhibiting the conditions or symptoms of the disease, (2) ameliorating a disease (e.g., reversing the conditions and/or symptoms) in a subject or patient experiencing or exhibiting the conditions or symptoms of the disease, and/or (3) effecting any measurable mitigation of a disease in a subject or patient experiencing or exhibiting the conditions or symptoms of the disease.

"Protein", "polypeptide" or "peptide" refers to a polymer of amino acid residues, including a wide range of protein molecules such as cytokines, chemokines, interleukins, interferons, growth factors, coagulation factors, anticoagulants, blood factors, bone morphogenic proteins, immunoglobulins, and enzymes. Some non-limiting examples of therapeutic proteins include the following therapeutic proteins, or fragments, variants or derivatives thereof: therapeutic proteins for the treatment of metabolic or endocrine disorders, comprising acid sphingomyelinase, adipotide, agalsidase-β, alglucosidase, α-galactosidase A, α-glucosidase, α-L-iduronidase, α-N-acetylglucosidase, amphiregulin, angiogenin (Ang1, Ang2, Ang3, Ang4, ANGPTL2, ANGPTL3, ANGPTL4, ANGPTL5, ANGPTL6, ANGPTL7), β-cellulin, β-glucuronidase, bone morphogenetic proteins BMPs (BMP1, BMP2, BMP3, BMP4, BMP5, BMP6, BMP7, BMP8a, BMP8b, BMP10, BMP15), CLN6 protein, epidermal growth factor (EGF), epigen, epiregulin, fibroblast growth factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, FGF-23), galsulphase, ghrelin, glucocerebrosidase, GM-CSF, heparin-binding EGF-like growth factor (HB-EGF), hepatocyte growth factor HGF, hepcidin, human albumin, increased albumin loss, idursulfase (iduronate-2-sulfatase), integrins αVβ3, αVβ5 and α5β1, iduronate sulfatase, laronidase, N-acetylgalactosamine-4-sulfatase (rhASB; galsulase, arylsulfatase A (ARSA), arylsulfatase B (ARSB)), N-acetylglucosamine-6-sulfatase, nerve growth factor (NGF, brain-derived neurotrophic factor (BDNF), neurotrophin-3 (NT-3), and neurotrophin-4/5(NT-4/5), neuregulin (NRG1, NRG2, NRG3, NRG4), neuropilin (NRP-1, NRP-2), myostatin, platelet-derived growth factor (PDGF (PDFF-A, PDGF-B, PDGF-C, PDGF-D), TGF β receptor (endothelial factor, TGF-β1 receptor, TGF-β2 receptor, TGF-β3 receptor), thrombopoietin (THPO) (megakaryocyte growth and development factor (MGDF)), transforming growth factor (TGF(TGF-a, TGF-β (TGFβ1, TGFβ2, and TGFβ3))), VEGF (VEGF-A, VEGF-B, VEGF-C, VEGF-D, VEGF-E, VEGF-F, and PIGF), nesiritide, trypsin, adrenocorticotropic hormone (ACTH), atrial natriuretic peptide (ANP), cholecystokinin, gastrin, leptin, oxytocin, somatostatin, vasopressin (antidiuretic hormone), calcitonin, exenatide, growth hormone (GH), auxin, insulin, insulin-like growth factor 1IGF-1, mecasermin rinfabate, IGF-1 analogs, mecasermin, IGF-1 analogs, pegvisomant, pramlintide, teriparatide (human parathyroid hormone residue 1-34), becaplermin, dibotermin-α (bone morphogenetic protein 2), histrelin acetate (gonadotropin releasing hormone; GnRH), octreotide, and palifermin (keratinocyte growth factor; KGF); therapeutic proteins for the treatment of hematologic disorder, circulatory disease, respiratory disease, cancer or neoplastic disease, infectious disease or immunodeficiency, comprising alteplase (tissue plasminogen activator; tPA), anistreplase, antithrombin III (AT-III), bivalirudin, darbepoetin-α, drotrecogin-α (activated protein C, erythropoietin, epoetin-α, hemoglobin-promoting auxin, erythropoietin, factor IX, factor VIIa, factor VIII, lepirudin, protein C concentrate, reteplase (deletion mutant protein of tPA), streptokinase, tenecteplase, urokinase, angiostatin, anti-CD22 immunotoxin, dinleukin-toxin linker, immunocyanin, MPS (metallopanstimulin), aflibercept, endostatin, collagenase, human deoxyribonuclease I, streptodornase, hyaluronidase, papain, L-asparaginase, Peg-asparaginase, rasburicase, human chronic gonadotropin (HCG), human follicle stimulating hormone (FSH), luteinizing hormone-α, prolactin, α-1-protease inhibitors, lactase, pancreatin (lipase, amylase, protease), adenosine deaminase (pegademase bovine, PEG-ADA), abatacept, alefacept, anakinra, etanercept, interleukin-1 (IL-1) receptor antagonist, thymosin, TNF-α antagonist, enfuvirtide, and thymosin α1; therapeutic proteins selected from adjuvants or immunostimulatory proteins, comprising: human adjuvant proteins, in particular the pattern recognition receptors TLR1, TLR2, TLR3, TLR4, TLR5, TLR6, TLR7, TLR8, TLR9, TLR10, TLR11; NOD1, NOD2, NOD3, NOD4, NOD5, NALP1, NALP2, NALP3, NALP4, NALP5, NALP6, NALP7, NALP8, NALP9, NALP10, NALP11, NALP12, NALP13, NALP14, 1IPAF, NAIP, CIITA, RIG-I, MDA5, and LGP2, signal transducers for TLR signaling, comprising adaptor proteins, including, for example, Trif and Cardif; small-GTPase signaling components (RhoA, Ras, Rac1, Cdc42, Rab, etc.), PIP signaling components (PI3K, Src-kinase, etc.), MyD88-dependent signaling components (MyD88, IRAK1, IRAK2, IRAK4, TIRAP, TRAF6, etc.), MyD88-independent signaling components (TICAM1, TICAM2, TRAF6, TBK1, IRF3, TAK1, IRAK1, etc.); activated kinases, including, for example, Akt, MEKK1, MKK1, MKK3, MKK4, MKK6, MKK7, ERK1, ERK2, GSK3, PKC kinase, PKD kinase, GSK3 kinase, JNK, p38MAPK, TAK1, IKK, and TAK1; activated transcription factors, including, for example, NF-κB, c-Fos, c-Jun, c-Myc, CREB, AP-1, Elk-1, ATF2, IRF-3, IRF-7, heat shock proteins such as HSP10, HSP60, HSP65, HSP70, HSP75 and HSP90, gp96, fibrinogen, the TypIII repeat addition domain A of fibronectin; or components of the complement system, including C1q, MBL, C1r, C1s, C2b, Bb, D, MASP-1, MASP-2, C4b, C3b, C5a, C3a, C4a, C5b, C6, C7, C8, C9, CR1, CR2, CR3, CR4, C1qR, C1INH, C4bp, MCP, DAF, H, I, P, and CD59, or induced target genes including, for example, β-defensin, cell surface protein; or human adjuvant proteins, including trif, flt-3 ligand, Gp96 or fibronectin, cytokines that induce or enhance the innate immune response, including IL-1α, IL1β, IL-2, IL-6, IL-7, IL-8, IL-9, IL-12, IL-13, IL-15, IL-16, IL-17, IL-18, IL-21, IL-23, TNF-α, IFN-α, IFN-β, IFN-γ, GM-CSF, G-CSF, M-CSF; chemokines, including IL-8, IP-10, MCP-1, MIP-1α, RANTES, eotaxin, CCL21; cytokines released by macrophages, including IL-1, IL-6, IL-8, IL-12 and TNF-α; and IL-1R1 and IL-1α; bacterial (adjuvant) proteins, in particular bacterial heat shock proteins or chaperones, including Hsp60, Hsp70, Hsp90, Hsp100; OmpA (outer membrane protein) from gram-negative bacteria; bacterial porins, including OmpF; bacterial toxins, including pertussis toxin (PT) from Bordetella pertussis, pertussis adenylate cyclase toxins CyaA and CyaC from Bordetella pertussis, PT-9K/129G mutant from pertussis toxin, tetanus toxin, cholera toxin (CT), cholera toxin B-subunit, CTK63 mutant from cholera toxin, CTE112K mutant from CT, Escherichia coli heat-labile enterotoxin (LT), B subunit from heat-labile enterotoxin (LTB), Escherichia coli heat-labile enterotoxin mutants with reduced toxicity, including LTK63 and LTR72; phenol soluble modulins; neutrophil activating protein (HP-NAP) from Helicobacter pylori; surfactant protein D; outer surface protein A lipoproteins from Borrelia burgdorferi, and Ag38(38kDa antigen) from Mycobacterium tuberculosis; proteins from bacterial pili; enterotoxin CT of Vibrio cholerae, pilin from pili of gram-negative bacteria, and surfactant protein A and bacterial flagellin, protozoan (adjuvant) proteins, in particular Tc52 from Trypanosoma cruzi, PFTG from Trypanosoma gondii, protozoan heat shock proteins, LeIF from Leishmania spp., actin-inhibiting protein-like protein from Toxoplasma gondii, viral (adjuvant) proteins, in particular respiratory syncytial virus fusion glycoprotein (F-protein), envelope proteins from MMT virus, murine leukemia virus proteins, hemagglutinin protein of wild-type measles virus, fungal (adjuvant) proteins, in particular fungal immunomodulatory protein (FIP; LZ-8); and keyhole limpet hemocyanin (KLH), OspA; therapeutic proteins for hormone replacement therapy, in particular estrogens, progestin or progesterone, and testosterone; therapeutic proteins for reprogramming somatic cells to pluripotent or totipotent stem cells, in particular Oct-3/4, the Sox gene family (Sox1, Sox2, Sox3 and Sox15), the Klf family (Klf1, Klf2, Klf4 and Klf5), the Myc family (c-myc, L-myc and N-myc), Nanog and LIN28; and therapeutic antibodies selected from antibodies for the treatment of cancer or a tumor disease, in particular 131T-tositumomab, 3F8, 8H9, abagovomab, adecatumumab, afutuzumab, alacizumab pegol, alemtuzumab, amatuximab, AME-133v, AMG102, anatumomab, apolizumab, basiliximab, bectumomab, belimumab, bevacizumab, bivatuzumab-DM1, blinatumomab, brentuximab vedotin, cantuzumab, cantuzumab mertansine, cantuzumab ravtansine, capromab pendetide, carlumab, catumaxomab, cetuximab, citatuzumab bogatox, cixutumumab, clivatuzumab tetraxetan, CNTO328, CNTO 95, conatumumab, dacetuzumab, dalotuzumab, denosumab, denosumab, drozitumab, ecromeximab, edrecolomab, elotuzumab, elsilimomab, enavatuzumab, ensituximab, epratuzumab, ertumaxomab, etaracizumab, farletuzumab, FBTA05, ficlatuzumab, figitumumab, flanvotumab, galiximab, galiximab, ganitumab, GC1008, gemtuzumab, gemtuzumab ozogamicin, girentuximab, glemnbatumumab vedotin, GS6624, HuC242-DM4, HuHMFG1, HuN901-DM1, zevalin, icrucumab, ID09C3, indatuximab ravtansine, inotuzumab ozogamicin, intetumumab, ipilimumab, iratumumab, labetuzumab, lexatumumab, lintuzumab, lorvotuzumab mertansine, lucatumumab, lumiliximab, mapatumumab, matuzumab, MDX-060, MEDI 522, mitumomab, mogamulizumab, MORab-003, MORab-009, moxetumomab pasudotox, MT103, nacolomab, naptumomab estafenatox, narnatumab, necitumumab, nimotuzumab, olaratumab, onartuzumab, oportuzumab monatox, oregovomab, PAM4, panitumumab, patritumab, pemtumomab, pertuzumab, priliximab, racotumomab, radretumab, ramucirumab, rilotumumab, rituximab, robatumumab, samalizumab, SGN-30, SGN-40, sibrotuzumab, siltuximab, tabalumab, tacatuzumab tetraxetan, taplitumomab paptox, tenatumomab, teprotumumab, TGN1412, ticilimumab (=tremelimumab), tigatuzumab, TNX-650, tositumomab, trastuzumab, TRBS07, tremelimumab, TRU-016, tucotuzumab celmoleukin, ublituximab, urelumab, vituzumab (IMMU-106), volociximab, Votumumab, WX-G250, zalutumumab and natalizumab; antibodies for the treatment of immune disorders, in particular efalizumab, epratuzumab, etrolizumab, fontolizumab, Ixekizumab, mepolizumab, milatuzumab, pooled immunoglobulins, priliximab, rituximab, rontalizumab, ruplizumab, sarilumab, vedolizumab, visilizumab, resilizumab, adalimumab, aselizumab, atinumab, atlizumab, belimumab, besilesomab, BMS-945429, briakinumab, brodalumab, canakinumab, certolizumab pegol, erlizumab, fezakinumab, golimumab, gomiliximab, infliximab, mavrilizumab, natalizumab, ocrelizumab, odulimomab, ofatumumab, ozoralizumab, pexelizumab, rovelizumab, SBI-087, secukinumab, sirukumab, talizumab, tocilizumab, toralizumab, TRU-015, TRU-016, ustekinumab, vepalimomab, zolimomab aritox, sifalimumab, lumiliximab, and Rho(D) immunoglobulin; antibodies for the treatment of infectious diseases, in particular, afimomab, CR6261, edobacomab, efungumab, exbivirumab, felvizumab, foravirumab, ibalizumab, libivirumab, motavizumab, nebacumab, tuvirumab, urtoxazumab, bavituximab, pagibaximab, palivizumab, panobacumab, PRO140, rafivirumab, raxibacumab, regavirumab, sevirumab, suvizumab, and tildrakizumab; antibodies for the treatment of hematological disorders, in particular abciximab, adalimumab, eculizumab, mepolizumab and milatuzumab; antibodies for immunomodulation, in particular anti-thymocyte globulin, basiliximab, siplizumab, daclizumab, gavilimomab, inolimomab, muromonab-CD3, odulimomab and siplizumab; antibodies for the treatment of diabetes, in particular gevokizumab, otelixizumab and teplizumab; antibodies for the treatment of Alzheimer's disease, in particular bapineuzumab, crenezumab, gantenerumab, ponezumab, R1450 and solanezumab; antibodies for the treatment of asthma, in particular benralizumab, enokizumab, keliximab, lebrikizumab, omalizumab, oxelumab, pascolizumab and tralokinumab; antibodies for the treatment of various disorders, in particular blosozumab, CaroRx, fresolimumab, fulranumab, romosozumab, stamulumab, tanezumab, and ranibizumab; erythropoietin (EPO), granulocyte colony stimulating factor (G-CSF), α-galactosidase A, α-L-iduronidase, thyrotropin-α, N-acetylgalactosamine-4-sulfatase (rhASB), pulmozyme, tissue plasminogen activator (TPA) activase, glucocerebrosidase, interferon (IF)β-1α, interferonβ-1b, interferon-γ, interferon-α, TNF-α, IL-1 to IL-36, human growth hormone (rHGH), human insulin (BHI), human chorionic gonadotropin-α, darbepoetin-α, follicle stimulating hormone (FSH) and factor VIII, antibodies and antibody derivatives such as bispecific antibodies, multispecific antibodies, ADCs, etc., for the treatment of a variety of disorders. Peptide is a compound formed by connecting α-amino acids with peptide bonds, and it is also an intermediate product of protein hydrolysis. Generally, the number of amino acids contained in a peptide is two to nine, and peptides are variously referred to according to the number of amino acids in the peptide: dipeptide, tripeptide, tetrapeptide, pentapeptide, etc., respectively. Peptides consisting of three or more amino acid molecules are known as polypeptides, which have a molecular weight of less than 10,000 Da, being capable of passing through a semipermeable membrane without being precipitated by trichloroacetic acid and ammonium sulfate. In some literature, peptides consisting of 2-10 amino acids are also known as oligopeptides (small molecule peptides); peptides consisting of 10-50 amino acids are known as polypeptides; peptides consisting of 50 or more amino acids are known as proteins, in other words, proteins are sometimes known as polypeptides.

"Small molecule compounds" include 7-methoxypteridine, 7-methylpteridine, abacavir, abafungin, abarelix, acebutolol, acenaphthene, acetaminophen, acetanilide, acetazolamide, acetohexamide, etretinate, acrivastine, adenine, adenosine, alatrafloxacin, albendazole, albuterol, alclofenac, aldesleukin, alemtuzumab, alfuzosin, alitretinoin, allobarbital, allopurinol, all-trans retinoic acid (ATRA), aloxiprin, alprazolam, alprenolol, altretamine, amifostine, amiloride, aminoglutethimide, aminophenazone, amiodarone hydrochloride, amitriptyline, amlodipine, amobarbital, amodiaquine, amoxapine, amphetamine, amphotericin, amphotericin B, ampicillin, amprenavir, amsacrine, amyl nitrate, amobarbital, anastrozole, anrinone, anthracene, anthracycline antibiotics, aprobarbital, arsenic trioxide, asparaginase, aspirin, astemizole, atenolol, atorvastatin, atovaquone, atrazine, atropine, atropine azathioprine, auranofin, azacitidine, azapropazone, azathioprine, azintamide, azithromycin, aztreonam, baclofen, barbital, live BCG vaccine, beclamide, beclomethasone, bendroflumethiazide, benezepril, benidipine, benorilate, benperidol, bentazepam, benzamide, benzanthracene, benzathine penicillin, benzhexol hydrochloride, benznidazole, benzodiazacyclo hepta-triene, benzoic acid, bephenium hydroxynaphthoate, betamethasone, bevacizumab (atorvastatin), bexarotene, bezafibrate, bicalutamide, bifonazole, biperiden, bisacodyl, bisantrene, bleomycin, bleomycin, bortezomib, brinzolamide, bromazepam, bromocriptine mesylate, bromperidol, brotizolam, budesonide, bumetanide, bupropion, busulfan, butalbital, butamben, butenafine hydrochloride, butobarbital, butobarbital (n-butobarbital), butoconazole, butoconazole nitrate, butyl p-hydroxybenzoate, caffeine, calcidiol, calciprotriene, calcitriol, calusterone, cambendazol, camphor, camptothecin, camptothecin analogue, candesartan, capecitabine, capsaicin, captopril, carbamazepine, carbimazole, carbofuran, carboplatin, carbromal, carimazole, carmustine, cefamandole, cephazolin, cefixime, ceftazidime, cefuroxime axetil, celecoxib, cefradine, cerivastatin, cetirizine, cetuximab, chlorambucil, chloramphenicol, chlordiazepoxide, chlormethiazole, chloroquine, chlorothiazide, chlorpheniramine, chlorproguanil hydrochloride, chlorpromazine, chlorpropamide, chlorprothixene, chlorpyrifos, chlortetracycline, chlorthalidone, chlorzoxazone, cholecalciferol, cilostazol, cimetidine, cinnarizine, cinoxacin, ciprofibrate, ciprofloxacin hydrochloride, cisapride, cisplatin, citalopram, cladribine, clarithromycin, clemastine fumarate, clioquinol, clobazam, clofarabine, clofazimine, clofibrate, clomifene citrate, clomipramine, clonazepam, clopidogrel, clotiazepam, clotrimazole, clotrimazole, cloxacillin, clozapine, cocaine, codeine, colchicine, colistin, conjugated estrogen, corticosterone, cortisone, cortisone acetate, cyclizine, cyclobarbital, cyclobenzaprine, cyclobutane-spirobarbiturate, cycloethane-spirobarbiturate, cycloheptane-spirobarbiturate, cyclohexane-spirobarbiturate, cyclopentane-spirobarbiturate, cyclophosphamide, cyclopropane-spirobarbiturate, cycloserine, ciclosporin, cyproheptadine, cyproheptadine hydrochloride, cytarabine, cytosine, dacarbazine, dactinomycin, danazol, danthron, dantrolene sodium, dapsone, darbepoetin alfa, darodipine, daunorubicin, decoquinate, dehydroepiandrosterone, delavirdine, demeclocycline, denileukin, deoxycorticosterone, desoximetasone, dexamethasone, dextroamphetamine, dextrochlorpheniramine, dexfenfluramine, dexrazoxane, dextropropoxyphene, heroin, amidotrizoic acid, diazepam, diazoxide, dichlorophene, dichlorprop, diclofenac, dicoumarin, didanosine, diflunisal, digitoxin, digoxin, dihydrocodeine, dihydroequilin, dihydroergotamine mesilate, diiodohydroxyquinoline, diltiazem hydrochloride, diloxanide furoate, dimenhydrinate, dimorphoramine, dinitolmide, diosgenin, diphenoxylate hydrochloride, biphenyl, dipyridamole, dirithromycin, disopyramide, disulfiram, diuron, docetaxel, domperidone, donepezil, doxazosin, doxazosin hydrochloride, doxorubicin (neutral), doxorubicin hydrochloride, doxycycline, dromostanolone propionate, droperidol, dyphylline, echinocandin, econazole, econazole nitrate, efavirenz, ellipticine, enalapril, enlimomab, enoximone, epinephrine, an epipodophyllotoxin derivative, epirubicin, epoetin alfa, eposartan, equilenin, equilin, ergocalciferol, ergotamine tartrate, erlotinib, erythromycin, estradiol, estramustine, estriol, estrone, ethacrvnic acid, ethambutol, ethinamate, ethionamide, ethopropazine hydrochloride, ethyl 4-aminobenzoate (benzocaine), ethyl p-hydroxybenzoate, ethinyl estradiol, etodolac, etomidate, etoposide, etretinate, exemestane, felbamate, felodipine, fenbendazole, fenbuconazole, fenbufen, fenchlorphos, fenclofenac, fenfluramine, fenofibrate, fenoldepam, fenoprofen calcium, fenoxycarb, fenpiclonil, fentanyl, fenticonazole, fexofenadine, filgrastim, finasteride, flecainide acetate, floxuridine, fludarabine, fluconazole, fluconazole, flucytosine, fludioxonil, fludrocortisone, fludrocortisone acetate, flufenamic acid, flunanisone, flunarizine hydrochloride, flunisolide, flunitrazepam, fluocortolone, fluometuron, fluorene, fluorouracil, fluoxetine hydrochloride, fluoxymesterone, flupentixol decanoate, fluphenthixol decanoate, flurazepam, flurbiprofen, fluticasone propionate, fluvastatin, folic acid, fosinopril, fosphenytoin sodium, frovatriptan, furosemide, fulvestrant, furazolidone, gabapentin, G-BHC (lindane), gefitinib, gemcitabine, gemfibrozil, gemtuzumab, glafenine, glibenclamide, gliclazide, glimepiride, glipizide, glutethimide, glibenclamide, glyceryl trinitrate (nitroglycerin), goserelin acetate, grepafloxacin, griseofulvin, guaifenesin, guanabenz acetate, guanine, halofantrine hydrochloride, haloperidol, hydrochlorothiazide, heptabarbital, heroin, hesperetin, hexachlorobenzene, hexethal, histrelin acetate, hydrocortisone, hydroflumethiazide, hydroxyurea, scopolamine, hypoxanthine, ibritumomab, ibuprofen, idarubicin, allylbutylbarbituric acid, ifosfamide, ihydroequilenin, imatinib mesylate, imipenem, indapamide, indinavir, indomethacin, indoprofen, interferon alpha-2a, interferon alpha-2b, iodamide, iopanoic acid, iprodione, irbesartan, irinotecan, isavuconazole, isocarboxazid, isoconazole, isoguanine, isoniazid, isopropyl barbiturate, isoproturon, isosorbide dinitrate, isosorbide mononitrate, isradipine, itraconazole, itraconazole, itraconazole (Itra), ivermectin, ketoconazole, ketoprofen, ketorolac, khellin, labetalol, lamivudine, lamotrigine, lanatoside C, lanosprazole, L-DOPA, leflunomide, lenalidomide, letrozole, folinic acid, leuprolide acetate, levamisole, levofloxacin, lidocaine, linuron, lisinopril, lomefloxacin, lomustine, loperamide, loratadine, lorazepam, lorefloxacin, lormetazepam, losartan mesylate, lovastatin, lisuride maleate, maprotiline hydrochloride, mazindol, mebendazole, meclozine hydrochloride, meclofenamic acid, medazepam, metildigoxin, medroxyprogesterone acetate, mefenamic acid, mefloquine hydrochloride, megestrol acetate, melphalan, mepenzolate bromide, meprobamate, meptazinol, purinethol, mesalazine, mesna, mesoridazine, mestranol, amidone, methaqualone, methocarbamol, mephenytoin, methotrexate, methoxsalen, methsuximide, methyclothiazide, methylphenidate, mephobarbital, methylparaben, methylprednisolone, methyltestosterone, methyprylon, methysergide maleate, metoclopramide, metolazone, metoprolol, metronidazole, mianserin hydrochloride, miconazole, midazolam, mifepristone, migltol, minocycline, minoxidil, mitomycin C, mitotane, mitoxantrone, mycophenolate mofetil, molindone, montelukast, morphine, moxifloxacin hydrochloride, nabumetone, nadolol, nalbuphine, nalidixic acid, nandrolone, tetracene, naphthalene, naproxen, naratriptan hydrochloride, natamycin, nelarabine, nelfinavir, nevirapine, nicardipine hydrochloride, niacinamide, niacin, acenocoumarol, nifedipine, nilutamide, nimodipine, nimorazole, nisodipine, nitrazepam, furantoin, furacillin, nizatidine, romosozumab, norethindrone, norfloxacin, norgestrel, nortriptyline hydrochloride, nystatin, estradiol, ofloxacin, olanzapine, omeprazole, omoconazole, ondansetron hydrochloride, oprelvekin, omidazole, oxaliplatin, oxamniquine, oxantel pamoate, oxaprozin, oxatomide, oxazepam, oxcarbazepine, oxfendazole, oxiconazole, oxprenolol, oxyphenbutazone, oxyphencyclimine hydrochloride, paclitaxel, palifermin, pamidronate, p-aminosalicylic acid, pantoprazole, paramethadione, paroxetine hydrochloride, pegademase, pegaspargase, pegfilgrastim, pemetrexed disodium, penicillamine, pentaerythritol tetranitrate, pentazocin, pentazocin, pentobarbital, pentobarbitone, pentostatin, pentoxifylline, perphenazine, phenazine pimozide, perylene, phenacemide, phenacetin, phenanthrene, phenindione, phenobarbitone, phenobarbital, phenolphthalein, phenoxybenzamine, phenoxybenzamine hydrochloride, phenoxymethylpenicillin, phensuximide, phenylbutazone, phenytoin, pindolol, pioglitazone, pipobroman, piroxicam, pizotifen maleate, platinum compounds, mithramycin, polyenoid, polymyxin B, porfimer sodium, posaconazole (Posa), pramipexole, prasterone, pravastatin, praziquantel, prazosin, prazosin hydrochloride, prednisolone, prednisone, primidone, probarbital, probenecid, probucol, procarbazine, prochlorperazine, progesterone, guanatol hydrochloride, promethazine, propofol, propoxur, propranolol, propyl paraben, propylthiouracil, prostaglandin, pseudoephedrine, pteridine-2-methyl-thiol, pteridine-2-thiol, pteridine-4-methyl-thiol, pteridine-4-thiol, pteridine-7-methyl-thiol, pteridine-7-thiol, pyrantel pamoate, pyrazinamide, pyrene, pyridostigmine, pyrimethamine, quetiapine, mepacrine, quinapril, quinidine, quinidine sulfate, quinine, quinine sulfate, rabeprazole sodium, ranitidine hydrochloride, rasburicase, ravuconazole, repaglinide, reposamal, reserpine, tretinoin, rifabutin, rifampin, rifapentine, rimexolone, risperidone, ritonavir, rituximab, rizatriptan benzoate, rofecoxib, ropinirole hydrochloride, rosiglitazone, saccharin, salbutamol, salicylamide, salicylic acid, saquinavir, sargramostim, butabarbital, seco-barbital, sertaconazole, sertindole, sertraline hydrochloride, simvastatin, sirolimus, sorafenib, sparfloxacin, spiramycin, spironolactone, dihydrotestosterone, stanozolol, stavudine, diethylstilbestrol, streptozocin, strychnine, sulconazole, sulconazole nitrate, sulfacetamide, sulfadiazine, sulfamerazine, sulfamethazine, sulfamethoxazole, sulfanilamide, sulfathiazole, sulindac, sulphabenzamide, sulphacetamide, sulphadiazine, sulphadoxine, sulfisoxazole, sulphamerazine, sulpha-methoxazole, sulphapyridine, sulfasalazine, sulfinpyrazone, sulpiride, sulthiame, sumatriptan succinate, sunitinib maleate, tacrine, tacrolimus, talbutal, tamoxifen citrate, tamulosin, targretin, taxane, tazarotene, telmisartan, temazepam, temozolomide, teniposide, tenoxicam, terazosin, terazosin hydrochloride, terbinafine hydrochloride, terbutaline sulfate, terconazole, terfenadine, testolactone, testosterone, tetracycline, tetrahydrocannabinol, tetroxoprim, thalidomide, thebaine, theobromine, theophylline, thiabendazole, thiamphenicol, thioguanine, thioridazine, thiotepa, thotoin, thymine, tiagabine hydrochloride, tibolone, ticlopidine, tinidazole, tioconazole, tirofiban, tizanidine hydrochloride, tolazamide, tolbutamide, tolcapone, topiramate, topotecan, toremifene, tositumomab, tramadol, trastuzumab, trazodone hydrochloride, tretinoin, triamcinolone, triamterene, triazolam, triazoles, triflupromazine, trimethoprim, trimipramine maleate, triphenylene, troglitazone, tromethamine, tropicamide, trovafloxacin, tybamate, ubidecarenone (coenzyme Q10), undecylenic acid, uracil, uramustine, uric acid, valproic acid, valrubicin, valsartan, vancomycin, venlafaxine hydrochloride, vigabatrin, vinbarbital, vinblastine, vincristine, vinorelbine, voriconazole, xanthine, zafirlukast, zidovudine, zileuton, zoledronate, zoledronic acid, zolmitriptan, zolpidem and zopiclone.

Antigens (abbreviated as Ag) refer to substances which may cause antibody production, and may be any substances capable of inducing immune response. Foreign molecules may be recognized by Immunoglobulin on B cells, or treated by antigen presenting cells, combined with a major histocompatibility complex to obtain a complex to reactivate T cells and trigger a continuous immune response.

An antigenic epitope, also known as an antigenic determinant, may be a special chemical group which consists of a continuous sequence (a protein primary structure) or a discontinuous protein three-dimensional structure and determines antigenicity. Most of antigenic epitopes are present on the surface of antigenic materials, and some of the antigenic epitopes are present inside the antigenic materials, and are exposed after treatment with enzymes or other means. One natural antigenic substance may have a variety of and a plurality of determinants. The larger the molecule of an antigen, the greater the number of the epitopes.

Tumor-associated antigens (TAA) refer to antigen molecules present on tumor cells or normal cells, including: embryonic proteins, glycoprotein antigens, squamous cell antigens, and the like, and are commonly used for clinical diagnosis of tumors. The tumor-associated antigens are not specific to the tumor cells, may be synthesized in minute amounts on the normal cells, are highly expressed when the tumor cells proliferate, and are therefore referred to as "associated antigens". Tumors derived from the same tissue type have the same tumor-associated antigens in different individuals.

Pathogenic microorganisms refer to microorganisms, or called pathogens, which may invade human bodies and cause infections and even infectious diseases. Among the pathogens, the most harmful are bacteria and viruses. The pathogenic microorganisms include prion body, fungus, bacteria, spirochete, mycoplasma, rickettsia, chlamydia, and virus. Pathogenic microorganism antigens refers to substances which are derived from the pathogens and have a function of triggering an immune response.

"Lipid encapsulation" refers to lipid nanoparticles which provide an active or a therapeutic agent (e.g., a nucleic acid (e.g., mRNA)), and comprise full encapsulation and partial encapsulation, or both. In some embodiments, the nucleic acid (e.g., mRNA) is completely encapsulated in the lipid nanoparticle.

In various embodiments, the lipid nanoparticle has an average diameter of: about 90 nm to about 600 nm, about 100 nm to about 550 nm, about 150 nm to about 500 nm, about 200 nm to about 400 nm, about 250 nm to about 300 nm, and about 200 nm to about 300 nm, and is substantially non-toxic.

### B Guided on-target lipid delivery (GOLD) lipid

In some aspects, the present disclosure comprises one or more guided on-target delivery (GOLD) lipids that result in the selective delivery of a lipid nanoparticle to a specific organ. The guided on-target delivery (GOLD) lipid may be one or more of an ionizable anionic steroid and/or an ionizable anionic polymer conjugated lipid. In some embodiments, the guided on-target delivery (GOLD) lipid is present in a composition in a molar ratio of about 5%, 10%, 15%, 20%, 22%, 24%, 26%, 28%, 30%, 32%, 34%, 36%, 38%, 40%, 45%, to about 50%, or any range that may be derived therefrom. In some embodiments, the guided on-target delivery (GOLD) lipid may be present in a molar ratio of about 5% to about 50%, about 5% to about 45%, about 10% to about 40%, about 20% to about 35%, or about 20% to about 30%.

In some embodiments, the GOLD compound may be an ionizable anionic steroid. In some embodiments, the present disclosure provides one or more lipids having one or more steroids and ionizable anionic groups. The ionizable anionic steroid may contain a group having a negative charge (regardless of pH). The ionizable anionic groups which can be used in the ionizable anionic steroid are carboxylate groups, sulfonate groups, or phosphate groups. The ionizable anionic group may be a carboxylate group. The carboxylate group may be a compound having a negative charge at a pH value of less than 5, 6, 7, 8, 9, 10, 11, 12, 13, or 14. The steroid and the steroid derivative may be a class of compounds having a tetracyclic 17-carbon cyclic structure, which may further contain one or more substitutions, wherein the substitutions include alkyl, alkoxy, hydroxy, oxo and acyl or double bonds between two or more carbon atoms. In some aspects, the ring structure of the steroid comprises three fused cyclohexyl rings and a fused cyclopentyl ring.

In some embodiments, the GOLD compound may be an ionizable anionic polymer conjugated lipid. Lipids are small molecules with two or more C6-C24 alkyl or alkenyl or alkynyl chains. In some aspects, the present disclosure provides one or more lipids having one or more polymer conjugated components and ionizable anionic groups. The ionizable anionic groups which can be used in the ionizable anionic polymer conjugated lipid are carboxylate groups, sulfonate groups, or phosphate groups. The ionizable anionic group may be a carboxylate group. The carboxylate group may be a compound having a negative charge at a pH value of less than 5, 6, 7, 8, 9, 10, 11, 12, 13, or 14. The polymer conjugated component may be a compound comprising a PEG chain connected to a glycerol group or containing one or more C6-C24 long chain alkyl or alkenyl groups or C6-C24 fatty acid groups connected to a linker group via a PEG chain.

### C Helper lipid

In some aspects of the present disclosure, a composition containing one or more helper lipids is mixed with a cationic lipid and a guided on-target delivery lipid to produce a lipid nanoparticle. In some embodiments, the cationic lipid and the guided on-target delivery lipid are mixed with 1, 2, 3, 4, or 5 different types of helper lipids. It is contemplated that the cationic lipid and the guided on-target delivery lipid may be mixed with a single type of a variety of different helper lipids. In some embodiments, the helper lipid comprises, but is not limited to, one or more of a phospholipid, a steroid or a steroid derivative, a polymer conjugated lipid and a modified lipid.

"Phospholipid" is any lipid comprising a phosphoester group. In some embodiments, the phospholipid is a structure containing one or two long chain C6-C24 alkyl or alkenyl groups, glycerol or sphingosine, one or two phosphoester groups, and optionally a small organic molecule. In some embodiments, the small organic molecule is an amino acid, sugar, or an amino-substituted alkoxy group, such as choline or ethanolamine. In some embodiments, the phospholipid is phosphatidyl choline. In some embodiments, the phospholipid is DOPE, DSPC, DPPC, DMPC, DOPC, POPC or SM. In some embodiments, the phospholipid is distearoyl phosphatidylcholine or dioleoyl phosphatidylethanolamine.

"Steroid and the steroid derivative" comprises any steroid or steroid derivative. As used herein, in some embodiments, the term "steroid" is a class of compounds having a tetracyclic 17-carbon cyclic structure, which may further contain one or more substitutions, wherein the substitutions include alkyl, alkoxy, hydroxy, oxo and acyl or double bonds between two or more carbon atoms. In one aspect, the ring structure of the steroid comprises three fused cyclohexyl rings and a fused cyclopentyl ring. In some embodiments, the steroid derivative comprises the above ring structure with one or more non-alkyl substitutions. In some embodiments, the steroid or the steroid derivative is a sterol. In some embodiments of the present disclosure, the steroid or the steroid derivative is a cholestane or a cholestane derivative. As described above, the cholestane derivative comprises one or more non-alkyl substitutions of the above ring system. In some embodiments, the cholestane or the cholestane derivative is a cholestene or a cholestene derivative, or a sterol or a sterol derivative. In other embodiments, the cholestane or the cholestane derivative is a cholestene and a sterol or a derivative thereof.

"Polymer conjugated lipid" refers to a lipid that inhibits aggregation of lipid nanoparticles or improves the stability of lipid nanoparticles or alters the immune response or alters the circulation time *in vivo.* The polymer conjugated lipid includes, but is not limited to, a polyethylene glycol conjugated lipid, a polylactic acid conjugated lipid, a polyamide conjugated lipid, a cationic polymer conjugated lipid, a poly-sarcosine (pSar) conjugated lipid, a poly(lactic-co-glycolic acid) (PLGA) conjugated lipid, a polyamino acid conjugated lipid, a polypeptide conjugated lipid, and a polypeptoid conjugated lipid, or a mixture thereof. In some embodiments, the "polyethylene glycol conjugated lipid" refers to any lipid to which a PEG group has been connected. In some embodiments, the PEG lipid is a diglyceride, also comprising a PEG chain connected to a glycerol group. In other embodiments, the PEG lipid is a compound containing one or more C6-C24 long chain alkyl or alkenyl groups or C6-C24 fatty acid groups connected to a linker group via a PEG chain. Some non-limiting examples of the PEG lipid includes PEG-modified phosphatidylethanolamine and phosphatidic acid, PEG-conjugated ceramide, PEG-modified dialkylamine, PEG-modified 1,2-diacyloxypropane-3-amine, PEG-modified diacylglycerol, and dialkylglycerol. In some embodiments, PEG-modified distearoyl phosphatidylethanolamine or PEG-modified dimyristoyl-sn-glycerol. In some embodiments, PEG modification is measured with the molecular weight of the PEG component of the lipid. In some embodiments, the PEG used for modification has a molecular weight of about 100 to about 15,000. In some embodiments, the molecular weight is about 200 to about 500, about 400 to about 5000, about 500 to about 3000, or about 1200 to about 3000. The molecular weight of the PEG used for modification is about 100, 200, 400, 500, 600, 800, 1000, 1250, 1500, 1750, 2000, 2250, 2500, 2750, 3000, 3500, 4000, 4500, 5000, 6000, 7000, 8000, 9000, 10000 or 12500 to about 15000. "Modified lipid" includes lipids modified by small-molecule compounds, vitamins, carbohydrates, peptides, proteins, nucleic acids, lipopolysaccharides, inorganic molecules or particles, metal ions or particles, and combinations of the substances described above.

### English abbreviation list

| Abbreviation | English |
|---|---|
| siRNA | small interference RNA |
| saRNA | small activating RNA |
| samRNA | self-amplifying mRNA |
| miRNA | microRNA |
| aiRNA | asymmetric interfering RNA |
| dsRNA | Dicer-substrate RNA |
| shRNA | small hairpin RNA |
| DOTAP | 1,2-dioleoyloxy-3-trimethylammonium propane chloride |
| DOTMA | N-[1-(2,3-dioleoyloxy)propyl]-N, N,N-trimethylammonium chloride |
| DSTAP | 1,2-stearoyl-3-trimethylammonium-propane |
| DMTAP | 1,2-dimyristoyl-3-trimethylammonium-propane |
| DDA | dimethyldioctadecylammonium |
| DOBAQ | N-(4-carboxybenzyl)-N,Ndimethyl-2,3-bis (oleoyloxy) propan-1-aminium |
| SM-102 | 8-[(2-hydroxyethyl)[6-oxo-6-(undecyloxy)hexyl]amino]-octanoic acid, 1-octylnonyl ester |
| Lipid 5 | 8-[(2-hydroxyethyl)[8-(nonyloxy)-8-oxooctyl]amino]-octanoic acid, 1-octylnonyl ester |
| A6 | di(dec-3-yn-1-yl) 9-((4-(dimethylamino)butanoyl)oxy)heptadecanedioate |
| DC-chol | 3β-[N-(N',N'-dimethylaminoethane)-carbamoyl]cholesterol |
| C12-200 | 1,1'-((2-(4-(2-((2-(bis(2-hydroxydodecyl)amino)ethyl)(2-hydroxydodecyl)amino)ethyl)piperazin-1-yl)ethyl)azanediyl)bis(dodecan-2-ol) |
| CKK-E12 | 3,6-bis(4-(bis(2-hydroxydodecyl)amino)butyl)piperazine-2,5-dione |
| 5A2-SC8 | 1,19-Bis[2-[2-methyl-3-(octylthio)-1-oxopropoxy]ethyl] 4,10,16-tris[3-[2-[2-methyl-3-(octylthio)-1-oxopropoxy]ethoxy]-3-oxopropyl]-4,7,10,13,16-pentaazanonadecanedioate |
| G0-C14 | N-[2-[Bis(2-hydroxytetradecyl)amino]ethyl]-16-hydroxy-14-(2-hydroxytetmdecyl)-4,7-bis[3-[[2-[(2-hydroantetradecyl)amino]ethyl]amino]-3-oxopropyl]-10-oxo-4,7,11,14-tetraazaoctacosanamide |
| OF-2 | 3,6-Bis[4-[bis[(9Z,12Z)-2-hydroxy-9,12-octadecadien-1-yl]amino]butyl]-2,5-piperazinedione |
| OF-Deg-Lin | 9,12-Octadecadienoic acid (9Z,12Z)-, 1,1 ' ,1 ' ' ,1 ' ' ' -[(3,6-dioxo-2,5-piperazinediyl)bis(4,1-butanediylnitrilodi-2,1-ethanediyl)] ester |
| 92-O17S | bis(2-(tetradecylthio)ethyl) 3,3'-((3-(1H-imidazol-1 -yl)propyl)azanediyl)dipropionate |
| OF-C4-Deg-Lin | 2-[4-[5-[4-[bis[2-[(9Z,12Z)-octadeca-9,12-dienoyl]oxethyl]amino]butyl]-3,6-dioxopiperazin-2-yl]butyl-[2-[(9Z,12Z)-octadeca-9,12-dienoyl]oxyethyl]amino]ethyl (9Z, 12Z)-octadeca-9,12-dienoate |
| A18-iso5-2DC18 | ethyl 1-(3-(2-ethylpiperidin-1-yl)propyl)-5,5-di((Z)-heptadec-8-en-1-yl)-2,5-dihydro-1H-imidazole-2-carboxylate |
| TT3 | N1,N3,N5-tris(3-(didodecylamino)propyl)benzene-1,3,5-tricarboxamide |
| FTT5 | hexa(octan-4-yl) 9,9',9",9"',9"",9""'-((((benzene-1,3,5-tricarbonyl)tris(azanediyl))tris(propane-3,1-diyl))tris(azanetriyl))hexanonanoate |
| BAMEA-O16B | bis(2-(dodecyldisulfaneyl)ethyl) 3,3 '-((3-methyl-9-oxo-10-oxa-13, 14-dithia-3,6-diazahexacosyl)azanediyl)dipropionate |
| Vc-Lipid | (S)-2-((R)-3,4-bis(benzyloxy)-5-oxo-2,5-dihydrofuran-2-yl)-2-hydroxyethyl 6-((3-(didodecylamino)propyl)(dodecyl)amino)hexanoate |
| C14-4 | 1,1'-((2-(2-(4-(2-((2-(2-(bis(2-hydroxytetradecyl)amino)ethoxy)ethyl)(2-hydroxytetradecyl)amino)ethyl)piperazin-1-yl)ethoxy)ethyl)azanediyl)bis(tetradecan-2-ol) |
| Lipid 14 | ((2-((4-(dimethylamino)butanoyl)oxy)ethyl)azanediyl)bis(octane-8,1-diyl) bis(2-hexyldecanoate) |
| 4A3-Cit | 2-[3-[3-[3-[bis[3-[2-[3-(3,7-dimethyloct-6-enylsulfanyl)-2-methylpropanoyl]oxyethoxy]-3-oxopropyl]amino]propyl-methylamino]propyl-[3-[2-[3-(3,7-dimethyloct-6-enylsulfanyl)-2-methylpropanoyl]oxyethoxy]-3-oxopropyl]amino]propanoyloxy]ethyl 3-(3,7-dimethyloct-6-enylsulfanyl)-2-methylpropanoate |
| ssPalmO-Phe | [4-[2-[2-[1-[2-[2-[4-[2-[2-[4-[(Z)-octadec-9-enoyl]oxyphenyl]acetyl]oxyethyl]piperidin-1-yl]ethyldisulfanyl]ethyl]piperidin-4-yl]ethoxy]-2-oxoethyl]phenyl] (Z)-octadec-9-enoate |
| DOPE | 1,2-dioleoyl-sn-glycero-3-phosphoethanolamine |
| DSPC | 1,2-distearoyl-sn-glycero-3 -phosphocholine |
| DPPC | 1,2-dipalmitoyl-sn-glycero-3-phosphocholine |
| DMPC | 1,2-dimyristoyl-sn-glycero-3 -phosphocholine |
| DOPC | 1,2-dioleoyl-sn-glycero-3-phosphocholine |
| POPC | 1-palmitoyl-2-oleoyl-sn-glycero-3-phosphocholine |
| SM | Sphingomyelin |
| PEG-DMG | 1,2-Dimyristoyl-rac-glycero-3-methoxypolyethylene glycol |
| PEG-DSPE | 1,2-Distearoyl-sn-glycero-3-phosphoethanolamine-Poly (ethylene glycol) |
| GOLD | Guided On-Target Lipid Delivery |
| ASO | Antisense oligonucleotide |
| Agomir | Agonist microRNA |
| Antagomir | Antagonist microRNA |
| pSar | Poly sarcosinylated |
| PLGA | Poly lactic-co-glycolic acid |
| circRNA | Circular RNA |
| tRNA | Transfer RNA |
| cDNA | Complementary DNA |
| pDNA | Plasmid DNA |

### DETAILED DESCRIPTION

In order to more clearly describe the objects, features, and advantages of the present invention, the present invention will be described below in detail with reference to the drawings and specific embodiments, but the embodiments of the present invention described below in detail are intended only to illustrate the content of the present invention and do not constitute any limitation to the present invention.

### Example 1: Synthesis of Compound

The following reagents for synthesis of the compound were all AR grade and purchased from Shanghai Titan Scientific Co.,Ltd.

A synthesis method of compound 1-1 was as follows:

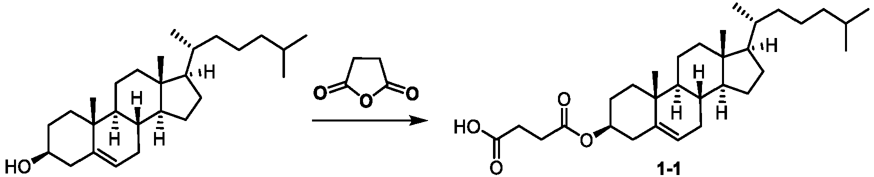

Succinic anhydride (259 mg, 2.59 mmol) was added into a 25 mL flask and dissolved in DCM (5 mL), and DMAP (32 mg, 0.259 mmol) was added. After the mixture was stirred at room temperature for 30 min, cholesterol (500 mg, 1.29 mmol) was added to the solution, and the mixture was stirred at room temperature overnight. The extent of the reaction was detected by TLC (10% MeOH/DCM). After completion of the reaction, the solution was diluted with 10 mL of DCM. Then the mixture was washed with 10 mL of water and 10 mL of saturated brine, dried over anhydrous Na₂SO₄, and filtered. The organic phase was concentrated to obtain about 610 mg of a crude product. The crude product was purified and separated using a Flash column (10 g silica gel, loading with 5 mL of DCM; the mobile phases were respectively: 50 mL of DCM; 50 mL of 1% methanol + 99% DCM; 50 mL of 2% methanol + 98% DCM; 50 mL of 5% methanol + 95% DCM) to obtain a pure product (compound 1-1, 360 mg, 52% yield).

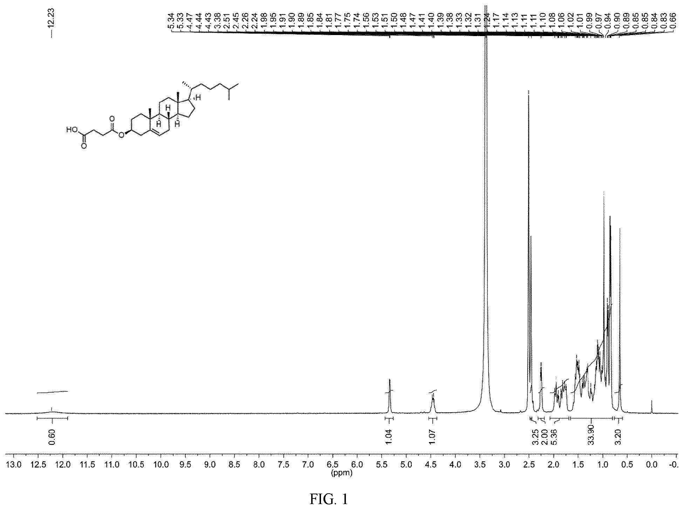

The NMR result of compound 1-1 was shown in FIG. 1. 1H NMR (400 MHz, CDCl₃): *δ* 5.37 (m, 1H), 4.50 (m, 1H), 2.65 (t, *J* = 6.0 Hz, 2H), 2.60 (t, *J* = 6.0 Hz, 2H), 2.35 (d, *J* = 7.0 Hz, 2H), 0.5-2.1 (m, 41H).

A synthesis method of compound 1-2 was as follows:

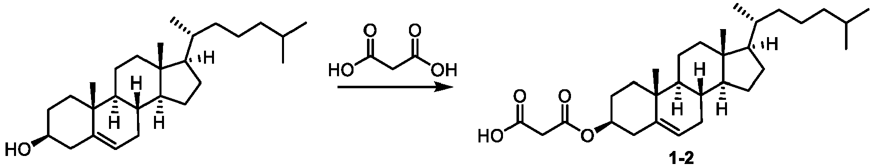

Malonic acid (1.1 g, 10.4 mmol) dissolved in DCM (20 mL) was added into a 50 mL flask, and DMAP (127 mg, 1.04 mmol) and DCC (536 mg, 2.6 mmol) were added. After the mixture was stirred at room temperature for 30 min, cholesterol (1 g, 2.6 mmol) was added to the solution, and the mixture was stirred at room temperature overnight. The extent of the reaction was detected by TLC (10% MeOH/DCM). After completion of the reaction, the solution was diluted with 20 mL of DCM. Then the mixture was washed with 20 mL of water and 20 mL of saturated brine, dried over anhydrous Na₂SO₄, and filtered. The organic phase was concentrated to obtain about 1.1 g of a crude product. The crude product was purified and separated using a Flash column (15 g silica gel, loading with 10 mL of DCM; the mobile phases were respectively: 50 mL of DCM; 50 mL of 1% methanol + 99% DCM; 50 mL of 2% methanol + 98% DCM; 50 mL of 5% methanol + 95% DCM) to obtain a pure product (compound 1-2, 840 mg, 68% yield).

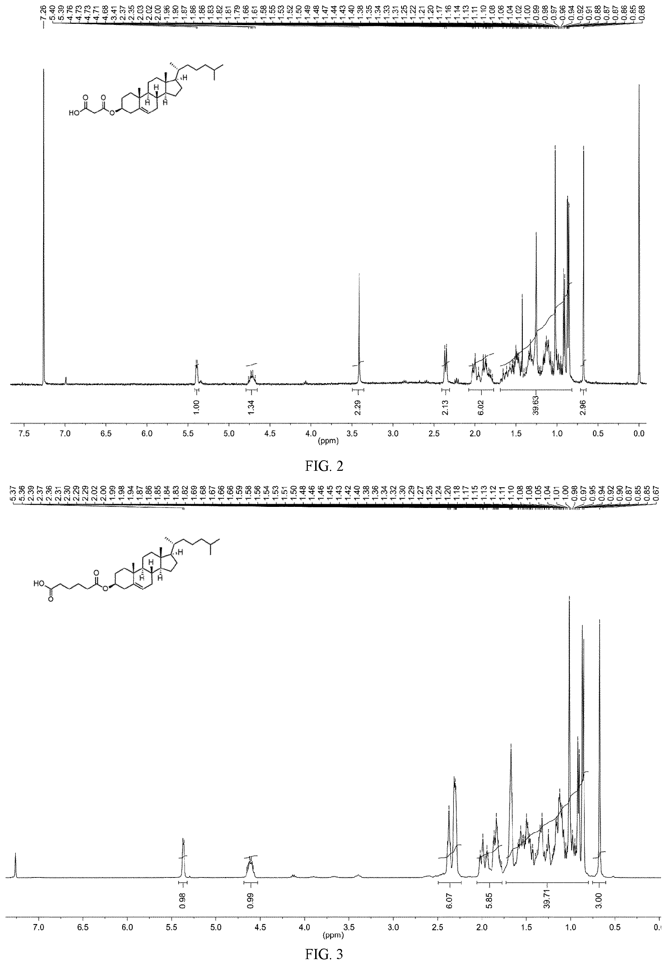

The NMR result of compound 1-2 was shown in FIG. 2. 1H NMR (400 MHz, CDCl3): *δ* 5.42 (m, 1H), 4.71-4.79 (m, 1H), 3.44 (s, 2H), 2.39 (d, *J=* 8.0 Hz, 2H), 0.5-2.1 (m, 41H).

A synthesis method of compound 1-3 was as follows:

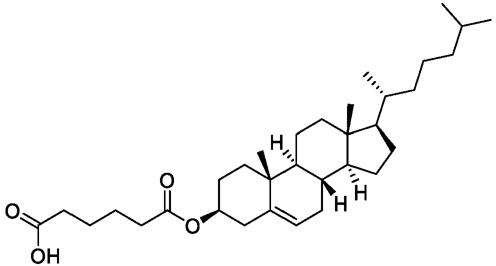

Adipic acid (4.38 g, 30 mmol), DMAP (1.22 g, 10 mmol), DCC (6.2 g, 30 mmol) and DCM (40 mL) were added into a 100 mL flask. After the mixture was stirred at room temperature for 30 min, cholesterol (3.86 g, 10 mmol) was added to the reaction solution. The extent of the reaction was detected by TLC (10% MeOH/DCM). After completion of the reaction, the reaction solution was filtered to remove solids, and the filtrate was washed with 15 mL of water and 15 mL of saturated brine, dried over anhydrous Na₂SO₄, and filtered. The organic phase was concentrated to obtain about 4.5 g of a crude product. The crude product was purified and separated using a Flash column (120 g silica gel; the mobile phases were respectively: 500 mL of *n*-heptane; 300 mL of 1% EtOAc + 99% *n*-heptane; 300 mL of 2% EtOAc + 98% n-heptane; 200 mL of 5% EtOAc + 95% *n*-heptane; 1000 mL of 8% EtOAc + 92% *n*-heptane) to obtain a pure product (compound 1-3, 2.2 g, 43% yield).

The NMR result of compound 1-3 was shown in FIG. 3. 1H NMR (400 MHz, CDCl3): *δ* 5.37-5.36 (m, 1H), 4.65-4.58 (m, 1H), 2.39-2.29 (m, 6H), 2.03-1.78 (m, 6H), 1.69-0.85 (m, 40H), 0.67 (s, 3H).

A synthesis method of compound 1-4 was as follows:

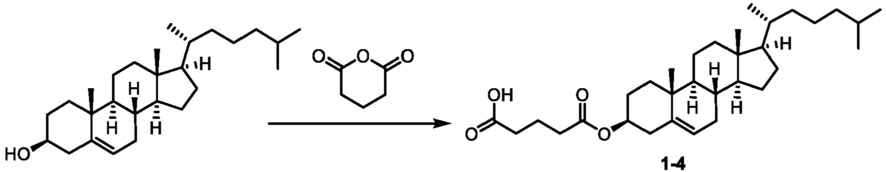

Glutaric anhydride (2.0 g, 20 mmol), DMAP (245 mg, 2 mmol), cholesterol (4.0 g, 10 mmol) and DCM (40 mL) were added into a 50 mL flask. After the mixture was stirred at room temperature overnight, the extent of the reaction was detected by TLC (30% EtOAc/*n*-heptane). After completion of the reaction, the mixture was washed with 15 mL of water and 15 mL of saturated brine, dried over anhydrous Na₂SO₄, and filtered. The organic phase was concentrated to obtain about 4.5 g of a crude product. The crude product was purified and separated using a Flash column (20 g silica gel; the mobile phases were respectively: 500 mL of *n*-heptane; 300 mL of 1% EtOAc + 99% *n*-heptane; 300 mL of 2% EtOAc + 98% *n*-heptane; 200 mL of 5% EtOAc + 95% *n*-heptane; 1000 mL of 8% EtOAc + 92% n-heptane) to obtain a pure product (compound 1-4, 1.6 g, 32% yield).

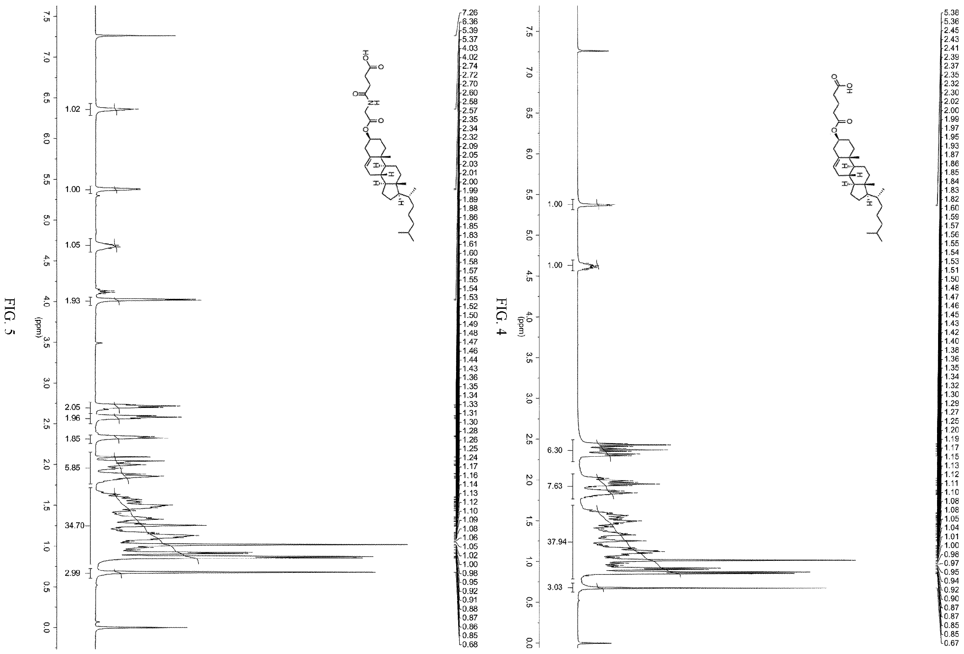

The NMR result of compound 1-4 was shown in FIG. 4. 1H NMR (400 MHz, CDCl3): *δ* 5.38-5.36 (m, 1H), 4.66-4.58 (m, 1H), 2.45-2.30 (m, 6H), 2.03-1.80 (m, 8H), 1.64-0.83 (m, 38H), 0.67 (s, 3H).

A synthesis method of compound 1-5 was as follows:

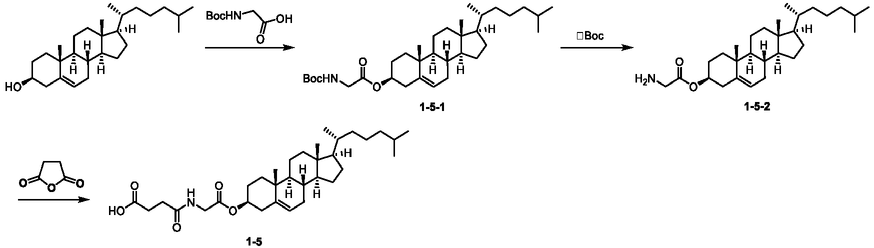

### Step 1: synthesis of compound 1-5-1

The reaction product cholesterol (5 g, 12.9 mmol) and Boc-glycine (2.26 g, 12.9 mmol) dissolved in DCM (50 mL) were added into a 250 mL flask, and DMAP (157 mg, 1.29 mmol) and DCC (4 g, 19.4 mmol) were added. The mixture was stirred at room temperature overnight. The extent of the reaction was detected by TLC (20% EtOAc/*n*-heptane). After completion of the reaction, the reaction solution was filtered to remove solids, and the filtrate was washed with 25 mL of water and 25 mL of saturated brine, dried over anhydrous Na₂SO₄, and filtered. The organic phase was concentrated to obtain about 7.1 g of a crude product. The crude product was purified and separated using a Flash column (60 g silica gel, loading with 50 mL of n-heptane; the mobile phases were respectively: 300 mL of n-heptane; 300 mL of 1% EtOAc + 99% n-heptane; 300 mL of 2% EtOAc + 98% n-heptane) to obtain a pure product (1-5-1, 5.3 g, 76% yield).

### Step 2: synthesis of compound 1-5-2

1-5-1 (5.3 g, 9.7 mmol) dissolved in DCM (30 mL) was added into a 100 mL flask, and TFA (6 mL) was added. After the mixture was stirred at room temperature for 2 h, the extent of the reaction was detected by TLC (20% EtOAc/*n*-heptane). After completion of the reaction, the reaction solution was concentrated, slurried with 50 mL of n-heptane, and filtered to obtain a solid. The solid was then dissolved with DCM (25 mL), then washed with 25 mL of a 10% Na₂CO₃ solution, 25 mL of water and 25 mL of saturated brine, dried over anhydrous Na₂SO₄ and filtered. The organic phase was concentrated to obtain a pure product (1-5-2, 3.3 g, 77% yield).

### Step 3: synthesis of compound 1-5

Succinic anhydride (225 mg, 2.25 mmol) was added into a 25 mL flask and dissolved in DCM (5 mL), and DMAP (27 mg, 0.225 mmol) was added. After the mixture was stirred at room temperature for 30 min, 1-5-2 (500 mg, 1.13 mmol) was added to the solution, and the mixture was stirred at room temperature overnight. The extent of the reaction was detected by TLC (10% MeOH/DCM, 0.1% acetic acid). After completion of the reaction, the solution was diluted with 10 mL of DCM. Then the mixture was washed with 10 mL of water and 10 mL of saturated brine, dried over anhydrous Na₂SO₄, and filtered. The organic phase was concentrated to obtain about 590 mg of a crude product. The crude product was purified and separated using a Flash column (10 g silica gel, loading with 5 mL of DCM; the mobile phases were respectively: 50 mL of 1% methanol + 99% of DCM; 50 mL of 2% methanol + 98% DCM; 50 mL of 5% methanol + 95% DCM; 50 mL of 10% methanol + 90% DCM) to obtain a pure product (compound 1-5, 320 mg, 52% yield).

The NMR result of compound 1-5 was shown in FIG. 5. 1H NMR (400 MHz, CDCl3): *δ* 5.38 (m, 1H), 4.65-4.71 (m, 1H), 4.02(d, *J=* 4.0 Hz, 2H), 2.70-2.74 (m, 2H), 2.57-2.60 (m, 2H), 2.32-2.35 (m, 2H), 0.5-2.1 (m, 44H).

A synthesis method of compound 1-6 was as follows:

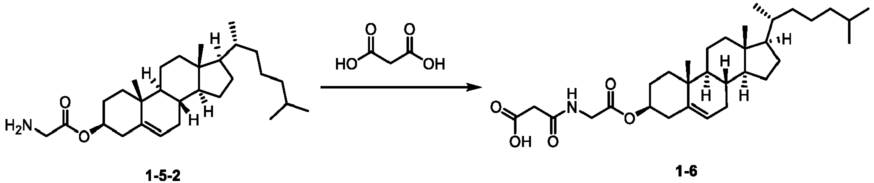

Malonic acid (500 mg, 4.8 mmol), DMAP (58.6 mg, 0.48 mmol), DCC (248 mg, 1.2 mmol) and DCM (10 mL) were added into a 50 mL flask. After the mixture was stirred at room temperature for 30 min, 1-5-2 (700 mg, 1.2 mmol) was added to the reaction solution. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 1.1 g of a crude product. The crude product was purified and separated using a Flash column (20 g silica gel; the mobile phases were respectively: 200 mL of *n*-heptane; 150 mL of 1% EtOAc + 99% *n*-heptane; 150 mL of 2% EtOAc + 98% *n*-heptane; 200 mL of 5% EtOAc + 95% *n*-heptane; 500 mL of 8% EtOAc + 92% *n*-heptane) to obtain a pure product (compound 1-6, 290 mg, 77% yield).

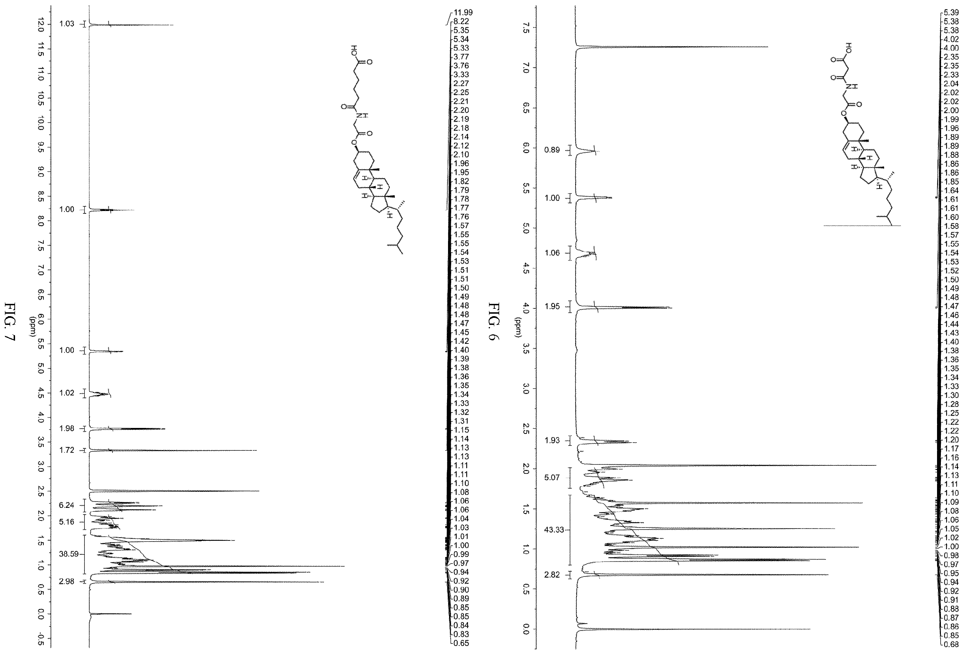

The NMR result of compound 1-6 was shown in FIG. 6. 1H NMR (400 MHz, CDCl3): *δ* 5.96 (s, 1H), 5.39-5.38 (m, 1H), 4.70-4.64 (m, 1H), 4.01 (d, *J*=4Hz, 2H), 2.39-2.33 (m, 2H), 2.08-1.78 (m, 5H), 1.68-0.85 (m, 43H), 0.68 (s, 3H).

A synthesis method of compound 1-7 was as follows:

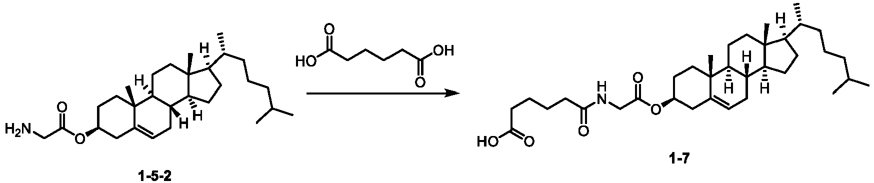

Adipic acid (701.6 mg, 4.8 mmol), DMAP (58.6 mg, 0.48 mmol), DCC (248 mg, 1.2 mmol) and DCM (20 mL) were added into a 100 mL flask. After the mixture was stirred at room temperature for 30 min, 1-5-2 (700 mg, 1.2 mmol) was added to the reaction solution. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 1.3 g of a crude product. The crude product was purified and separated using a Flash column (20 g silica gel; the mobile phases were respectively: 200 mL of *n*-heptane; 150 mL of 1% EtOAc + 99% *n*-heptane; 150 mL of 2% EtOAc + 98% *n*-heptane; 200 mL of 5% EtOAc + 95% *n*-heptane; 500 mL of 8% EtOAc + 92% *n*-heptane) to obtain a pure product (compound 1-7, 360 mg, 44% yield).

The NMR result of compound 1-7 was shown in FIG. 7. 1H NMR (400 MHz, *d6*-DMSO): *δ* 11.99 (s, 1H), 8.22 (t, *J*=8Hz, 1H), 5.35-5.33 (m, 1H), 4.51-4.43 (m, 1H), 3.77 (d, *J*=4Hz, 2H), 3.33 (s, 2H), 2.27-2.10 (m, 6H), 1.98-1.75 (m, 5H), 1.59-0.83 (m, 38H), 0.65 (s, 3H).

A synthesis method of compound 1-8 was as follows:

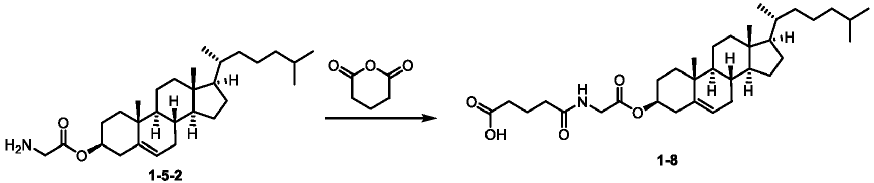

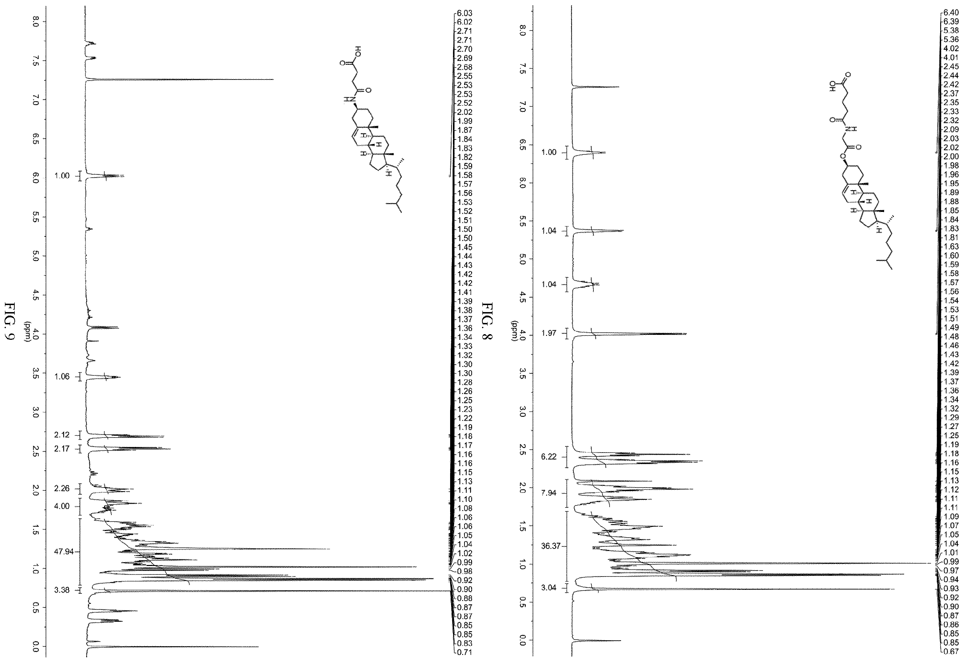

Glutaric anhydride (194 mg, 1.7 mmol), DMAP (21 mg, 0.17 mmol) and DCM (5 mL) were added into a 50 mL flask. After the mixture was stirred at room temperature for 30 min, 1-5-2 (500 mg, 0.85 mmol) was added to the reaction solution. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 720 mg of a crude product. The crude product was purified and separated using a Flash column (20 g silica gel; the mobile phases were respectively: 200 mL of n-heptane; 150 mL of 1% EtOAc + 99% *n-*heptane; 150 mL of 2% EtOAc + 98% n-heptane; 200 mL of 5% EtOAc + 95% n-heptane; 500 mL of 8% EtOAc + 92% *n*-heptane) to obtain a pure product (compound 1-8, 320 mg, 67% yield). The NMR result of compound 1-8 was shown in FIG. 8. 1H NMR (400 MHz, CDCl3): *δ* 6.42-6.39 (m, 1H), 5.38-5.36 (m, 1H), 4.71-4.63 (m, 1H), 4.02-4.01 (m, 2H), 2.45-2.32 (m, 6H), 2.09-1.78 (m, 8H), 1.63-0.85 (m, 36H), 0.67 (s, 3H).

A synthesis method of compound 1-9 was as follows:

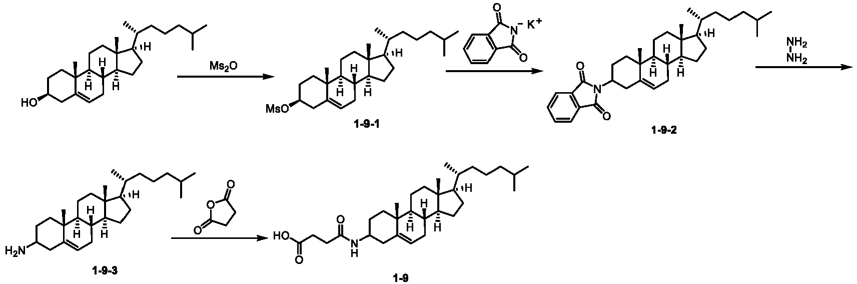

### Step 1: synthesis of compound 1-9-1

Cholesterol (5.0 g, 12.9 mmol), triethylamine (2.6 g, 25.8 mmol) and DCM (50 mL) were added into a 100 mL flask. Methanesulfonic anhydride (3.4 g, 19.4 mmol) was added dropwise to the solution at 0-5 °C in an ice bath. After the dropwise addition, the mixture was reacted at 0-5 °C for 2 h, slowly heated to room temperature, and stirred overnight. The extent of the reaction was detected by TLC (20% EtOAc/n-heptane). After completion of the reaction, the mixture was washed with 5 mL of water and 5 mL of saturated brine, dried over anhydrous Na₂SO₄, and filtered. The organic phase was concentrated to obtain about 7 g of a crude product. The crude product was purified and separated using a Flash column (200 g silica gel, loading with 500 mL of *n*-heptane; the mobile phases were respectively: 500 mL of *n*-heptane; 500 mL of 0.3% EtOAc + 99.7% *n-*heptane; 500 mL of 1% EtOAc + 99% *n*-heptane; 2000 mL of 2% EtOAc + 98% *n*-heptane) to obtain a pure product (1-9-1, 3.6 g, 60% yield).

### Step 2: synthesis of compound 1-9-2

1-9-1 (4.0 g, 8.6 mmol), potassium phthalimide (1.75 g, 9.5 mmol) and DMF (40 mL) were added into a 100 mL flask. The mixture was heated to 90 °C and stirred for 16 h. The extent of the reaction was detected by TLC (30% EtOAc/*n*-heptane). After completion of the reaction, 15 mL of water was added for separation of layers, the aqueous layer was extracted with ethyl acetate (20 mL × 2), and the organic phases were combined, washed with 15 mL of saturated brine, dried over anhydrous Na₂SO₄, and filtered. The organic phase was concentrated to obtain about 3 g of a crude product. The crude product was purified and separated using a Flash column (200 g silica gel, loading with 500 mL of *n*-heptane; the mobile phases were respectively: 300 mL of *n*-heptane; 300 mL of 1% EtOAc + 99% *n*-heptane; 300 mL of 3% EtOAc + 97% *n*-heptane; 300 mL of 5% EtOAc + 95% *n*-heptane; 2000 mL of 10% EtOAc + 90% *n*-heptane) to obtain a pure product (1-9-2, 1 g, 22% yield).

### Step 3: synthesis of compound 1-9-3

1-9-2 (200 mg, 38 mmol), hydrazine hydrate (133 mg, 0.95 mmol) and absolute ethanol (2 mL) were added into a 100 mL flask. The mixture was heated to 80-85°C and stirred for 3 h. The extent of the reaction was detected by TLC (30% EtOAc/n-heptane). After completion of the reaction, the mixture was filtered, and the filtrate was concentrated to dryness. 2 mL of DCM was added. The mixture was dried over anhydrous Na₂SO₄ and filtered to obtain a filtrate, which was directly used in the next step.

### Step 4: synthesis of compound 1-9

Succinic anhydride (76.1 mg, 0.76 mmol), DMAP (9.3 mg, 0.076 mmol) and DCM (1 mL) were added into a 50 mL flask. After the mixture was stirred at room temperature for 30 min, the solution of 1-9-3 obtained in the previous step was added into the reaction solution. The extent of the reaction was detected by TLC (30% EtOAc/*n*-heptane). After completion of the reaction, the mixture was diluted with 10 mL of DCM, washed with 5 mL of water and 5 mL of saturated brine, dried over anhydrous Na₂SO₄, and filtered. The organic phase was concentrated to obtain about 180 mg of a crude product. The crude product was purified and separated using a Flash column (20 g silica gel; the mobile phases were respectively: 100 mL of n-heptane; 100 mL of 1% EtOAc + 99% *n*-heptane; 100 mL of 2% EtOAc + 98% *n*-heptane; 100 mL of 5% EtOAc + 95% *n*-heptane; 300 mL of 8% EtOAc + 92% n-heptane) to obtain a pure product (compound 1-9, 80 mg, 43% yield).

The NMR result of compound 1-9 was shown in FIG. 9. 1H NMR (400 MHz, CDCl3): *δ* 6.03-6.02 (m, 1H), 3.47-3.43 (m, 1H), 2,71-2.68 (m, 2H), 2.55-2.52 (m, 2H), 2.07-1.98 (m, 2H), 1.87-1.71 (m, 4H), 1.59-0.82 (m, 48H), 0.71 (s, 3H).

A synthesis method of compound 1-25 was as follows:

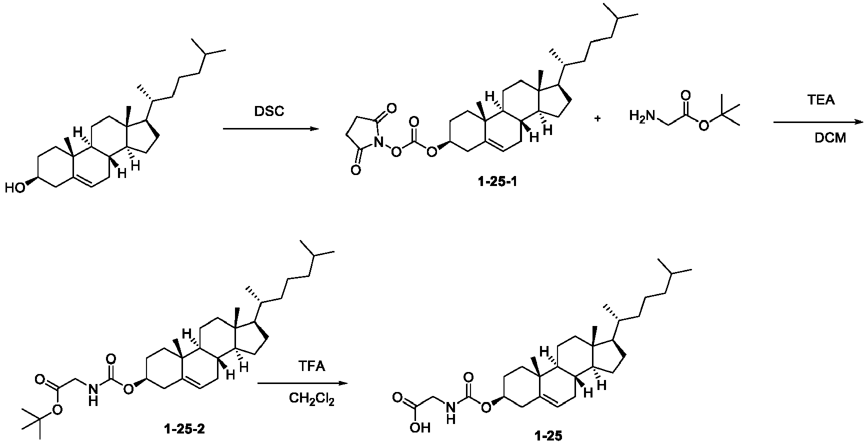

### Step 1: synthesis of compound 1-25-1

Cholesterol (11.6 g, 30 mmol), triethylamine (9.2 g, 90 mmol), DSC (11.5 g, 45 mmol) and acetonitrile (110 mL) were added to a 250 mL three-necked flask. The mixture was heated to 45 °C and stirred overnight. The extent of the reaction was detected by TLC (20% EtOAc/*n*-heptane). After completion of the reaction, the organic phase was concentrated to obtain about 10 g of a crude product. The crude product was purified and separated using a Flash column (100 g silica gel; the mobile phases were respectively: 300 mL of *n*-heptane; 200 mL of 1% EtOAc + 99% *n-*heptane; 200 mL of 5% EtOAc + 95% *n*-heptane; 300 mL of 8% EtOAc + 92% *n*-heptane; 1000 mL of 15% EtOAc + 85% *n*-heptane) to obtain a pure product (1-25-1, 6.1 g, 38% yield).

### Step 2: synthesis of compound 1-25-2

1-25-1 (541.8 mg, 1.03 mmol), triethylamine (114.3 mg, 1.13 mmol), *tert*-butyl glycinate (131.2 mg, 1.1 mmol) and DCM (5 mL) were added into a 25 mL three-necked flask. The mixture was stirred at room temperature overnight. The extent of the reaction was detected by TLC (20% EtOAc/*n-*heptane). After completion of the reaction, 10 mL of DCM was added. The mixture was washed with 5 mL of an aqueous sodium bicarbonate solution and 5 mL of an aqueous sodium chloride solution, dried over anhydrous Na₂SO₄, and filtered. The organic phase was concentrated to obtain about 800 mg of a crude product. The crude product was purified and separated using a Flash column (10 g silica gel; the mobile phases were respectively: 200 mL of *n*-heptane; 200 mL of 1% EtOAc + 99% *n*-heptane; 200 mL of 5% EtOAc + 95% *n*-heptane; 300 mL of 8% EtOAc + 92% *n*-heptane) to obtain a pure product (1-25-2, 350 mg, 38% yield).

### Step 3: synthesis of compound 1-25

1-25-2 (350 mg, 0.64 mmol) dissolved in DCM (9 mL) was added into a 50 mL flask, and TFA (3 mL) was added. After the mixture was stirred at room temperature for 2 h, the extent of the reaction was detected by TLC (20% EtOAc/*n*-heptane). After completion of the reaction, the reaction solution was concentrated, and MTBE (20 mL) was added and dissolved. The pH was adjusted to 8-9 with 10% NaHCO₃ solution, and then the pH was adjusted to 1-2 with 1 M diluted hydrochloric acid. The mixture was stirred for 20 min, and separation of layers was performed. The aqueous phase was extracted with 20 mL of MTBE, and the organic phases were combined, dried over anhydrous Na₂SO₄, and filtered. The organic phase was concentrated to obtain 300 mg of a crude product. The crude product was purified and separated using a Flash column (8 g silica gel; the mobile phases were respectively: 200 mL of *n*-heptane; 100 mL of 5% EtOAc + 95% *n*-heptane; 100 mL of 10% EtOAc + 90% *n*-heptane; 100 mL of 20% EtOAc + 80% *n*-heptane; 200 mL of 30% EtOAc + 70% *n*-heptane) to obtain a pure product (compound 1-25, 100 mg, 32% yield).

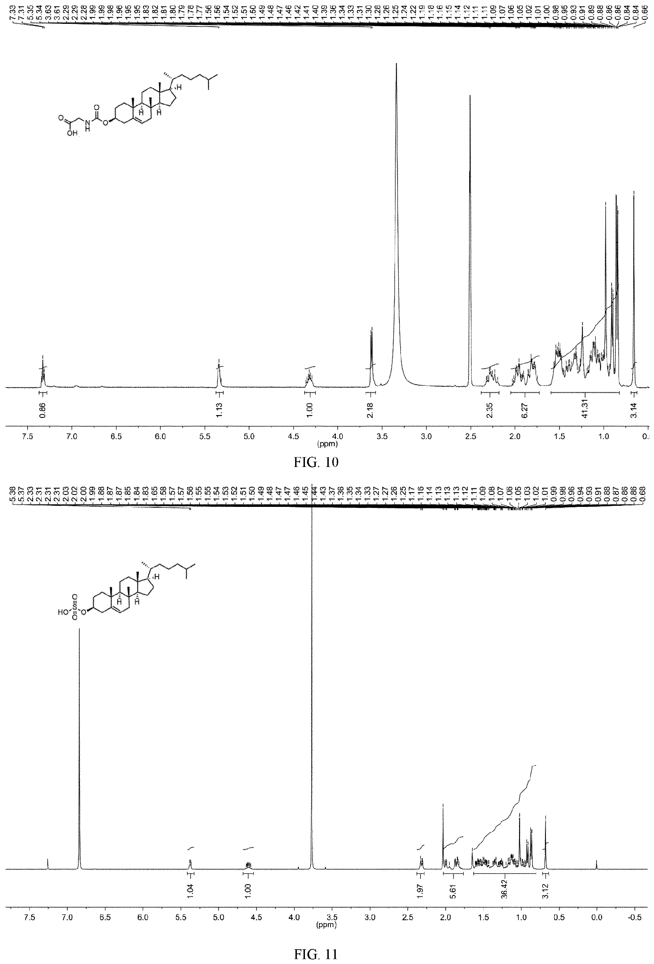

The NMR result of compound 1-25 was shown in FIG. 10. 1H NMR (400 MHz, *d6*-DMSO): *δ* 7.33 (t, *J*=8Hz, 1H), 5.35-5.32 (m, 1H), 4.36-4.29 (m, 1H), 3.63-3.61 (m, 2H), 2.32-2.20 (m, 2H), 2.03-1.75 (m, 6H), 1.56-0.84 (m, 41H), 0.66 (s, 3H).

A synthesis method of compound 1-32 was as follows:

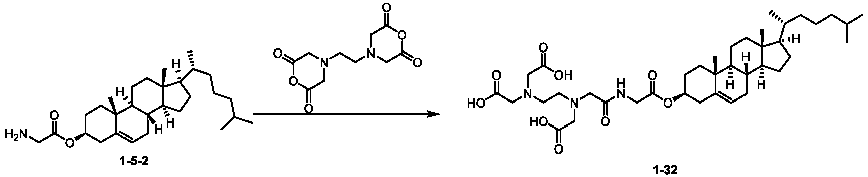

1-5-2 (500 mg, 0.9 mmol), EDTA anhydride (236 mg, 0.9 mmol) and toluene (5 mL) were added into a 50 mL flask. The mixture was heated to 90 °C and stirred for 16 h. The extent of the reaction was detected by TLC (10% MeOH/DCM). After completion of the reaction, 5 mL of water and 15 mL of THF were added, the mixture was stirred overnight, and separation of layers was performed. The aqueous layer was extracted with 10 mL of THF, and the organic phase was dried over dried over anhydrous Na₂SO₄, and filtered. The organic phase was concentrated to obtain a product (1-32, 40 mg, 6.2% yield).

A synthesis method of compound 1-36 was as follows:

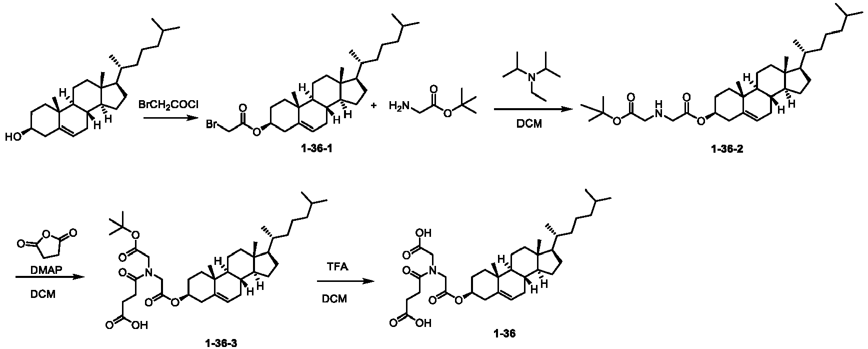

### Step 1: synthesis of compound 1-36-1

Bromoacetyl chloride (13.03 g, 82.8 mmol) dissolved in DCM (50 mL) was added into a 500 mL flask and cooled to 0-5 °C in an ice-salt bath, a solution of cholesterol (16 g, 41.4 mmol) in DCM (110 mL) was added slowly dropwise into the solution, and the mixture was stirred at room temperature overnight after the dropwise addition. The extent of the reaction was detected by TLC (20% EtOAc/n-heptane). After completion of the reaction, 50 mL of water was added to quench the reaction, liquid separation was performed, and the aqueous phase was extracted with DCM (50 mL × 2). The organic phases were combined, washed with 50 mL of saturated brine, dried over anhydrous Na₂SO₄, and filtered. The organic phase was concentrated to obtain about 22 g of a crude product. The crude product was purified and separated using a Flash column (200 g silica gel, loading with 500 mL of n-heptane; the mobile phases were respectively: 500 mL of n-heptane; 500 mL of 0.1% EtOAc + 99.9% n-heptane; 500 mL of 0.3% EtOAc + 99.7% n-heptane; 500 mL of 0.5% EtOAc + 99.5% n-heptane; 2000 mL of 1% EtOAc + 99% n-heptane) to obtain a pure product (1-36-1, 16 g, 76% yield).

### Step 2: synthesis of compound 1-36-2

1-36-1 (8.0 g, 15.8 mmol) and DIPEA (4.1 g, 31.6 mmol) dissolved in DCM (40 mL) were added into a 50 mL flask, and *tert*-butyl glycinate (4.1 g, 31.6 mmol) was added. The mixture was stirred at room temperature overnight. The extent of the reaction was detected by TLC (20% EtOAc/*n-*heptane). After completion of the reaction, the mixture was washed with 10 mL of water and 10 mL of saturated brine, dried over anhydrous Na₂SO₄, and filtered. The organic phase was concentrated to obtain about 12.1 g of a crude product. The crude product was purified and separated using a Flash column (100 g silica gel, loading with 300 mL of *n*-heptane; the mobile phases were respectively: 500 mL of *n*-heptane; 500 mL of 0.1% EtOAc + 99.9% n-heptane; 500 mL of 0.3% EtOAc + 99.7% n-heptane; 2000 mL of 0.5% EtOAc + 99.5% *n*-heptane) to obtain a pure product (1-36-2, 7.5 g, 85% yield).

### Step 3: synthesis of compound 1-36-3

Succinic anhydride (174.9 mg, 1.75 mmol), DMAP (106.8 mg, 0.87 mmol) and DCM (10 mL) were added into a 50 mL flask. After the mixture was stirred at room temperature for 30 min, 1-36-2 (1.0 g, 1.75 mmol) was added to the reaction solution. The extent of the reaction was detected by TLC (5% MeOH/DCM). After completion of the reaction, the mixture was washed with 5 mL of water and 5 mL of saturated brine, dried over anhydrous Na₂SO₄, and filtered. The organic phase was concentrated to obtain about 1.8 g of a crude product. The crude product was purified and separated using a Flash column (40 g silica gel; the mobile phases were respectively: 200 mL of DCM; 200 mL of 1% MeOH + 99% DCM; 500 mL of 1.5% MeOH + 98.5% DCM) to obtain a pure product (1-36-3, 1.1 g, 92% yield).

### Step 4: synthesis of compound 1-36

1-36-3 (300 mg, 0.46 mmol) dissolved in DCM (5 mL) was added into a 50 mL flask, and TFA (2 mL) was added. After the mixture was stirred at room temperature for 2 h, the extent of the reaction was detected by TLC (5% MeOH/DCM). After completion of the reaction, the reaction solution was concentrated, and MTBE (20 mL) was added and dissolved. The pH was adjusted to 8-9 with 10% NaHCOs solution, and then the pH was adjusted to 1-2 with 1 M diluted hydrochloric acid. The mixture was stirred for 20 min, and separation of layers was performed. The aqueous phase was extracted with 20 mL of MTBE, and the organic phases were combined, dried over anhydrous Na₂SO₄, and filtered. The organic phase was concentrated to obtain 300 mg of a crude product. The crude product was purified and separated using a Flash column (8 g silica gel; the mobile phases were respectively: 100 mL of DCM; 100 mL of 1% MeOH + 99% DCM; 100 mL of 1.5% MeOH + 98.5% DCM; 100 mL of 2% MeOH + 98% DCM, 100 mL of 3% MeOH + 97% DCM; 300 mL of 8% MeOH + 92% DCM) to obtain a pure product (1-36, 140 mg, 52% yield).

The synthesis method of Compound 1-38 is as follows:

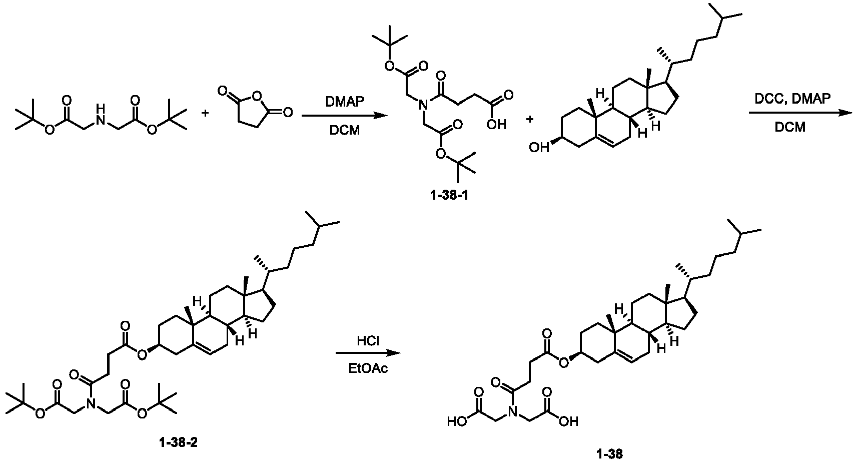

### Step 1: Synthesis of Compound 1-38-1

To a 100 mL flask were added succinic anhydride (4.08 g, 40.8 mmol), DMAP (498 mg, 4.08 mmol), and DCM (50 mL). After stirred at room temperature for 10 min, the reaction solution was added with a starting material (5.0 g, 5.4 mmol). The extent of the reaction was detected by TLC (5% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 8 g of a crude product 1-38-1, yield 100%.

### Step 2: Synthesis of Compound 1-38-2

To a 50 mL flask was added Compound 1-38-1 (1.0 g, 2.9 mmol) dissolved in DCM (10 mL), and DMAP (176.9 mg, 1.45 mmol) and DCC (597.4 mg, 2.9 mmol) were added. After stirred at room temperature for 30 min, the solution was added with cholesterol (1.12 g, 2.9 mmol) and stirred at room temperature overnight. The extent of the reaction was detected by TLC (20% EtOAc/n-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 1.9 g of a crude product. Flash columns were run for purification (using 20 g of silica gel; the mobile phases were respectively: 300 mL of n-heptane, 200 mL of 1% EtOAc+99% n-heptane, 200 mL of 5% EtOAc+95% n-heptane and 300 mL of 8% EtOAc+92% n-heptane) and separation to obtain 1.1 g of a pure product 1-38-2, yield 53%.

### Step 3: Synthesis of Compound 1-38

To a 50 mL flask was added Compound 1-38-2 (400 mg, 0.56 mmol) dissolved in DCM (2 mL), and a solution (4 mL) of hydrogen chloride in ethyl acetate was added. After the mixture was stirred at room temperature for 2 h, the extent of the reaction was detected by TLC (20% EtOAc/n-heptane). After completion of the reaction, the reaction solution was concentrated, and MTBE (20 mL) was added and dissolved. The pH was adjusted to 8-9 with 10% NaHCO₃ solution, and then the pH was adjusted to 1-2 with 1 M diluted hydrochloric acid. The mixture was stirred for 20 min, and separation of layers was performed. The aqueous phase was extracted with 20 mL of MTBE, and the organic phases were combined, dried over anhydrous Na₂SO₄, and filtered. The organic phase was concentrated to obtain 300 mg of a crude product. Flash columns were run for purification (using 8 g of silica gel; the mobile phases were respectively: 200 mL of DCM, 100 mL of 1% MeOH + 99% DCM, 100 mL of 1.5% MeOH + 98.5% DCM, 100 mL of 2% MeOH + 98% DCM and 200 mL of 3% MeOH + 97% DCM) and separation to obtain 120 mg of a pure product 1-38, yield 36%.

The synthesis method of Compound 1-39 is as follows:

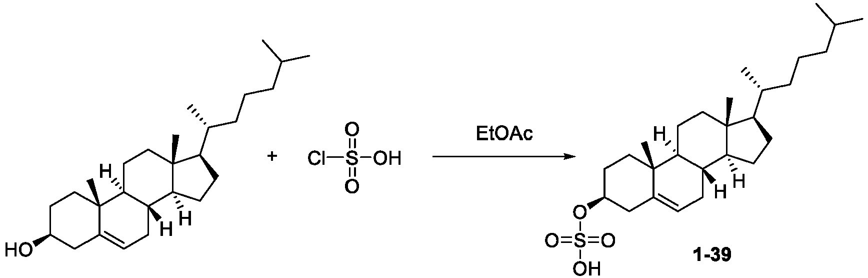

To a 100 mL flask were added cholesterol (1.16 g, 3 mmol, 1.0 eq), and 6 mL of ethyl acetate. The mixture was cooled in an ice-salt bath to -5 °C, slowly added with chlorosulfonic acid (384.5 mg, 3.3 mmol, 1.1 eq) dropwise, and stirred at a constant temperature for 3 h. The extent of the reaction was detected by TLC (20% EtOAc/n-heptane). After completion of the reaction, the solution was filtered, and the filtrate was washed with 5 mL of water and 5 mL of saturated brine. The organic phase was dried over anhydrous Na₂SO₄, and filtered. The organic phase was concentrated to obtain about 3 g of a crude product. Flash columns were run for purification (using 80 g of silica gel, loaded with 500 mL of n-heptane; the mobile phases were respectively: 200 mL of n-heptane, 100 mL of 0.5% EtOAc + 99.5% n-heptane, 100 mL of 0.8% EtOAc + 99.2% n-heptane, 100 mL of 1% EtOAc + 99% n-heptane, 100 mL of 1.5% EtOAc + 98.5% n-heptane, 100 mL of 2% EtOAc + 98% n-heptane, 100 mL of 3% EtOAc + 97% n-heptane, and 600 mL of 10% EtOAc + 90% *n-*heptane) and separation to obtain 150 mg of a pure product, Compound 1-39, yield 28%.

The NMR results of Compound 1-39 are shown in FIG. 11. 1H NMR (400 MHz, CDCl₃): *δ* 5.38-5.36 (m, 1H), 4.64-4.58 (m, 1H), 2.33-2.30 (m, 2H), 2.02-1.83 (m, 6H), 1.61-0.86 (m, 36H), 0.68 (s, 3H).

The synthesis method of Compound 1-41 is as follows:

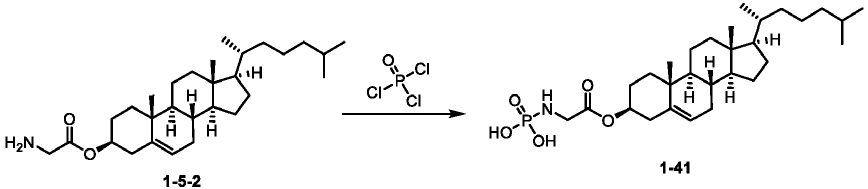

To a 50 mL flask were added Compound 1-5-2 (543.8 mg, 1.0 mmol), pyridine (237.3 mg, 3.0 mmol), and THF (10 mL). The mixture was cooled down to -40 °C with dry ice, quickly added with phosphorus oxychloride (536.6 mg, 3.5 mmol) dropwise, and stirred at -10 °C for 3 h. The extent of the reaction was detected by TLC (10% MeOH/DCM). After completion of the reaction, the solution was added with 2 mL of dilute hydrochloric acid, stirred for 1 h, and layered. The aqueous phase was extracted with THF (5 mL × 2). The organic phases were combined and washed with 5 mL of an aqueous sodium chloride solution. The organic phase was dried with anhydrous Na₂SO₄, and filtered. The organic phase was concentrated to obtain about 260 mg of a pure product 1-41, yield 50%.

The synthesis method of Compound 1-42 is as follows:

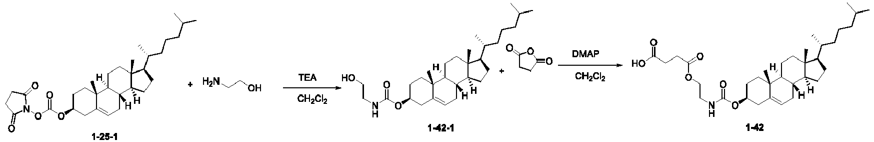

### Step 1: Synthesis of Compound 1-42-1

To a 25 mL three-necked flask were added Compound 1-25-1 (500 mg, 0.95 mmol), ethanolamine (58 mg, 0.95 mmol), triethylamine (105 mg, 1.04 mmol), and DCM (5 mL). The mixture was stirred at room temperature for 3 h. The extent of the reaction was detected by TLC (30% EtOAc/n-heptane). After completion of the reaction, the solution was added with DCM (10 mL), and washed with 5 mL of an aqueous sodium bicarbonate solution and 5 mL of an aqueous sodium chloride solution. The organic phase was dried over anhydrous Na₂SO₄, and filtered. The organic phase was concentrated to obtain about 800 mg of a crude product. Flash columns were run for purification (using 10 g of silica gel; the mobile phases were respectively: 200 mL of n-heptane, 200 mL of 1% EtOAc + 99% n-heptane, 200 mL of 5% EtOAc + 95% n-heptane, and 300 mL of 8% EtOAc + 92% n-heptane) and separation to obtain 300 mg of a pure product 1-42-1, yield 64%.

### Step 2: Synthesis of Compound 1-42

To a 50 mL flask were added succinic anhydride (122.6 mg, 1.23 mmol), DMAP (14.97 mg, 0.12 mmol), and DCM (3 mL). After stirred at room temperature for 30 min, the reaction solution was added with Compound 1-42-1 (300 mg, 0.61 mmol). The extent of the reaction was detected by TLC (30% EtOAc/n-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 300 mg of a crude product. Flash columns were run for purification (using 20 g of silica gel; the mobile phases were respectively: 500 mL of n-heptane, 300 mL of 1% EtOAc + 99% n-heptane, 300 mL of 2% EtOAc + 98% *n*-heptane, 200 mL of 5% EtOAc + 95% *n*-heptane, and 300 mL of 8% EtOAc + 92% *n*-heptane) and separation to obtain 100 mg of a pure product, Compound 1-42, yield 28%.

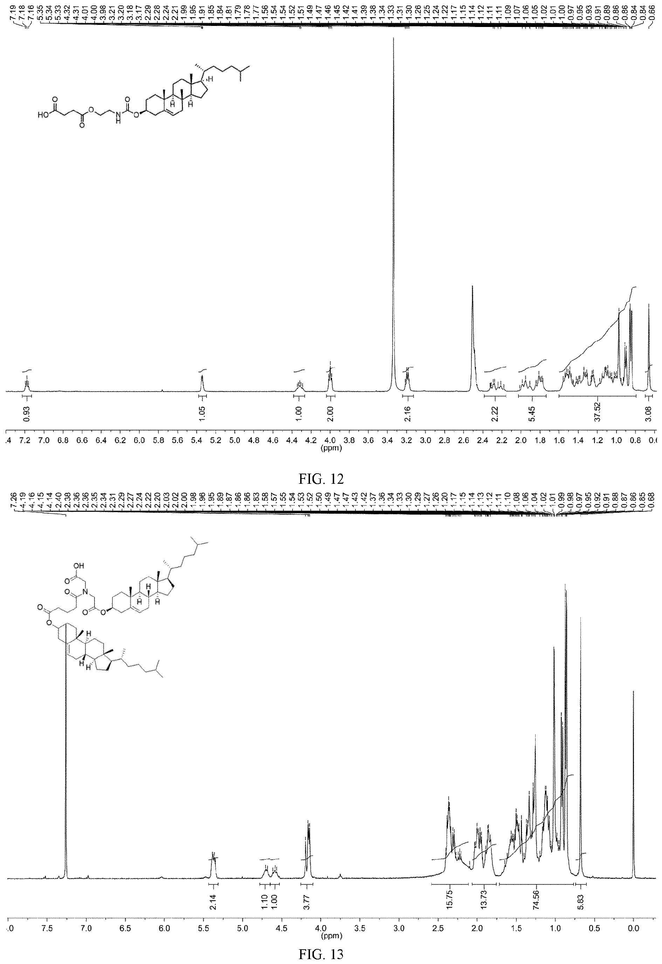

The NMR results of Compound 1-42 are shown in FIG. 12. 1H NMR (400 MHz, *d6*-DMSO): *δ* 7.18 (t, *J*=8Hz, 1H), 5.35-5.33 (m, 1H), 4.35-4.29 (m, 1H), 4.00 (t, *J*=8Hz, 2H), 3.21-3.17 (m, 2H), 2.32-2.18 (m, 2H), 2.01-1.77 (m, 6H), 1.56-0.84 (m, 37H), 0.66 (s, 3H).

The synthesis method of Compound 1-44 is as follows:

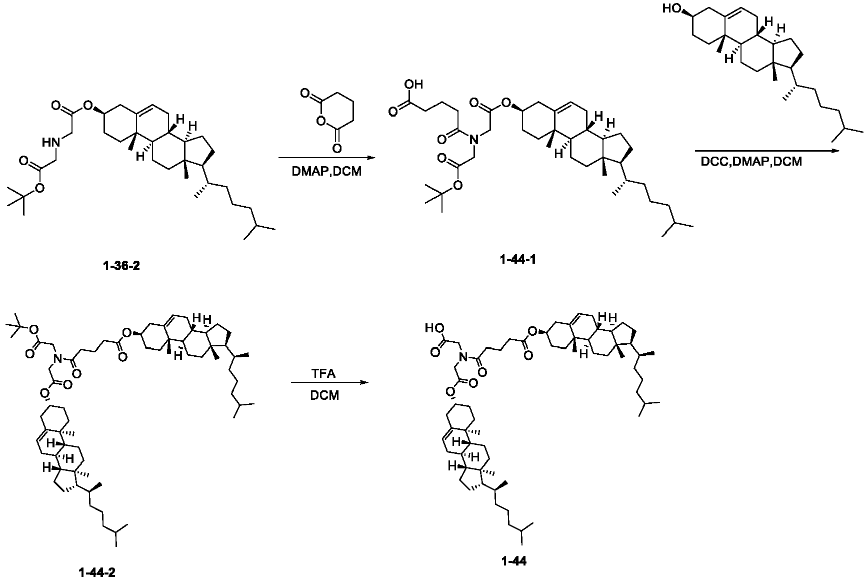

### Step 1: Synthesis of Compound 1-44-1

To a 50 mL flask were added glutaric anhydride (286.3 mg, 2.5 mmol), DMAP (30.7 mg, 0.25 mmol), and DCM (7 mL). After stirred at room temperature for 30 min, the reaction solution was added with 1-36-2 (700 mg, 1.25 mmol). The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 1.5 g of a crude product. Flash columns were run for purification (using 20 g of silica gel; the mobile phases were respectively: 200 mL of *n*-heptane, 150 mL of 1% EtOAc + 99% *n*-heptane, 150 mL of 2% EtOAc + 98% *n*-heptane, 200 mL of 5% EtOAc + 95% n-heptane, and 500 mL of 8% EtOAc + 92% *n*-heptane) and separation to obtain 700 mg of a pure product 1-44-1, yield 83%. ¹H NMR (400 MHz, CDCl₃): *δ* 5.38-5.36 (m, 1H), 4.70-4.63 (m, 1H), 4.16-4.05 (m, 4H), 2.72-2.64 (m, 4H), 2.35-2.31(m, 2H), 2.02-0.85 (m, 49H), 0.67(s,3H).

### Step 2: Synthesis of Compound 1-44-2

To a 50 mL flask was added Compound 1-44-1 (500 mg, 0.74 mmol) dissolved in DCM (5 mL), and DMAP (18.2 mg, 0.15 mmol) and DCC (230.3 mg, 1.12 mmol) were added. After stirred at room temperature for 30 min, the solution was added with cholesterol (287.7 mg, 0.74 mmol) and stirred at room temperature overnight. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 700 mg of a crude product. Flash column was run for purification (using 12 g of silica gel; the mobile phases were respectively: 300 mL of *n*-heptane, 200 mL of 1% EtOAc + 99% *n*-heptane, 200 mL of 5% EtOAc + 95% *n*-heptane and 300 mL of 8% EtOAc + 92% *n*-heptane) and separation to obtain 400 mg of a pure product 1-44-2, yield 52%. ¹H NMR (400 MHz, CDCl₃): *δ* 5.36 (m, 2H), 4.60 (m, 2H), 4.09-4.07 (m, 4H), 2.36-2.29 (m, 9H), 2.03-1.83 (m, 14H), 1.62-0.86 (m, 72H), 0.68(s,6H).

### Step 3: Synthesis of Compound 1-44

To a 50 mL flask was added Compound 1-44-2 (400 mg, 0.38 mmol) dissolved in DCM (12 mL) and TFA (4 mL) was added. After the mixture was stirred at room temperature for 2 h, the extent of the reaction was detected by TLC (20% EtOAc/n-heptane). After completion of the reaction, the reaction solution was concentrated, followed by adding MTBE (20 mL) for dissolution. Then the pH was adjusted to 8-9 using a 10% NaHCO₃ solution, and to 1-2 using 1 M diluted hydrochloric acid. The solution was stirred for 20 min and layered. The aqueous phase was extracted with MTBE (20 mL), and the organic phases were combined, dried over anhydrous Na₂SO₄, and filtered. The organic phase was concentrated to obtain 520 mg of a crude product. Flash columns were run for purification (using 8 g of silica gel; the mobile phases were respectively: 200 mL of *n*-heptane, 100 mL of 1% EtOAc + 99% *n*-heptane, 100 mL of 5% EtOAc + 95% *n*-heptane, 100 mL of 10% EtOAc + 90% *n*-heptane, and 200 mL of 20% EtOAc + 80% *n-*heptane) and separation to obtain 250 mg of a pure product, Compound 1-44, yield 67%.

The NMR results of Compound 1-44 are shown in FIG. 13. 1H NMR (400 MHz, CDCl₃): *δ* 5.39-5.35 (m, 2H), 4.71-4.68 (m, 1H), 4.61-4.56 (m, 1H), 4.19-4,14 (m, 4H), 2.40-2.20 (m, 8H), 2.03-1.83 (m, 6H), 1.65-0.85 (m, 72H), 0.68(s,6H).

The synthesis method of Compound 1-45 is as follows:

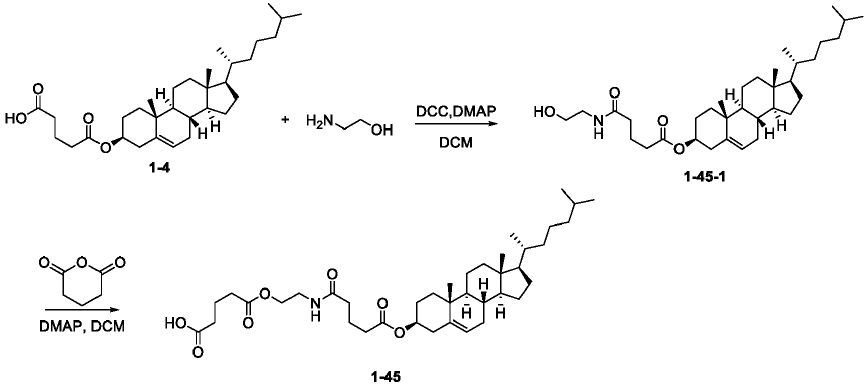

### Step 1: Synthesis of Compound 1-45-1

To a 100 mL flask was added ethanolamine (341.6 mg, 5.59 mmol) dissolved in DCM (30 mL), and DMAP (136.6 mg, 1.12 mmol) and DCC (1.15 g, 5.59 mmol) were added. After stirred at room temperature for 30 min, the solution was added with Compound 1-4 (2.8 g, 5.59 mmol) and stirred at room temperature overnight. The extent of the reaction was detected by TLC (10% MeOH/EtOAc). After completion of the reaction, the reaction solution was filtered to remove solids, and the filtrate was washed with 5 mL of water and 5 mL of saturated brine, dried over anhydrous Na₂SO₄, filtered and concentrated to obtain about 3.4 g of a crude product. Flash columns were run for purification (using 120 g of silica gel; the mobile phases were respectively: 300 mL of DCM, 200 mL of 1% MeOH + 99% DCM, 200 mL of 2% MeOH + 98% DCM, 300 mL of 3% MeOH + 97% DCM and 800 mL of 5% MeOH + 95% DCM) and separation to obtain 2.2 g of a pure product 1-45-1, yield 73%. ¹H NMR (400 MHz, CDCl₃): *δ* 6.18 (s, 1H), 5.37-5.36 (m, 1H), 4.62-4.58 (m, 1H), 3.73-3.70 (m, 2H), 3.48 (s, 1H), 3.43-3.39 (m, 2H), 3.06 (s, 1H), 2.37-2.25 (m, 6H), 2.11-1.79 (m, 13H), 1.61-0.85 (m, 43H), 0.68-0.68 (m, 3H).

### Step 2: Synthesis of Compound 1-45

To a 100 mL flask were added glutaric anhydride (209.81 mg, 1.84 mmol), DMAP (44.5 mg, 0.37 mmol), and DCM (10 mL). After stirred at room temperature for 30 min, the reaction solution was added with Compound 1-46-1 (1.0 g, 1.84 mmol). The extent of the reaction was detected by TLC (5% MeOH/DCM). After completion of the reaction, the reaction solution was filtered to remove solids, and the filtrate was washed with 5 mL of water and 5 mL of saturated brine, dried over anhydrous Na₂SO₄, filtered and concentrated to obtain about 1.7 g of a crude product. Flash columns were run for purification (using 120 g of silica gel; the mobile phases were respectively: 300 mL of DCM, 200 mL of 1% MeOH + 99% DCM, 200 mL of 2% MeOH + 98% DCM, 300 mL of 3% MeOH + 97% DCM and 300 mL of 5% MeOH + 95% DCM) and separation to obtain 180 mg of a pure product, Compound 1-45, yield 15%.

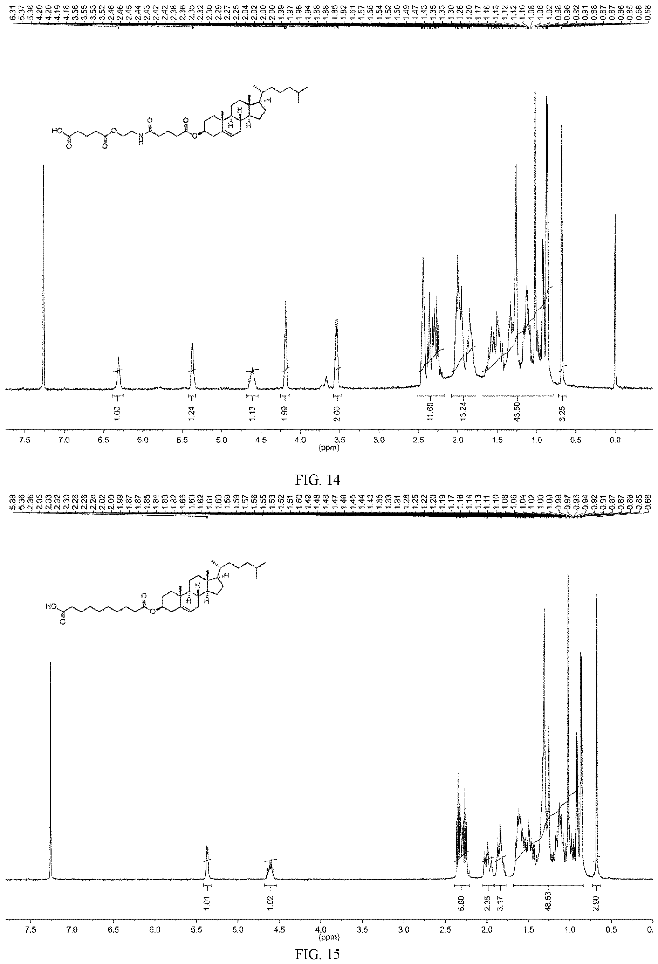

The NMR results of Compound 1-45 are shown in FIG. 14. 1H NMR (400 MHz, CDCl₃): *δ* 6.31 (s, 1H), 5.37-5.36 (m, 1H), 4.65-4.61 (m, 1H), 4.20-4.18 (m, 2H), 3.56-3.52 (m, 2H), 2.46-2.24 (m, 12H), 2.04-1.82 (m, 13H), 1.61-0.85 (m, 43H), 0.68 (m, 3H).

The synthesis method of Compound 1-47 is as follows:

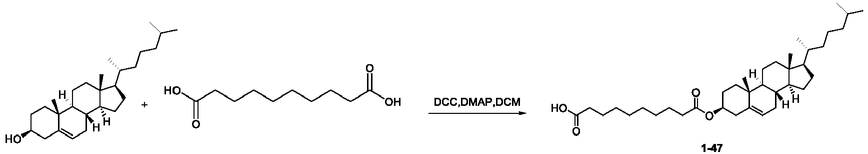

To a 100 mL flask were added DCC (1.52 g, 7.37 mmol, 1.5 eq), DMAP (300.16 mg, 2.46 mmol, 0.5 eq), and sebacic acid (1.49 g, 7.37 mmol, 1.5 eq) dissolved in DCM (40 mL). The mixture was stirred for 30 min. Cholesterol (1.9 g, 4.91 mmol, 1.0 eq) was added to the solution, and the solution was stirred overnight at room temperature after the dropwise addition was finished. The extent of the reaction was detected by TLC (35% EtOAc/*n*-heptane). After completion of the reaction, the solution was filtered, and the filtrate was washed with 5 mL of water and 5 mL of saturated brine. The organic phase was dried over anhydrous Na₂SO₄, and filtered. The organic phase was concentrated to obtain about 3 g of a crude product. Flash columns were run for purification (using 80 g of silica gel, loaded with 500 mL of *n*-heptane; the mobile phases were respectively: 200 mL of *n*-heptane, 100 mL of 0.5% EtOAc + 99.5% *n*-heptane, 100 mL of 0.8% EtOAc + 99.2% *n*-heptane, 100 mL of 1% EtOAc + 99% *n*-heptane, 100 mL of 1.5% EtOAc + 98.5% n-heptane, 100 mL of 2% EtOAc + 98% n-heptane, 100 mL of 3% EtOAc + 97% *n*-heptane, and 1000 mL of 10% EtOAc + 90% n-heptane) and separation to obtain 1 g of a pure product, Compound 1-47, yield 36%.

The NMR results of Compound 1-47 are shown in FIG. 15. 1H NMR (400 MHz, CDCl₃): *δ* 5.38-5.36 (m, 1H), 4.65-4.57 (m, 1H), 2.36-2.24 (m, 6H), 2.03-1.95 (m, 2H), 1.87-1.78 (m, 3H), 1.65-0.85 (m, 48H), 0.68 (s, 3H).

The synthesis method of Compound 1-48 is as follows:

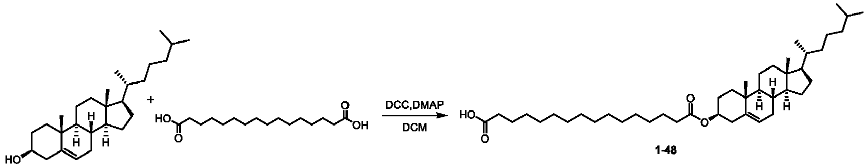

To a 100 mL flask were added DCC (1.04 g, 5.04 mmol, 1.3 eq), DMAP (236.9 mg, 1.94 mmol, 0.5 eq), and 16-diacid (1.45 g, 5.04 mmol, 1.3 eq) dissolved in DCM (30 mL) with stirring for 30 min. Cholesterol (1.5 g, 3.88 mmol, 1.0 eq) was added to the solution, and the solution was stirred overnight at room temperature after the dropwise addition was finished. The extent of the reaction was detected by TLC (20% EtOAc/*n*-heptane). After completion of the reaction, the solution was filtered, and the filtrate was washed with 5 mL of water and 5 mL of saturated brine. The organic phase was dried over anhydrous Na₂SO₄, and filtered. The organic phase was concentrated to obtain about 3 g of a crude product. Flash columns were run for purification (using 80 g of silica gel, loaded with 500 mL of *n*-heptane; the mobile phases were respectively: 200 mL of *n*-heptane, 100 mL of 0.5% EtOAc + 99.5% *n*-heptane, 100 mL of 0.8% EtOAc + 99.2% *n*-heptane, 100 mL of 1% EtOAc + 99% *n*-heptane, 100 mL of 1.5% EtOAc + 98.5% *n*-heptane, 100 mL of 2% EtOAc + 98% n-heptane, 100 mL of 3% EtOAc + 97% *n*-heptane, and 600 mL of 10% EtOAc + 90% *n-*heptane) and separation to obtain 700 mg of a pure product, Compound 1-48, yield 28%.

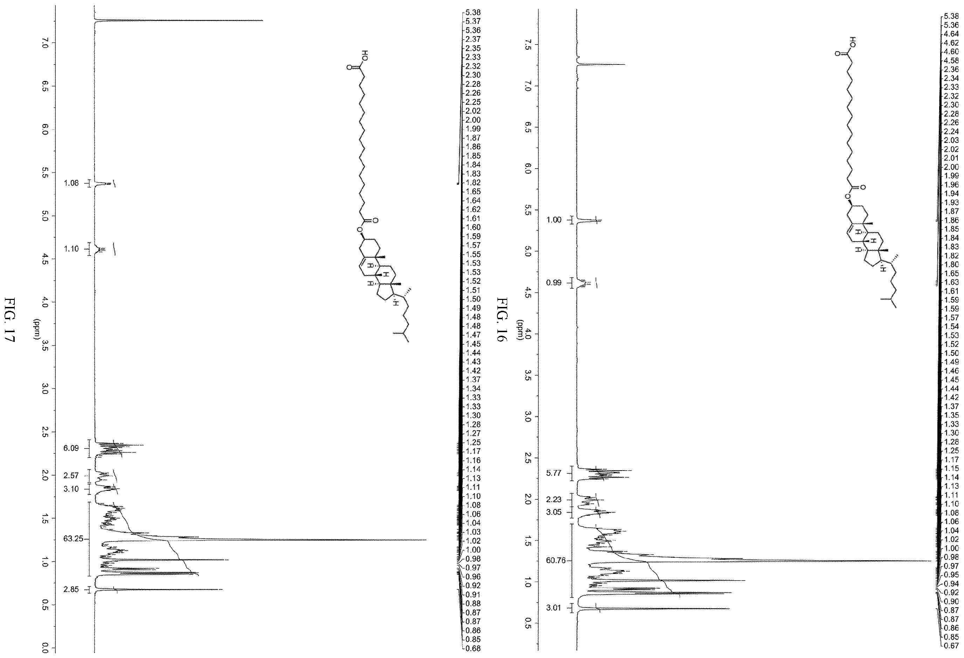

The NMR results of Compound 1-48 are shown in FIG. 16. 1H NMR (400 MHz, CDCl₃): *δ* 5.38-5.36 (m, 1H), 4.65-4.57 (m, 1H), 2.36-2.24 (m, 6H), 2.03-1.93 (m, 2H), 1.87-1.80 (m, 3H), 1.65-0.85 (m, 60H), 0.67 (s, 3H).

The synthesis method of Compound 1-49 is as follows:

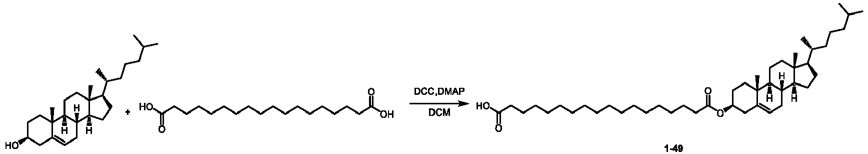

To a 100 mL flask were added DCC (1.04 g, 5.04 mmol, 1.3 eq), DMAP (236.9 mg, 1.94 mmol, 0.5 eq), and 18-diacid (1.59 g, 5.04 mmol, 1.3 eq) dissolved in DCM (30 mL) with stirring for 30 min. Cholesterol (1.5 g, 3.88 mmol, 1.0 eq) was added to the solution, and the solution was stirred overnight at room temperature after the dropwise addition was finished. The extent of the reaction was detected by TLC (20% EtOAc/*n*-heptane). After completion of the reaction, the solution was filtered, and the filtrate was washed with 5 mL of water and 5 mL of saturated brine. The organic phase was dried over anhydrous Na₂SO₄, and filtered. The organic phase was concentrated to obtain about 3 g of a crude product. Flash columns were run for purification (using 80 g of silica gel, loaded with 500 mL of *n*-heptane; the mobile phases were respectively: 200 mL of *n*-heptane, 100 mL of 0.5% EtOAc + 99.5% *n*-heptane, 100 mL of 0.8% EtOAc + 99.2% *n*-heptane, 100 mL of 1% EtOAc + 99% *n*-heptane, 100 mL of 1.5% EtOAc + 98.5% n-heptane, 100 mL of 2% EtOAc + 98% *n*-heptane, and 600 mL of 3% EtOAc + 97% *n*-heptane) and separation to obtain 500 mg of a pure product, Compound 1-49, yield 19%.

The NMR results of Compound 1-49 are shown in FIG. 17. 1H NMR (400 MHz, CDCl₃): *δ* 5.38-5.36 (m, 1H), 4.62-4.58 (m, 1H), 2.37-2.21 (m, 6H), 2.02-1.95 (m, 2H), 1.87-1.82 (m, 3H), 1.67-0.85 (m, 63H), 0.68 (s, 3H).

The synthesis method of Compound 1-50 is as follows:

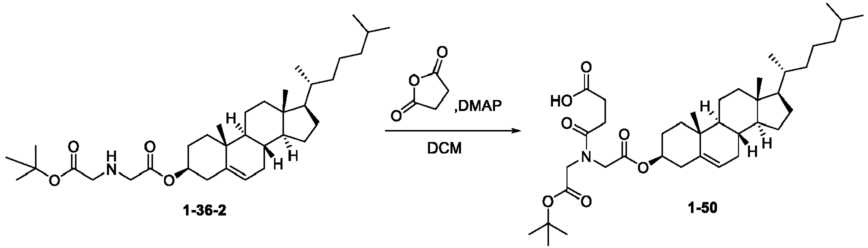

To a 50 mL flask were added succinic anhydride (699.6 mg, 7.0 mmol), DMAP (328.5 mg, 2.7 mmol), and DCM (30 mL). After stirred at room temperature for 30 min, the reaction solution was added with Compound 1-36-2 (3.0 g, 5.4 mmol). The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 4.5 g of a crude product. Flash columns were run for purification (using 20 g of silica gel; the mobile phases were respectively: 500 mL of *n*-heptane, 300 mL of 1% EtOAc + 99% *n*-heptane, 300 mL of 2% EtOAc + 98% *n*-heptane, 200 mL of 5% EtOAc + 95% n-heptane, and 1000 mL of 8% EtOAc + 92% *n*-heptane) and separation to obtain 2.5 g a pure product, Compound 1-50, yield 70%.

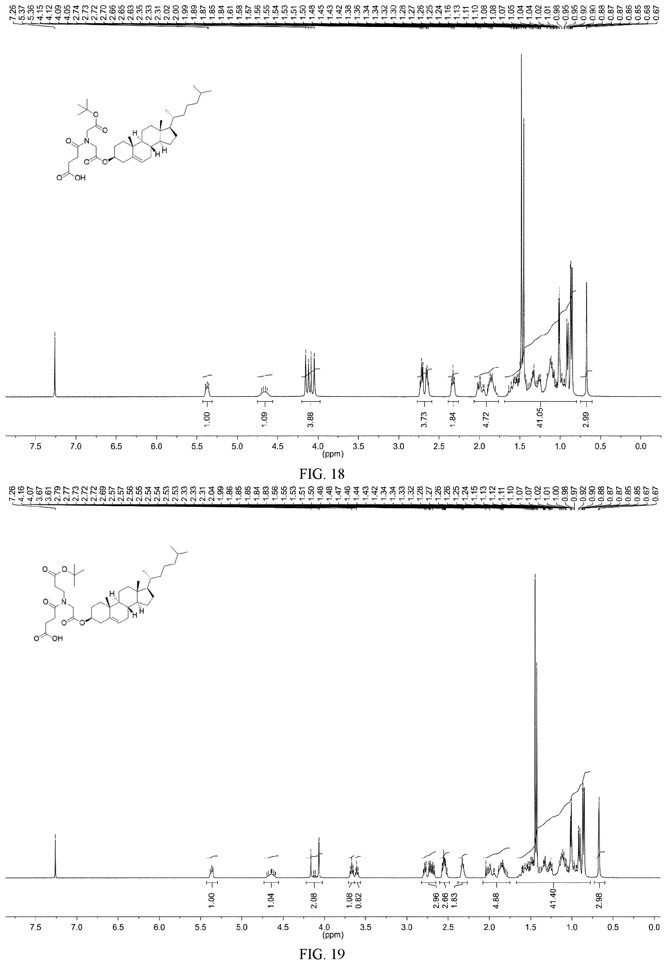

The NMR results of Compound 1-50 are shown in FIG. 18. 1H NMR (400 MHz, CDCl₃): *δ* 5.39-5.36 (m, 1H), 4.70-4.63 (m, 1H), 4.15-4.05 (m, 4H), 2.74-2.63 (m, 4H), 2.33 (t, *J*=8Hz, 2H), 2.03-1.80 (m, 5H), 1.64-0.85 (m, 41H), 0.68-0.67 (m, 3H).

The synthesis method of Compound 1-51 is as follows:

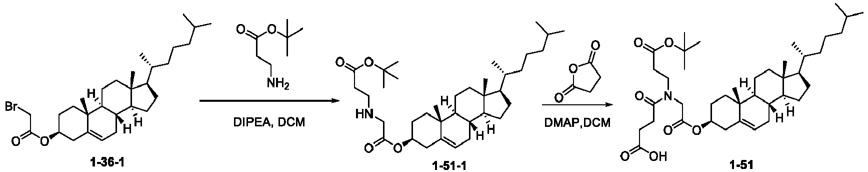

### Step 1: Synthesis of Compound 1-51-1

To a 50 mL flask were added Compound 1-36-1 (4.0 g, 7.9 mmol) and DIPEA (2.3 g, 15.8 mmol) dissolved in DCM (40 mL), and tert-butyl 3-carbamate (4.1 g, 31.6 mmol) was added. The mixture was stirred at room temperature overnight. The extent of the reaction was detected by TLC (30% EtOAc/*n*-heptane). After completion of the reaction, the filtrate was washed with 10 mL of water and 10 mL of saturated brine. The organic phase was dried over anhydrous Na₂SO₄, and filtered. The organic phase was concentrated to obtain about 12.1 g of a crude product. Flash columns were run for purification (using 100 g of silica gel, loaded with 300 mL of *n*-heptane; the mobile phases were respectively: 300 mL of *n*-heptane, 300 mL of 0.1% EtOAc + 99.9% *n*-heptane, 300 mL of 0.3% EtOAc + 99.7% *n*-heptane, and 1000 mL of 0.5% EtOAc + 99.5% *n*-heptane) and separation to obtain 2.3 g of a pure product 1-51-1, yield 51%.

### Step 2: Synthesis of Compound 1-51

To a 50 mL flask were added succinic anhydride (546 mg, 5.5 mmol), DMAP (256.4 mg, 2.1 mmol), and DCM (30 mL). After the solution was stirred at room temperature for 30 min, the 1-51-1(2.4 g, 4.2 mmol) was added to the reaction mixture. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 2.0 g of a crude product. Flash columns were run for purification (using 20 g of silica gel; the mobile phases were respectively: 300 mL of 1% EtOAc + 99% *n*-heptane, 300 mL of 2% EtOAc + 98% *n*-heptane, 200 mL of 5% EtOAc + 95% n-heptane, 300 mL of 8% EtOAc + 92% n-heptane, and 500 mL of 20% EtOAc + 80% *n*-heptane) and separation to obtain 1.3 g of a pure product, Compound 1-51, yield 46%.

The NMR results of Compound 1-51 are shown in FIG. 19. 1H NMR (400 MHz, CDCl₃): *δ* 5.38-5.35 (m, 1H), 4.70-4.59 (m, 1H), 4.16-4.07 (m, 4H), 3.67 (t, *J*=8Hz, 1H), 3.61 (t, *J*=8Hz, 1H), 2.80-2.67 (m, 3H), 2.57-2.51 (m, 3H), 2.34-2.31 (m, 2H), 2.04-1.78 (m, 5H), 1.61-0.84 (m, 41H), 0.67 (m, 3H).

The synthesis method of Compound 1-52 is as follows:

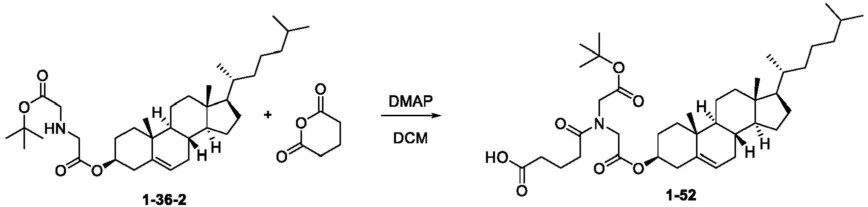

To a 50 mL flask were added glutaric anhydride (204.53 mg, 1.79 mmol), DMAP (109.5 mg, 0.89 mmol), and DCM (10 mL). After the mixture was stirred at room temperature for 30 min, the 1-36-2 (1.0 g, 1.79 mmol) was added to the reaction solution. The extent of the reaction was detected by TLC (5% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 4.5 g of a crude product. Flash columns were run for purification (using 20 g of silica gel; the mobile phases were respectively: 500 mL of *n*-heptane, 300 mL of 1% MeOH + 99% DCM, 300 mL of 1.5% MeOH + 98.5% DCM, and 600 mL of 2% MeOH + 98% DCM) and separation to obtain 1.1 g of a pure product, Compound 1-52, yield 92%.

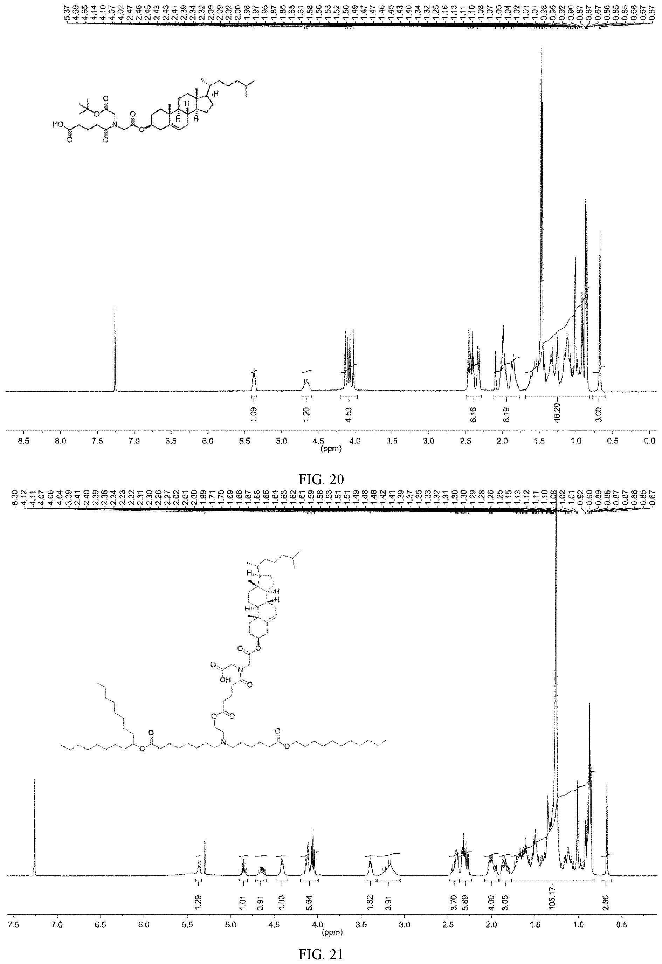

The NMR results of Compound 1-52 are shown in FIG. 20. 1H NMR (400 MHz, CDCl₃): *δ* 5.37 (m, 1H), 4.69-4.65 (m, 1H), 4.14-4.02 (m, 4H), 2.47-2.32 (m, 6H), 2.09-1.85 (m, 8H), 1.58-0.85 (m, 46H), 0.67 (t, *J*=4Hz, 3H).

The synthesis method of Compound 1-53 is as follows:

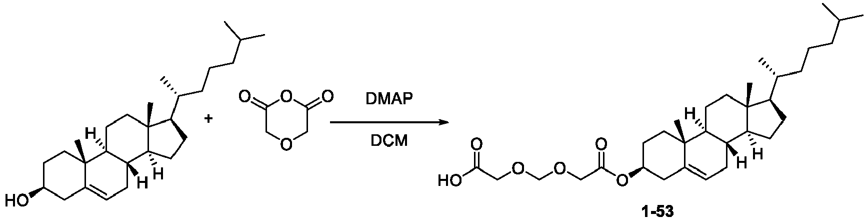

To a 100 mL flask were added cholesterol (1.0 g, 2.59 mmol, 1.0 eq), DMAP (315.9 mg, 2.59 mmol, 1.0 eq), and diethylene glycol anhydride (360.23 mg, 3.1 mmol, 1.2 eq) dissolved in DCM (10 mL). After addition, the solution was stirred overnight at room temperature. The extent of the reaction was detected by TLC (20% EtOAc/n-heptane). After completion of the reaction, 5 mL of water and 5 mL of saturated brine were added for rinse. The organic phase was dried over anhydrous Na₂SO₄, and filtered. The organic phase was concentrated to obtain about 2 g of a crude product. Flash columns were run for purification (using 60 g of silica gel, loaded with 500 mL *n-*heptane; the mobile phases were respectively: 200 mL of *n*-heptane, 100 mL of 1% EtOAc + 99% *n*-heptane, 100 mL of 1.5% EtOAc + 98.5% n-heptane, 100 mL of 2% EtOAc + 98% n-heptane, and 600 mL of 3% EtOAc + 97% *n*-heptane) and separation to obtain 500 mg of a pure product 1-53, yield 40%. ¹H NMR (400 MHz, CDCl₃): *δ* 5.39-5.37 (m, 1H), 4.73-4.69 (m, 1H), 4.20 (s, 2H), 2.35-2.33 (m, 2H), 2.03-1.94 (m, 2H), 1.90-1.80 (m, 3H), 1.63-0.85 (m, 36H), 0.67 (s, 3H).

The synthesis method of Compound 1-55 is as follows:

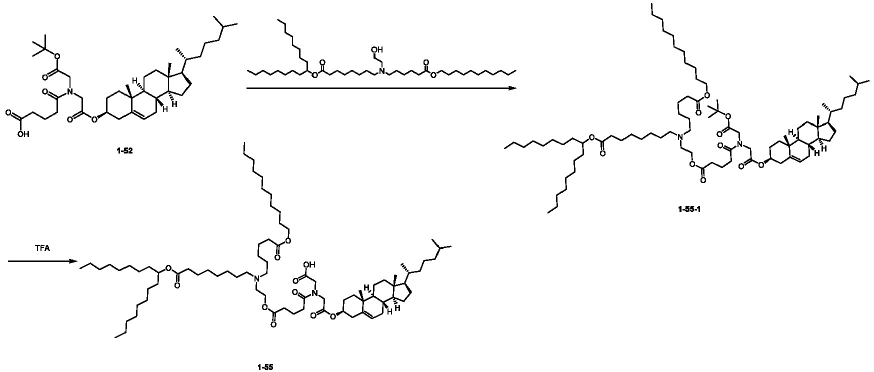

### Step 1: Synthesis of Compound 1-55-1

To a 50 mL flask was added Compound 1-52 (473.1 mg, 0.7 mmol) dissolved in DCM (5 mL), and DMAP (18.2 mg, 0.15 mmol) and DCC (230.3 mg, 1.12 mmol) were added. After stirred at room temperature for 30 min, the solution was added with SM-102 (500 mg, 0.7 mmol) and stirred at room temperature overnight. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 700 mg of a crude product. Flash columns were run for purification (using 12 g of silica gel; the mobile phases were respectively: 300 mL of *n*-heptane, 200 mL of 1% EtOAc+99% *n*-heptane, 200 mL of 5% EtOAc+95% *n*-heptane and 300 mL of 8% EtOAc+92% *n*-heptane) and separation to obtain 550 mg of a pure product 1-55-1, yield 57%.

### Step 2: Synthesis of Compound 1-55

To a 50 mL flask were added Compound 1-55-1 (550 mg, 0.4 mmol) dissolved in DCM (6 mL), and TFA (3 mL) was added. After the mixture was stirred at room temperature for 2 h, the extent of the reaction was detected by TLC (20% EtOAc/*n*-heptane). After completion of the reaction, the reaction solution was concentrated, followed by adding MTBE (20 mL) for dissolution. Then the pH was adjusted to 8-9 using a 10% NaHCOs solution, and to 1-2 using diluted hydrochloric acid. The solution was stirred for 20 min and layered. The aqueous phase was extracted with 20 mL of MTBE, and the organic phases were combined, dried over anhydrous Na₂SO₄, and filtered. The organic phase was concentrated to obtain 520 mg of a crude product. Flash columns were run for purification (using 8 g of silica gel; the mobile phases were respectively: 200 mL of *n*-heptane, 100 mL of 1% EtOAc + 99% n-heptane, 100 mL of 5% EtOAc + 95% *n*-heptane, 100 mL of 10% EtOAc + 90% *n*-heptane, and 200 mL of 20% EtOAc + 80% n-heptane) and separation to obtain 200 mg of a pure product, Compound 1-55, yield 38%.

The NMR results of Compound 1-55 are shown in FIG. 21. 1H NMR (400 MHz, CDCl₃): *δ* 5.37-5.30 (m, 1H), 4.88-4.82 (m, 1H), 4.69-4.61 (m, 1H), 4.42-4.39 (m, 2H), 4.18-4.04 (m, 6H), 3.40-3.38 (m, 2H), 3.25-3.16 (m, 4H), 2.46-2.38 (m, 4H), 2.34-2.27 (m, 6H), 2.04-1.94 (m, 4H), 1.88-1.79 (m, 3H), 1.73-0.85 (m, 105H), 0.67(s, 3H).

The synthesis method of Compound 1-56 is as follows:

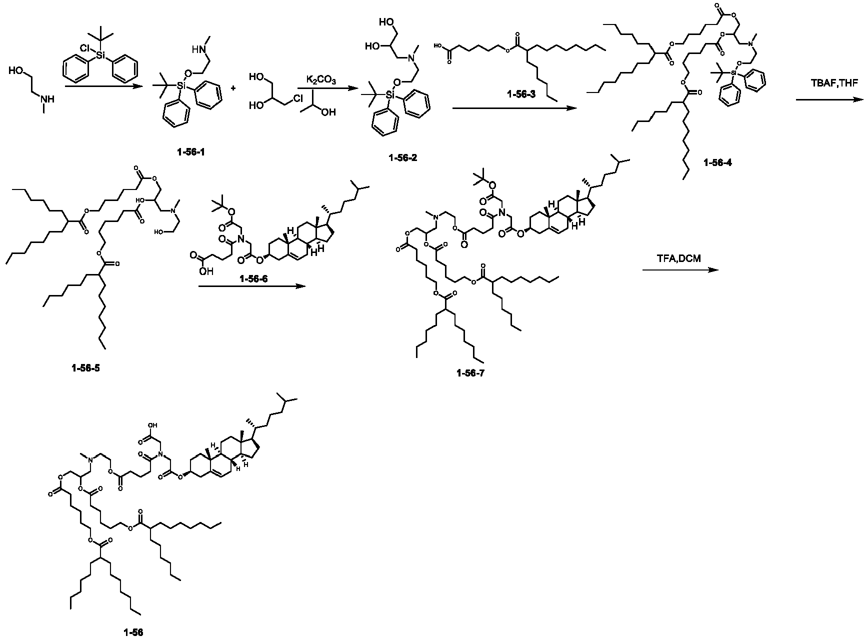

### Step 1: Synthesis of Compound 1-56-1

To a 500 mL flask were added N-methylethanolamine (3.4 g, 45.3 mmol) and DCM (50 mL). The mixture was cooled down to 0-5 °C in an ice-salt bath. A solution of TBDPSCI (12.44 g, 45.3 mmol) in DCM (50 mL) was slowly added to the solution dropwise. After the dropwise addition was finished, the solution was stirred at room temperature for 2 h. The extent of the reaction was detected by TLC (10% MeOH/DCM). After completion of the reaction, 30 mL of water was added to quench the reaction, and the solution was separated. The aqueous phase was extracted with DCM (50 mL × 2). The organic phase were combined and washed with 50 mL of saturated brine. The organic phase was dried over anhydrous Na₂SO₄, and filtered. The organic phase was concentrated to obtain about 12 g of a crude product. Flash columns were run for purification (using 200 g of silica gel, loaded with 300 mL of *n*-heptane; the mobile phases were respectively: 300 mL of *n*-heptane, 300 mL of 0.1% EtOAc + 99.9% *n*-heptane, 300 mL of 0.3% EtOAc + 99.7% *n*-heptane, 300 mL of 1% EtOAc + 99% *n*-heptane, 300 mL of 1% EtOAc + 99% *n*-heptane, 300 mL of 0.2% MeOH + 99.8% DCM, and 500 mL of 0.3% MeOH + 99.7% DCM) and separation to obtain 9 g of a pure product 1-56-1, yield 63%.

### Step 2: Synthesis of Compound 1-56-2

To a 100 mL flask were added Compound 1-56-1 (5.0 g, 16 mmol), potassium carbonate (5.5 g, 39.9 mmol), and 3-chloro-1,2-propanediol (2.12 g, 19.1 mmol) dissolved in isopropanol (15 mL). The mixture was heated to 80 °C and stirred overnight. The extent of the reaction was detected by TLC (10% MeOH/DCM). After completion of the reaction, the organic phase was filtered and concentrated to obtain about 10.1 g of a crude product. Flash columns were run for purification (using 100 g of silica gel; the mobile phases were respectively: 200 mL of 0.1% MeOH + 99.9% DCM, 200 mL of 0.2% MeOH + 99.8% DCM, 200 mL of 0.3% MeOH + 99.7% DCM, 200 mL of 0.5% MeOH + 99.5% DCM, and 300 mL of 1% MeOH + 99% DCM) and separation to obtain 3.6 g of a pure product 1-56-2, yield 58%.

### Step 3: Synthesis of Compound 1-56-4

To a 50 mL flask were added Compound 1-56-3 (1.0 g, 2.7 mmol), DCC (556.8 mg, 2.7 mmol), DMAP (82.4 mg, 0.67 mmol), and DCM (3 mL). After stirred at room temperature for 30 min, the reaction solution was added with Compound 1-56-2 (523 mg, 1.35 mmol). The extent of the reaction was detected by TLC (30% EtOAc/n-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 1.5 g of a crude product. Flash columns were run for purification (using 50 g of silica gel; the mobile phases were respectively: 200 mL of *n*-heptane, 150 mL of 1% EtOAc + 99% *n*-heptane, 150 mL of 2% EtOAc + 98% *n*-heptane, 200 mL of 5% EtOAc + 95% *n*-heptane, and 500 mL of 8% EtOAc + 92% *n*-heptane) and separation to obtain 1.2 g of a pure product 1-56-4, yield 81%.

### Step 4: Synthesis of Compound 1-56-5

To a 50 mL flask was added Compound 1-56-4 (1.0 g, 0.92 mmol) dissolved in THF (5 mL). The mixture was cooled down to -5 °C in an ice-salt bath, added with TBAF (717.9 mg, 2.75 mmol), and stirred at room temperature overnight. The extent of the reaction was detected by TLC (10% MeOH/DCM). After completion of the reaction, 5 mL of an aqueous ammonium chloride solution was added to quench the reaction, and the solution was separated. The aqueous phase was extracted with EtOAc (10 mL × 2). The organic phases were combined and washed with 5 mL of saturated brine. The organic phase was dried over anhydrous Na₂SO₄, and filtered. The organic phase was concentrated to obtain about 700 mg of a crude product. Flash columns were run for purification (using 12 g of silica gel; the mobile phases were respectively: 300 mL of *n*-heptane, 200 mL of 1% EtOAc + 99% n-heptane, 200 mL of 5% EtOAc + 95% n-heptane, 200 mL of 8% EtOAc + 92% *n*-heptane, 200 mL of 15% EtOAc + 85% *n*-heptane and 400 mL of 30% EtOAc + 70% *n-*heptane) and separation to obtain 350 mg of a pure product 1-56-5, yield 45%.

### Step 5: Synthesis of Compound 1-56-7

To a 50 mL flask were added Compound 1-56-6 (113.9 mg, 0.17 mmol), DCC (35 mg, 0.17 mmol), DMAP (10.4 mg, 0.08 mmol), and DCM (5 mL). After stirred at room temperature for 30 min, the reaction solution was added with Compound 1-56-5 (140 mg, 0.17 mmol). The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 350 mg of a crude product. Flash columns were run for purification (using 20 g of silica gel; the mobile phases were respectively: 100 mL of *n*-heptane, 100 mL of 1% EtOAc + 99% *n*-heptane, 100 mL of 2% EtOAc + 98% n-heptane, 100 mL of 5% EtOAc + 95% n-heptane, 100 mL of 8% EtOAc + 92% *n*-heptane and 200 mL of 20% EtOAc + 80% n-heptane) and separation to obtain 350 mg of a pure product 1-56-7, yield 60%.

### Step 6: Synthesis of Compound 1-56

To a 50 mL flask was added Compound 1-56-7 (180 mg, 0.12 mmol) dissolved in EtOAc (1 mL), and a solution (1 mL) of 4 M hydrogen chloride in ethyl acetate was added. After the mixture was stirred at room temperature for 2 h, the extent of the reaction was detected by TLC (20% MeOH/DCM). The organic phase was concentrated to obtain 210 mg of a crude product. Flash columns were run for purification (using 8 g of silica gel; the mobile phases were respectively: 100 mL of *n*-heptane, 100 mL of 5% EtOAc + 95% n-heptane, 100 mL of 10% EtOAc + 90% *n-*heptane, 100 mL of 20% EtOAc + 80% *n*-heptane, and 200 mL of 30% EtOAc + 70% *n-*heptane) and separation to obtain 50 mg of a pure product, Compound 1-56, yield 29%.

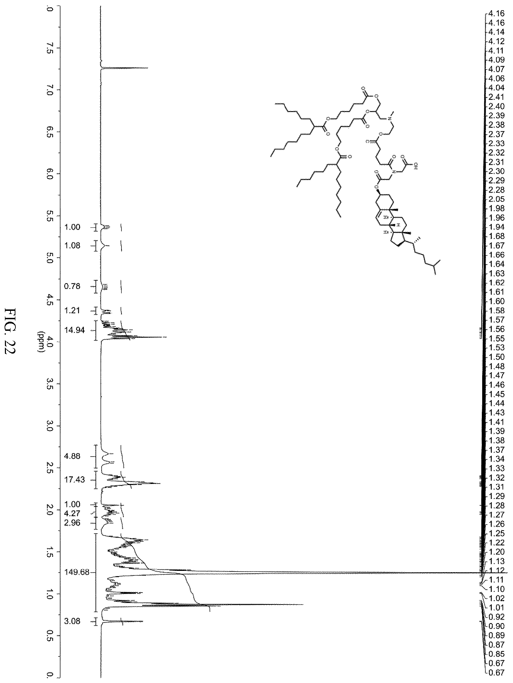

The NMR results of Compound 1-56 are shown in FIG. 22. 1H NMR (400 MHz, CDCl₃): *δ* 5.39-5.34 (m, 1H), 5.15 (m, 1H), 4.69-4.62 (m, 1H), 4.38-4.34 (m, 1H), 4.25-4.04 (m, 15H), 2.67-2.55 (m, 5H), 2.42-2.26 (m, 17H), 2.05-1.92 (m, 5H), 1.89-1.84 (m, 3H), 1.69-0.80 (m, 150H), 0.67 (m, 3H).

DODMA, DSPC, cholesterol, PEG2000-DMG, etc. used in the following examples are all from AVT (Shanghai) Pharmaceutical Tech Co., Ltd., and Luc-mRNA is from Shanghai Hongene Biotech Corporation.

### Example 2: Preparation of Lipid Nanoparticle TMF1 (containing 20% Compound 1-1)

1. DODMA (cationic lipid), DSPC (helper lipid), cholesterol (helper lipid), PEG2000-DMG (helper lipid), and Compound 1-1 (GOLD lipid) were dissolved with ethanol to obtain oil phase (ethanol phase) stock solutions at molar ratios of 37.0%, 7.5%, 34.2%, 1.3%, and 20.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF1 at a weight ratio of total lipid to mRNA of 22.5:1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 3: Preparation of Lipid Nanoparticle TMF2 (containing 5% Compound 1-1):

1. DODMA (cationic lipid), DSPC (helper lipid), cholesterol (helper lipid), PEG2000-DMG (helper lipid), and Compound 1-1 (GOLD lipid) were dissolved with ethanol to obtain oil phase (ethanol phase) stock solutions at molar ratios of 44.0%, 8.9%, 40.6%, 1.5%, and 5.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF2 at a weight ratio of total lipid to mRNA of 22.6: 1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 4: Preparation of Lipid Nanoparticle TMF3 (containing 20% Compound 1-2):

1. DODMA (cationic lipid), DSPC (helper lipid), cholesterol (helper lipid), PEG2000-DMG (helper lipid), and Compound 1-2 (GOLD lipid) were dissolved with ethanol to obtain oil phase (ethanol phase) stock solutions at molar ratios of 37.0%, 7.5%, 34.2%, 1.3%, and 20.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF3 at a weight ratio of total lipid to mRNA of 24.2:1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 5: Preparation of Lipid Nanoparticle TMF4 (containing 20% Compound 1-3):

1. DODMA (cationic lipid), DSPC (helper lipid), sitosterol (helper lipid), PEG2000-DMG (helper lipid), and Compound 1-3 (GOLD lipid) were dissolved with ethanol to obtain oil phase (ethanol phase) stock solutions at molar ratios of 37.0%, 7.5%, 34.2%, 1.3%, and 20.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF4 at a weight ratio of total lipid to mRNA of 24.5: 1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 6: Preparation of Lipid Nanoparticle TMF5 (containing 20% Compound 1-5):

1. DODMA (cationic lipid), DSPC (helper lipid), cholesterol (helper lipid), PEG2000-DMG (helper lipid), and Compound 1-5 (GOLD lipid) were dissolved with ethanol to obtain oil phase (ethanol phase) stock solutions at molar ratios of 37.0%, 7.5%, 34.2%, 1.3%, and 20.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF5 at a weight ratio of total lipid to mRNA of 24.8: 1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 7: Preparation of Lipid Nanoparticle TMF6 (containing 20% Compound 1-6):

1. DODMA (cationic lipid), DOPE (helper lipid), cholesterol (helper lipid), PEG2000-DMG (helper lipid), and Compound 1-6 (GOLD lipid) were dissolved with ethanol to obtain oil phase (ethanol phase) stock solutions at molar ratios of 37.0%, 7.5%, 34.2%, 1.3%, and 20.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF6 at a weight ratio of total lipid to mRNA of 24.7: 1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 8: Preparation of Lipid Nanoparticle TMF7 (containing 38.5% Compound 1-1):

1. DODMA (cationic lipid), DSPC (helper lipid), Compound 1-1 (GOLD lipid), and PEG2000-DMG (helper lipid) were dissolved with ethanol to obtain oil phase (ethanol phase) stock solutions at molar ratios of 50%, 10%, 38.5%, and 1.5%, respectively.
2. The luciferase-expressing mRNA stock was diluted to 0.3 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF7 at a weight ratio of total lipid to mRNA of 24.9: 1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 9: Preparation of Lipid Nanoparticle TMF8 (containing 25% Compound 1-1):

1. DODMA (cationic lipid), DSPC (helper lipid), and Compound 1-1 (GOLD lipid) were dissolved with ethanol to obtain oil phase (ethanol phase) stock solutions at molar ratios of 50%, 25%, and 25%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF8 at a weight ratio of total lipid to mRNA of 30.7:1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 10: Preparation of Lipid Nanoparticle TMF9 (containing 40% Compound 1-1):

1. DODMA (cationic lipid) and Compound 1-1 (GOLD lipid) were dissolved with ethanol to obtain oil phase (ethanol phase) stock solutions at molar ratios of 60% and 40%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF9 at a weight ratio of total lipid to mRNA of 31.4: 1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 11: Preparation of Lipid Nanoparticle TMF10 (containing 38.5% Compound 1-1 and 10% Compound 1-56):

1. DODMA (cationic lipid), Compound 1-56 (GOLD lipid), compound 1-1 (GOLD lipid), and PEG2000-DMG (helper lipid) were dissolved with ethanol to obtain oil phase (ethanol phase) stock solutions at molar ratios of 50%, 10%, 38.5%, and 1.5%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF10 at a weight ratio of total lipid to mRNA of 26.2:1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

### Example 12: Preparation of Lipid Nanoparticle TMF38 (containing 100% Compound 1-56):

1. Compound 1-56 (GOLD lipids) was dissolved with ethanol to obtain a 100% oil phase (ethanol phase) stock solution.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to prepare TMF38 at a weight ratio of total lipid to mRNA of 24.8: 1. Then the sample was concentrated with an ultrafiltration tube having a molecular weight cut-off of 100 kd, followed by washing with pH 7.4 PBS buffer, and finally replaced with 120 mg/mL sucrose PBS buffer to obtain the final sample.

Lipid nanoparticles TMF11-37 were prepared in a similar manner to lipid nanoparticles TMF7, using the corresponding starting materials. TMF38 was prepared in a similar manner to TMF9, using the corresponding starting materials. The details of the lipid nanoparticle component are shown in Table 3.

**Table 3: List of lipid nanoparticle components prepared from representative GOLD lipid compounds**

| | Cationic lipid | Helper lipid | | | Gold lipid |
|---|---|---|---|---|---|
| TMF1 | DODMA 37% | DSPC 7.5% | Cholesterol 34.2% | PEG2000-DMG 1.3% | Compound 1-1 20% |
| TMF2 | DODMA 44% | DSPC 8.9% | Cholesterol 40.6% | PEG2000-DMG 1.5% | Compound 1-1 5% |
| TMF3 | DODMA 37% | DSPC 7.5% | Cholesterol 34.2% | PEG2000-DMG 1.3% | Compound 1-2 20% |
| TMF4 | DODMA 37% | DSPC 7.5% | Sitosterol 34.2% | PEG2000-DMG 1.3% | Compound 1-3 20% |
| TMF5 | DODMA 37% | DSPC 7.5% | Cholesterol 34.2% | PEG2000-DMG 1.3% | Compound 1-5 20% |
| TMF6 | DODMA 37% | DOPE 7.5% | Cholesterol 34.2% | PEG2000-DMG 1.3% | Compound 1-6 20% |
| TMF7 | DODMA 50% | DSPC 10% | - | PEG2000-DMG 1.5% | Compound 1-1 38.5% |
| TMF8 | DODMA 50% | DSPC 25% | - | - | Compound 1-1 25% |
| TMF9 | DODMA 60% | - | - | - | Compound 1-1 40% |
| TMF10 | DODMA 50% | - | - | PEG2000-DMG 1.5% | Compound 1-1 + Compound 1-56 38.5%+10% |
| TMF11 | DODMA 50% | DSPC 10% | - | PEG2000-DMG 1.5% | Compound 1-3 38.5% |
| TMF12 | DODMA 50% | DSPC 10% | - | PEG2000-DMG 1.5% | Compound 1-4 38.5% |
| TMF13 | DODMA 50% | DSPC 10% | - | PEG2000-DMG 1.5% | Compound 1-5 38.5% |
| TMF14 | DODMA 50% | DSPC 10% | - | PEG2000-DMG 1.5% | Compound 1-6 38.5% |
| TMF15 | DODMA 50% | DSPC 10% | - | PEG2000-DMG 1.5% | Compound 1-7 38.5% |
| TMF16 | DODMA 50% | DSPC 10% | - | PEG2000-DMG 1.5% | Compound 1-8 38.5% |
| TMF17 | DODMA 50% | DSPC 10% | - | PEG2000-DMG 1.5% | Compound 1-9 38.5% |
| TMF18 | DODMA 50% | DSPC 10% | - | PEG2000-DMG 1.5% | Compound 1-25 38.5% |
| TMF19 | DODMA 50% | DSPC 10% | - | PEG2000-DMG 1.5% | Compound 1-26 38.5% |
| TMF20 | DODMA 50% | DSPC 10% | - | PEG2000-DMG 1.5% | Compound 1-32 38.5% |
| TMF21 | DODMA 50% | DSPC 10% | - | PEG2000-DMG 1.5% | Compound 1-37 38.5% |
| TMF22 | DODMA 50% | DSPC 10% | - | PEG2000-DMG 1.5% | Compound 1-39 38.5% |
| TMF23 | DODMA 50% | DSPC 10% | - | PEG2000-DMG 1.5% | Compound 1-42 38.5% |
| TMF24 | DODMA 50% | DSPC 10% | - | PEG2000-DMG 1.5% | Compound 1-44 38.5% |
| TMF25 | DODMA 50% | DSPC 10% | - | PEG2000-DMG 1.5% | Compound 1-45 38.5% |
| TMF26 | DODMA 50% | DSPC 10% | - | PEG2000-DMG 1.5% | Compound 1-46 38.5% |
| TMF27 | DODMA 50% | DSPC 10% | - | PEG2000-DMG 1.5% | Compound 1-48 38.5% |
| TMF28 | DODMA 50% | DSPC 10% | - | PEG2000-DMG 1.5% | Compound 1-49 38.5% |
| TMF29 | DODMA 50% | DSPC 10% | - | PEG2000-DMG 1.5% | Compound 1-50 38.5% |
| TMF30 | DODMA 50% | DSPC 10% | - | PEG2000-DMG 1.5% | Compound 1-51 38.5% |
| TMF31 | DODMA 50% | DSPC 10% | - | PEG2000-DMG 1.5% | Compound 1-52 38.5% |
| TMF32 | DODMA 50% | DSPC 10% | - | PEG2000-DMG 1.5% | Compound 1-55 38.5% |
| TMF33 | DODMA 50% | DSPC 10% | - | PEG2000-DMG 1.5% | Compound 1-56 38.5% |
| TMF34 | CKK-E12 50% | DSPC 10% | - | PEG2000-DMG 1.5% | Compound 1-56 38.5% |
| TMF35 | C12-200 50% | DSPC 10% | - | PEG2000-DMG 1.5% | Compound 1-56 38.5% |
| TMF36 | - | DSPC 10% | Cholesterol 38.5% | PEG2000-DMG 1.5% | Compound 1-56 50% |
| TMF37 | - | - | - | PEG2000-DMG 1.5% | Compound 1-56 98.5% |
| TMF38 | - | - | - | | Compound 1-56 100% |

### Example 13: Detection of Lipid Nanoparticles

The size and polydispersity index (PDI) of the lipid nanoparticles were determined at a 90° side scatter angle using a NanoBrook 90plus PLAS (Brookhaven Instruments, US) by the dynamic light scattering (DLS) technology, and the results are shown in Tables 4, 5, and 6. The content and encapsulation efficiency of the lipid nanoparticles were determined using a Qubit RNA HS quantitation assay kit (ThermoFisher Scientific, UK) according to the manufacturer's instructions. The determination results are shown in Tables 4, 5, and 6.

**Table 4: Characterization data for lipid nanoparticles prepared from representative GOLD lipid compounds**

| Lipid nanoparticle No. | GOLD lipid used | Size(nm) | PDI (polydispersity index) | Encapsulation efficiency |
|---|---|---|---|---|
| TMF1 | 20%**1-1** | 156.2±0.9 | 0.134±0.052 | 63.2% |
| TMF2 | 5% **1-1** | 216.1±1.5 | 0.091±0.051 | 67.4% |
| TMF3 | 20% **1-2** | 209.6±3.2 | 0.176±0.001 | 80.6% |
| TMF4 | 20% **1-3** | 217.3±4.5 | 0.102±0.028 | 88.2% |
| TMF5 | 20% **1-5** | 255.9±5.7 | 0.130±0.041 | 65.2% |
| TMF6 | 20% **1-6** | 318.6±2.7 | 0.211±0.050 | 85.8% |
| TMF7 | 38.5% **1-1** | 275.5±1.4 | 0.179±0.018 | 56.9% |

**Table 5: Characterization data for lipid nanoparticles prepared from representative GOLD lipid compounds**

| Lipid nanoparticle No. | GOLD lipid | Cationic lipid | Class of component | Size(nm) | PDI (polydispersity index) | Encapsulation efficiency % |
|---|---|---|---|---|---|---|
| TMF11 | 38.5% Compound 1-3 | 50% DODMA | IV | 217.3±4.5 | 0.102±0.028 | 88.2 |
| TMF12 | 38.5% Compound 1-4 | 50% DODMA | IV | 153.88±3.17 | 0.098±0.045 | 70.4 |
| TMF13 | 38.5% Compound 1-5 | 50% DODMA | IV | 255.9±5.7 | 0.130±0.041 | 65.2 |
| TMF14 | 38.5% Compound 1-6 | 50% DODMA | IV | 318.6±2.7 | 0.211±0.050 | 85.8 |
| TMF15 | 38.5% Compound 1-7 | 50% DODMA | IV | 203.58±2.43 | 0.157±0.018 | 59.1 |
| TMF16 | 38.5% Compound 1-8 | 50% DODMA | IV | 193.210102.76 | 0.150±0.046 | 52.1 |
| TMF17 | 38.5% Compound 1-9 | 50% DODMA | IV | 234.28±1.96 | 0.168±0.026 | 13.6 |
| TMF18 | 38.5% Compound 1-25 | 50% DODMA | IV | 238.83±1.98 | 0.145±0.037 | 77.6 |
| TMF19 | 38.5% Compound 1-26 | 50% DODMA | IV | 245.95±1.40 | 0.161±0.019 | 54.9 |
| TMF20 | 38.5% Compound 1-32 | 50% DODMA | IV | 193.74±1.53 | 0.178±0.032 | 78.1 |
| TMF21 | 38.5% Compound 1-37 | 50% DODMA | IV | 254.71±3.34 | 0.102±0.068 | 7 |
| TMF22 | 38.5% Compound 1-39 | 50% DODMA | IV | 220.3 8±2.72 | 0.193±0.058 | 13.3 |
| TMF23 | 38.5% Compound 1-42 | 50% DODMA | IV | 264.24±4.68 | 0.185±0.037 | 19.5 |
| TMF24 | 38.5% Compound 1-44 | 50% DODMA | IV | 350.56±2.82 | 0.231±0.022 | 48.6 |
| TMF25 | 38.5% Compound 1-45 | 50% DODMA | IV | 331.40±17.99 | 0.203±0.031 | -4.6 |
| TMF26 | 38.5% Compound 1-46 | 50% DODMA | IV | 242.11±2.15 | 0.159±0.014 | 42.7 |
| TMF27 | 38.5% Compound 1-48 | 50% DODMA | IV | 277.53±2.46 | 0.247±0.023 | 22.2 |
| TMF28 | 38.5% Compound 1-49 | 50% DODMA | IV | 300.52±3.33 | 0.222±0.047 | 19.7 |
| TMF29 | 38.5% Compound 1-50 | 50% DODMA | IV | 263.24±2.47 | 0.165±0.051 | 6.6 |
| TMF30 | 38.5% Compound 1-51 | 50% DODMA | IV | 257.02±2.89 | 0.221±0.030 | 9.4 |
| TMF31 | 38.5% Compound 1-52 | 50% DODMA | IV | 256.26±4.11 | 0.182±0.026 | -5.2 |
| TMF32 | 38.5% Compound 1-55 | 50% DODMA | IV | 257.74±2.90 | 0.144±0.034 | 47.7 |
| TMF33 | 38.5% Compound 1-56 | 50% DODMA | IV | 287.14±3.55 | 0.234±0.039 | 49.2 |

**Table 6: Characterization data for different lipid nanoparticles prepared from GOLD lipids 1-56**

| Lipid nanoparticle No. | GOLD lipid | Cationic lipid + auxiliary phospholipid | Class of component | Size(nm) | PDI (polydispersity index) | Encapsulation efficiency % |
|---|---|---|---|---|---|---|
| TMF33 | 38.5% Compound 1-56 | DODMA | IV | 287.14±3.55 | 0.234±0.039 | 49.2 |
| TMF34 | 38.5% Compound 1-56 | CKK-E12 | IV | 277.67±1.89 | 0.219±0.009 | 89.5 |
| TMF35 | 38.5% Compound 1-56 | C12-200 | IV | 254.40±0.45 | 0.221±0.026 | 80.3 |
| TMF36 | 50% Compound 1-56 | Chol+DSPC+PEG2000-DMG | IV | 218.14±5.90 | 0.121±0.043 | -1.9 |
| TMF37 | 98.5% Compound 1-56 | PEG2000-DMG | II | 177.33±1.36 | 0.093±0.031 | -0.7 |
| TMF38 | 100% Compound 1-56 | None | I | 374.22±2.72 | 0.265±0.019 | 1.4 |

### Example 14: Delivery System Organ Distribution Detection

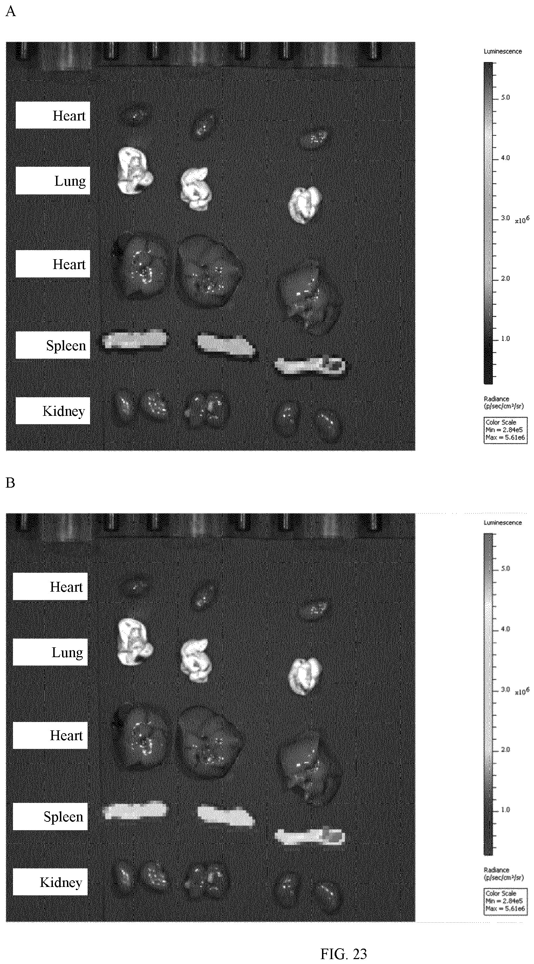

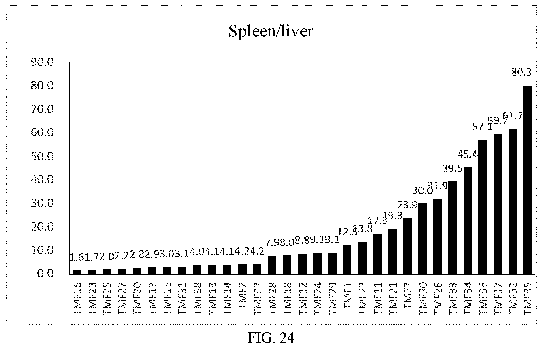

The liposomes prepared in Examples 1, 2, 3, 4, 5, 6, 7, 8, 10, and 12 were injected at a dose of 0.5 mg/kg through the angular venous plexus of the fundus to 6-8 week-old female ICR mice. 4 h after the administration, 15 mg/mL of D-luciferin potassium salt was intraperitoneally injected at a dose of 150 mg/kg. 10 min after injection of luciferase substrate, mice were placed under an in vivo imaging system (IVIS Lumina XRMS Series III, PerkinElmer) and observed for in vivo fluorescence intensity and distribution. The mice were then sacrificed, organs (heart, liver, spleen, lung and kidney) were isolated and imaged ex vivo to observe the fluorescence intensity and distribution in the different organs. Organ distribution of Luc-mRNA delivered by representative lipid compounds is shown in Tables 7, 8 and FIG. 23. The spleen-to-liver selectivity data are summarized in FIG. 24, and the results show that as long as the ratio of the spleen to the liver is higher than 1, the selectivity for the spleen is better and the spleen targeting effect can be achieved. The higher the ratio is, the better the spleen targeting effect is. Among them, the lipid nanoparticles TMF36, TMF17, TMF32, and TMF35 have the highest spleen-to-liver selectivity. 4 h after intravenous administration to the mice, the ratio of the mean fluorescence intensity of TMF36, TMF17, TMF32, and TMF35 in the spleen and liver (spleen/liver) is 57.1, 59.7, 61.7, and 80.3, respectively. The mean fluorescence intensity in different organs represents the delivery efficiency of the corresponding delivery system in different organs. The lipid nanoparticle of the present invention is able to successfully deliver nucleic acid molecules into the spleen and enable the expression of the nucleic acid molecules, and the delivery efficiency for the spleen is significantly higher than that for the liver and other tissues/organs.

**Table 7: Expression intensity of Luc-mRNA delivered by lipid nanoparticles prepared from representative GOLD lipids.**

| Lipid nanoparticle No. | GOLD lipid used | Mean fluorescence intensity | | |
|---|---|---|---|---|
| | | Liver [p/s/cm²/sr] | Spleen [p/s/cm²/sr] | Spleen/liver |
| TMF1 | 20% Compound 1-1 | 9.14E+04 | 1.14E+06 | 12.47 |
| TMF2 | 5% Compound 1-1 | 4.25E+05 | 1.78E+06 | 4.18 |
| TMF7 | 38.5% Compound 1-1 | 2.61E+05 | 5.51E+06 | 23.9 |
| TMF11 | 38.5% Compound 1-3 | 5.69E+05 | 9.99E+06 | 17.3 |
| TMF12 | 38.5% Compound 1-4 | 2.27E+04 | 1.71E+05 | 8.8 |
| TMF13 | 38.5% Compound 1-5 | 5.48E+05 | 2.09E+06 | 4.1 |
| TMF14 | 38.5% Compound 1-6 | 8.84E+05 | 3.09E+06 | 4.1 |
| TMF15 | 38.5% Compound 1-7 | 1.17E+05 | 3.71E+05 | 3.0 |
| TMF16 | 38.5% Compound 1-8 | 9.10E+04 | 1.27E+05 | 1.6 |
| TMF17 | 38.5% Compound 1-9 | 2.95E+05 | 1.91E+07 | 59.7 |
| TMF18 | 38.5% Compound 1-25 | 8.83E+05 | 7.09E+06 | 8.0 |

| TMF19 | 38.5% Compound 1-26 | 4.38E+05 | 1.29E+06 | 2.9 |
|---|---|---|---|---|
| TMF20 | 38.5% Compound 1-32 | 2.12E+06 | 5.27E+06 | 2.8 |
| TMF21 | 38.5% Compound 1-37 | 2.27E+05 | 3.04E+06 | 19.3 |
| TMF22 | 38.5% Compound 1-39 | 4.24E+04 | 5.86E+05 | 13.8 |
| TMF23 | 38.5% Compound 1-42 | 6.95E+05 | 1.19E+06 | 1.7 |
| TMF24 | 38.5% Compound 1-44 | 6.66E+04 | 6.05E+05 | 9.1 |
| TMF25 | 38.5% Compound 1-45 | 1.38E+04 | 2.84E+04 | 2.0 |
| TMF26 | 38.5% Compound 1-46 | 1.99E+05 | 6.36E+06 | 31.9 |
| TMF27 | 38.5% Compound 1-48 | 2.05E+05 | 3.20E+05 | 2.2 |
| TMF28 | 38.5% Compound 1-49 | 1.21E+05 | 9.07E+05 | 7.9 |
| TMF29 | 38.5% Compound 1-50 | 7.47E+04 | 7.06E+05 | 9.1 |
| TMF30 | 38.5% Compound 1-51 | 3.88E+04 | 1.06E+06 | 30.0 |
| TMF31 | 38.5% Compound 1-52 | 8.88E+04 | 1.74E+05 | 3.1 |
| TMF32 | 38.5% Compound 1-55 | 5.26E+04 | 3.25E+06 | 61.7 |
| TMF33 | 38.5% Compound 1-56 | 6.18E+05 | 2.44E+07 | 39.5 |

**Table 8: Expression intensity of Luc-mRNA delivered by different lipid nanoparticles prepared from GOLD lipids 1-56**

| Lipid nanoparticle No. | GOLD lipid used | Mean fluorescence intensity | | |
|---|---|---|---|---|
| | | Liver [p/s/cm²/sr] | Spleen [p/s/cm²/sr] | Spleen/liver |
| TMF33 | DODMA + Compound 1-56 | 6.18E+05 | 2.44E+07 | 39.5 |
| TMF34 | CKK-E12 + Compound 1-56 | 1.01E+05 | 4.58E+06 | 45.4 |
| TMF35 | C12-200 + Compound 1-56 | 4.27E+04 | 3.43E+06 | 80.3 |
| TMF36 | 50% Compound 1-56 Chol + DSPC + PEG2000-DMG | 1.71E+03 | 9.74E+04 | 57.1 |
| TMF37 | 98.5% Compound 1-56 + | 5.08E+03 | 2.12E+04 | 4.2 |
| | PEG2000-DMG | | | |
| TMF38 | 100% Compound 1-56 | 3.21E+03 | 1.28E+04 | 4.0 |

The present invention has been described in detail above, and it will be understood by those skilled in the art that the present invention is not limited to the scope of protection, but rather, to be capable of understanding the mechanism and content of the present invention and implementing it. All equivalent changes or modifications in line with the spirit of the present invention should fall within the scope of protection of the present invention.

### Industrial Practicability

The organ-specific delivery composition provided for nucleic acid can specifically deliver preventive agents / therapeutic agents (especially nucleic acid components) to the target organs, especially to organs outside the liver, providing more options for the delivery of nucleic acid drugs, gene drugs, vaccines, etc., especially for the development and application of nucleic acid preventive agents and therapeutic agents.

## Claims

1. A lipid nanoparticle for organ-targeted delivery comprising a guided on-target lipid delivery lipid selected from one of or a combination of more than one of an ionizable anionic steroid and/or an ionizable anionic polymer conjugated lipid.

2. The lipid nanoparticle according to claim 1, wherein the ionizable anionic steroid is selected from a compound of Formula I or a pharmaceutically acceptable salt, a prodrug, a stereoisomer, or a deuteride thereof: 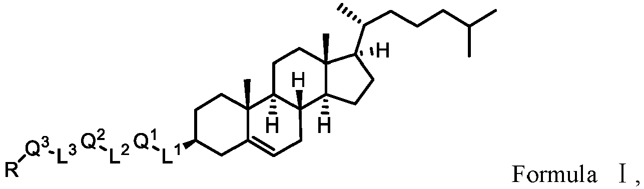
wherein L¹ is absent, -C-C-, -C=C-, -C≡C-,, -O(C=O)-, -(C=O)O-, -C(=O)-, -O(C=O)O-, -O-, - S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-; Q¹ is absent or C1-C8 linear or branched hydrocarbyl;
L² is absent, -C-C-, -C=C-, -C=C-, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-; Q² is absent or C1-C8 linear or branched hydrocarbyl;
L³ is absent, -C-C-, -C=C-, -C=C-, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-; Q³ is absent or C1-C8 linear or branched hydrocarbyl;
R^{a} is H, deuterium, C1-C8 linear or branched hydrocarbyl, or C1-C8 linear or branched hydrocarbyl substituted with carboxyl; or -Q⁴-M, wherein Q⁴ is absent or C1-C8 linear or branched hydrocarbyl, and M is 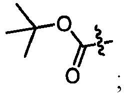 x is 0, 1, or 2;
R is 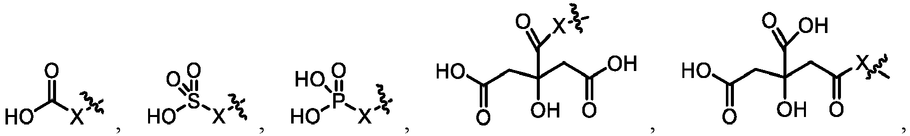 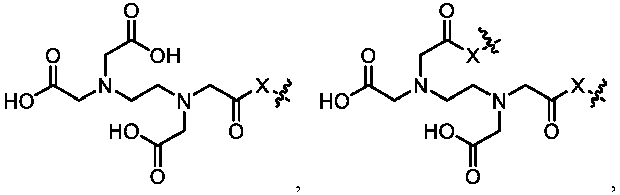 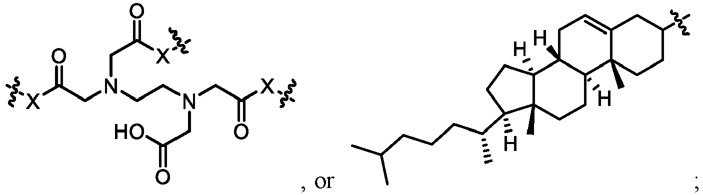 X is C, O, NR^{b}, or S; R^{b} is H, deuterium, or C1-C8 linear or branched hydrocarbyl.

3. The lipid nanoparticle according to claim 2, wherein the ionizable anionic steroid is a compound selected from those shown in Table 1, or a pharmaceutically acceptable salt, a prodrug, a stereoisomer, or a deuteride thereof.

4. The lipid nanoparticle according to claim 1, wherein the ionizable anionic polymer conjugated lipid is selected from a compound of Formula II or a pharmaceutically acceptable salt, a prodrug, a stereoisomer, or a deuteride thereof: 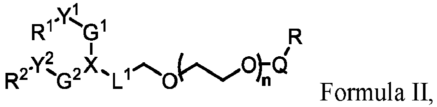
wherein L¹ is absent, a carbon-carbon single bond, a carbon-carbon double bond, a carbon-carbon triple bond, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-; Q is absent or C1-C8 linear or branched hydrocarbyl;
R^{a} is H, deuterium, C1-C8 linear or branched hydrocarbyl, or C1-C8 linear or branched hydrocarbyl substituted with carboxyl;
X is CorN;
R is 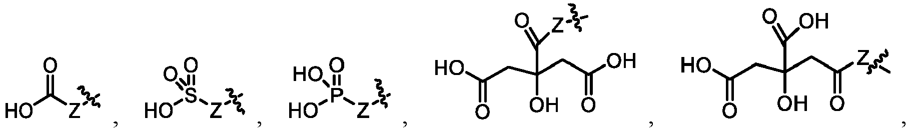 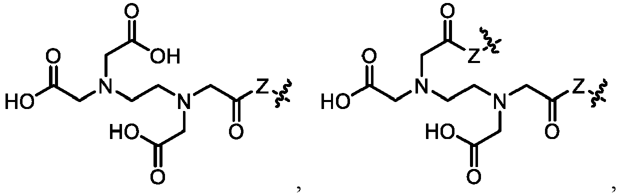 or 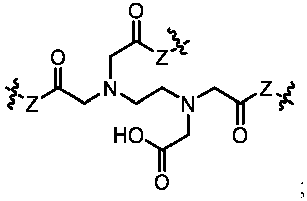 Z is C, O, NR^{b}, or S;
one of Y¹ and Y² is absent, -C-C-, -C=C-, -C=C-, -O(C=O)-, -C(OH)-, -(C=O)O-, -C(=O)-, - O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-, and the other one of Y¹ and Y² is absent, -C-C-, -C=C-, -C=C-, -O(C=O)-, -C(OH)-, -(C=O)O-, -C(=O)-, -O(C=O)O-, -O-, -S(O)ₓ-, -S-S-, -C(=O)S-, -SC(=O)-, - NR^{a}C(=O)-, -C(=O)NR^{a}-, -NR^{a}C(=O)NR^{a}-, -OC(=O)NR^{a}-, or -NR^{a}C(=O)O-; R^{a} is H, deuterium, C1-C8 linear or branched hydrocarbyl, or C1-C8 linear or branched hydrocarbyl substituted with carboxyl;
G¹ and G² are each independently absent or substituted C1-C12 linear or branched hydrocarbyl;
R¹ and R² are each independently substituted C6-C24 linear or branched hydrocarbyl;
x is 0, 1, or 2.

5. The lipid nanoparticle according to claim 4, wherein the ionizable anionic polymer conjugated lipid is a compound selected from those shown in Table 2, or a pharmaceutically acceptable salt, a prodrug, a stereoisomer, or a deuteride thereof.

6. The lipid nanoparticle according to claim 1, wherein the lipid nanoparticle further comprises a helper lipid selected from one of or a combination of more than one of a phospholipid and/or a steroid and/or a polymer conjugated lipid and/or a modified lipid.

7. The lipid nanoparticle according to claim 6, wherein the phospholipid is selected from one of or a combination of more than one of DOPE, DSPC, DPPC, DMPC, DOPC, POPC, and SM.

8. The lipid nanoparticle according to claim 6, wherein the steroid is selected from any one of or a combination of more than one of cholesterol, sitosterol, stigmasterol, and ergosterol.

9. The lipid nanoparticle according to claim 6, wherein the polymer conjugated lipid is selected from one or more of a polyethylene glycol conjugated lipid, a polylactic acid conjugated lipid, a polyamide conjugated lipid, a cationic polymer conjugated lipid, a poly-sarcosine (pSar) conjugated lipid, a poly(lactic-co-glycolic acid) (PLGA) conjugated lipid, a polyamino acid conjugated lipid, a polypeptide conjugated lipid, and a polypeptoid conjugated lipid.

10. The lipid nanoparticle according to claim 9, wherein the polyethylene glycol conjugated lipid is selected from any one of or a combination of more than one of PEG1000-DMG, PEG5000-DMG, PEG2000-DMG, and PEG2000-DSPE.

11. The lipid nanoparticle according to claim 6, wherein the modified lipid is selected from a lipid modified by any one of a small-molecule compound, a vitamin, a carbohydrate, a peptide, a protein, a nucleic acid lipopolysaccharide, an inorganic molecule or particle, and a metal ion or particle, or a combination thereof.

12. The lipid nanoparticle according to claim 6, wherein the lipid nanoparticle further comprises a cationic lipid selected from one of or a combination of more than one of a permanently cationic lipid and/or an ionizable cationic lipid.

13. The lipid nanoparticle according to claim 12, wherein the permanently cationic lipid is selected from one of or a combination of more than one of DOTAP, DODMA, DSTAP, DMTAP, DDA, and DOBAQ.

14. The lipid nanoparticle according to claim 12, wherein the ionizable cationic lipid is selected from one of or a combination of more than one of SM-102, Lipid 5, A6, DC-chol, C12-200, CKK-E12, 5A2-SC8, G0-C14, OF-2, 306Oi10, OF-Deg-Lin, 92-0175, OF-C4-Deg-Lin, A18-iso5-2DC18, TT3, FTT5, BAMEA-O16B, Vc-Lipid, C14-4, Lipid 14, 4A3-Cit, and ssPalmO-Phe.

15. The lipid nanoparticle according to claim 12, wherein the guided on-target delivery lipid, the helper lipid, and the cationic lipid are in a molar ratio of (0.1-1):(0.5-2):1.

16. The lipid nanoparticle according to any one of claims 1 to 15, wherein the lipid nanoparticle targets the following organs: lung, heart, brain, spleen, lymph node, bone, skeletal muscle, stomach, small intestine, large intestine/colon and rectum, kidney, bladder, breast, testis, ovary, uterus, thymus, brainstem, cerebellum, cerebrum, spinal cord, eye, ear, tongue, or skin, preferably spleen.

17. A composition comprising a therapeutic or prophylactic agent and the lipid nanoparticle according to any one of claims 1 to 16.

18. The composition according to claim 17, wherein the prophylactic or therapeutic agent is selected from any one of or a combination of more than one of a nucleic acid, a protein, a polypeptide, a small-molecule compound, and a cell.

19. The composition according to claim 17, wherein the lipid nanoparticle and the prophylactic or therapeutic agent are in a mass ratio of 10:1 to 100:1.

20. The composition according to claim 17, wherein the composition has an average particle size of 20 nm to 600 nm.

21. The composition according to claim 17, wherein the composition has a polydispersity index (PDI) of 0.001 to 0.5.

22. The composition according to claim 18, wherein the nucleic acid is selected from a single-stranded DNA, a double-stranded DNA, a single-stranded RNA, a double-stranded RNA, a short isomer, a plasmid DNA, a complementary DNA/cDNA, an antisense oligonucleotide/ASO, a small interfering nucleic acid/siRNA, a small activating nucleic acid/saRNA, an asymmetric interfering nucleic acid/aiRNA, a micro nucleic acid/miRNA, a micro nucleic acid agonist/miRNA agomir, a micro nucleic acid inhibitor/miRNA antagomir, a Dicer enzyme substrate nucleic acid/dsRNA, a small hairpin nucleic acid/shRNA, a transfer RNA/tRNA, a messenger RNA/mRNA, a circular RNA/circRNA, a self-amplifying mRNA/samRNA, and an aptamer.

23. The composition according to claim 22, wherein the therapeutic or prophylactic agent comprises at least one mRNA encoding an antigen or a fragment thereof or an epitope thereof, or mRNA encoding a therapeutic protein.

24. The composition according to claim 23, wherein the mRNA is selected from a monocistronic mRNA and a polycistronic mRNA.

25. The composition according to claim 23, wherein the mRNA comprises one or more functional nucleotide analogs or chemically modified forms of nucleotides.

26. The composition according to claim 25, wherein the functional nucleotide analogs are selected from one of or a combination of more than one of a locked nucleic acid (LNA), a peptide nucleic acid (PNA), and a morpholine ring oligonucleotide nucleic acid mimic or functional analog.

27. The composition according to claim 25, wherein the chemical modifications of nucleotides are selected from one or more of the following modifications:
(1) a modification to the backbone of a linking nucleotide in the nucleotide sequence of the nucleic acid molecule;
(2) a modification to ribose in the nucleotide sequence of the nucleic acid molecule; and
(3) a modification to a base in the nucleotide sequence of the nucleic acid molecule.

28. The composition according to claim 27, wherein the modification to the backbone is a phosphorothioate bond.

29. The composition according to claim 27, wherein the modification to ribose is selected from one of or a combination of more than one of 2'-fluororibose, ribose, 2'-deoxyribose, arabinose, and hexose.

30. The composition according to claim 25, wherein the chemical modifications of nucleotides are selected from one of or a combination of more than one of 5-methylcytosine, pseudouridine, 1-methylpseudouridine, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouri dine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

31. The composition according to claim 23, wherein the antigen is a pathogenic antigen.

32. The composition according to claim 31, wherein the pathogenic antigen is selected from any one of or a combination of more than one of a tumor-associated antigen and a pathogenic microbial antigen.

33. The composition according to any one of claims 17-32, wherein the composition targets the following organs: lung, heart, brain, spleen, lymph node, bone, skeletal muscle, stomach, small intestine, large intestine/colon and rectum, kidney, bladder, breast, testis, ovary, uterus, thymus, brainstem, cerebellum, cerebrum, spinal cord, eye, ear, tongue, or skin, preferably spleen.

34. Use of the composition according to any one of claims 17-33 in the preparation of a medicament.

35. A medicament comprising the composition according to any one of claims 17-33 and a pharmaceutically acceptable auxiliary material.
